# EUROPEAN PATENT APPLICATION

(11) **EP 4 663 208 A1**
(43) Date of publication of application: **17.12.2025**
(21) Application number: 24753712.9
(22) Date of filing: 08.02.2024
(51) Int. Cl.: A61K 49/18, A61K 9/00, A61K 49/12, A61K 47/69

(54) **POLYSACCHARIDE CROSS-LINKED COLLOIDAL PARTICLES SQUEEZABLE BETWEEN INTERNAL BODY STRUCTURES WITHOUT AGGREGATION, AND USE THEREOF AS MODIFIER FOR IN VIVO INJECTION**

(30) Priority: 08.02.2023 KR 20230016863; 09.02.2023 KR 20230017237; 07.06.2023 KR 20230072904; 19.10.2023 KR 20230140261
(71) Applicant: Inventera Inc., Seoul 06588 (KR)
(72) Inventor: SHIN, Tae-Hyun, Uiwang-si, Gyeonggi-do 16003 (KR); KIM, Ji-wook, Seoul 07361 (KR)
(74) Representative: Schiweck Weinzierl Koch Patentanwälte Partnerschaft mbB
(86) International application number: PCT/KR2024/095103
(87) International publication number: WO 2024/167391

(57) **Abstract**

The present invention relates to polysaccharide-crosslinked colloidal particles and their use as modifiers for substances intended for in vivo administration.

The present invention provides a polysaccharide-crosslinked colloidal particle platform that is not only capable of serving as a drug delivery vehicle for functional nanoparticles or drugs intended for in vivo administration, but also can be engineered to regulate in vivo biodistribution and excretion.

## Description

### TECHNICAL FIELD

The present invention relates to polysaccharide-crosslinked colloidal particles that are squeezable without aggregation within in vivo structures, and to their use as modifiers for substances intended for in vivo administration.

### BACKGROUND ART

When nanoparticles are administered into the body for in vivo applications, it is essential that they exhibit biocompatibility or bioinertness. In other words, when injected into the human body, they must not trigger allergic or immune rejection responses and must be non-toxic.

Nanoparticles composed of metal oxides, quantum dots, and noble metals exhibit unique optical, magnetic, and electrical properties that are distinct from those of molecular-scale materials. These physicochemical properties can be utilized to observe, modulate, and control biological processes at the molecular level, enabling medically useful functionalities. Despite such potential, nanoparticles introduced into the body are often recognized as foreign by the immune system and are subject to phagocytosis by macrophages, leading to accumulation in organs and difficulty in clearance. Particularly, in the case of quantum dots and other heavy metal-based particles, there is a risk of toxicity if the particles are not excreted and instead degrade within the body.

Nanomedicines, which leverage intrinsic therapeutic characteristics, drug delivery capability, diagnostic functions, imaging, vaccine action, and compatibility with miniaturized medical devices, have been applied to the diagnosis and treatment of diseases. In order for a nanomedicine to be used as a diagnostic agent for in vivo disease diagnosis, it must be engineered to be safe and formulated in a manner that allows elimination from the body without undergoing metabolic degradation.

Meanwhile, polymer-drug conjugates, such as those utilizing polyethylene glycol (PEG), polyglutamate, and hyaluronic acid (HA) as drug delivery vehicles, have been widely studied.

Nanoparticles also possess a high surface-to-volume ratio, enabling a large number of drug molecules to be conjugated per unit mass of nanoparticle, making them highly suitable for use as drug delivery platforms. Such nanomedicines can carry and deliver different types of drugs simultaneously to exert synergistic therapeutic effects, for example, in cancer therapy.

Examples include liposomes and polymeric micelles that are designed to encapsulate drugs and allow slow diffusion at the target site, both of which have been clinically approved for cancer treatment. Viral nanomedicines designed to deliver small interfering RNA (siRNA) are also notable examples. Modified nanostructures derived from adeno-associated viruses, lentiviruses, and plant viruses such as *tobacco mosaic virus* and *potato virus X* have been spotlighted as siRNA delivery vectors.

In the design of nanomedicines or drug delivery vehicles, strategies for targeted delivery to the disease site are among the most critical factors influencing diagnostic and therapeutic accuracy and efficiency. Currently, the majority of nanomedicines are thought to be delivered to tumor tissues via the enhanced permeability and retention (EPR) effect. According to this hypothesis, tumor vasculature tends to be more permeable and less connected to lymphatic drainage compared to normal tissues due to rapid tumor growth, allowing nanoparticles with diameters in the nanometer-to-micrometer range to preferentially penetrate and accumulate.

However, a report published in Nature Reviews Materials in 2016 noted that the amount of nanomedicine actually delivered to tumor tissue via the EPR effect accounts for only approximately 0.7% of the injected dose, with the amount taken up by tumor cells being less than 0.01%. This is primarily due to the presence of extracellular matrix (ECM) structures, which act as a mesh between cells and hinder the diffusion and mobility of nanoparticles due to their relatively large size compared to small molecules.

Recently, efforts have been made to enhance delivery efficiency targeted to the disease site by considering interactions between nanoparticles and the in vivo microenvironment. For example, advanced delivery vehicles are being developed by modulating nanoparticle interactions with proteins, phagocytosis, blood circulation half-life, extravasation, renal permeability, tumor microenvironment interactions, tumor penetration, and intracellular uptake. These interactions are known to be influenced by key physicochemical properties of nanoparticles, such as size, shape, surface properties, flexibility, porosity, and surface ligands. Therefore, if these physicochemical characteristics of nanoparticles can be precisely controlled to suit a given biological environment, it is expected that next-generation nanomaterials capable of significantly improving the accuracy and efficiency of disease diagnosis and therapy can be developed.

### DETAILED DESCRIPTION OF THE INVENTION

### TECHNICAL PROBLEM

The present invention relates to the design of polysaccharide-crosslinked colloidal particles that function as modifiers capable of exerting a "stealth" effect-similar to PEGylation of drugs-by regulating interactions with the body's immune system. By modifying drugs, including functional nanoparticles, with the polysaccharide-crosslinked colloidal particles, the modified drugs can evade immune recognition, thereby enhancing therapeutic delivery and efficacy.

The invention also seeks to provide a platform technology of polysaccharide-crosslinked colloidal particles that are engineered to be squeezable and capable of migrating within in vivo structures without aggregation, while allowing precise control over various physicochemical properties to suit the specific in vivo biological environment through which they pass.

The invention further provides polysaccharide-crosslinked colloidal particles as in vivo administrable modifiers for imparting or enhancing colloidal stability in body fluids to drugs, such as functional nanoparticles and/or non-particulate drugs, including biologically insoluble or poorly soluble drugs.

In addition, the invention provides a platform technology for polysaccharide-crosslinked colloidal particles that are not only engineered to function as drug delivery carriers for functional nanoparticles or drugs intended for in vivo administration, but also configurable to regulate in vivo biodistribution and excretion.

Furthermore, the invention provides a platform technology for polysaccharide-crosslinked colloidal particles that can be engineered either to evade immune phagocytosis, thereby prolonging circulation time in the bloodstream, or to be phagocytosed by macrophages and excreted through urine or feces via renal or hepatic clearance mechanisms.

### TECHNICAL SOLUTION

According to a first aspect of the present invention, there is provided an in vivo administrable complex comprising:
(a) a polysaccharide-crosslinked colloidal particle formed by intra- and/or inter-molecular crosslinking of 1 to 3 branched polysaccharides or 2 to 30 cyclic polysaccharides at hydroxyl groups of constituent monosaccharide building blocks using a crosslinker; and
(b) a functional nanoparticle or a drug for in vivo administration, bound to a surface-exposed functional group derived from the crosslinker;

wherein the type and number of the crosslinker-derived functional groups available for conjugating the functional nanoparticle or the drug are controlled;
and wherein the polysaccharide-crosslinked colloidal particle and/or the in vivo administrable complex is engineered to be dispersible in vivo without aggregation, and to be squeezable, such that it is capable of migrating within in vivo structures due to its soft and deformable characteristics.

According to a second aspect of the present invention, there is provided a composition for modifying a substance intended for in vivo administration, comprising:
a polysaccharide-crosslinked colloidal particle formed by intra- and/or inter-molecular crosslinking of 1 to 3 branched polysaccharides or 2 to 30 cyclic polysaccharides at hydroxyl groups of constituent monosaccharide building blocks using a crosslinker,
wherein the polysaccharide-crosslinked colloidal particle is engineered to:
   (i) resist enzymatic hydrolysis in vivo;
   (ii) be dispersible in vivo without aggregation and to be squeezable, such that it is capable of migrating within in vivo structures;
   (iii) exhibit bio-inertness by minimizing interactions with biological systems and suppressing biological responses; and
   (iv) be filterable through the kidney;
and wherein the type and number of crosslinker-derived functional groups exposed on the surface of the polysaccharide-crosslinked colloidal particle are controlled such that the substance intended for in vivo administration can be conjugated thereto.

In the first and second aspects of the present invention, the polysaccharide-crosslinked colloidal particle may have a hydrodynamic diameter of 2 to 20 nm, preferably 10 nm or less, more preferably 8 nm or less, and wherein carboxylic acid (-COOH) groups are exposed on the particle surface, resulting in a surface charge ranging from -20 mV to 0 mV, such that the particle is filterable through the kidney but is not permeable through the vascular wall of normal capillaries, and is excreted exclusively via lymphatic vessels.

In the first and second aspects of the present invention, when the polysaccharide-crosslinked colloidal particle is formed by intra- and/or inter-molecular crosslinking of branched polysaccharides or cyclic polysaccharides at -OH functional groups of their constituent monosaccharide units using a crosslinker, the ratio of monosaccharide units crosslinked by the crosslinker may be controlled to be from 2% to 60% relative to the total number of monosaccharide units, such that the particle is bio-inert by minimizing interactions with biological systems or biological responses and/or is filterable through the kidney

For example, the present invention enables the manufacture of squeezable polysaccharide-crosslinked colloidal particles by reacting a branched or cyclic polysaccharide aqueous solution with (i) an epoxide compound that modifies the -OH functional groups of constituent monosaccharide building blocks and/or (ii) a crosslinker, at room temperature, without requiring expensive catalysts, thereby forming intra- and/or inter-molecular crosslinks at the -OH functional groups of the monosaccharide building blocks.

Additionally, by modulating at least one of the following parameters-molecular weight of dextran, molecular weight of the polysaccharide, length of the polysaccharide main chain, type of crosslinker, amount and rate of crosslinker addition during synthesis, and degree of further chemical modification-the physicochemical properties of the polysaccharide-crosslinked colloidal particle can be finely and diversely engineered. Thus, the present invention provides a platform technology for squeezable polysaccharide-crosslinked colloidal particles.

For instance, in the polysaccharide-crosslinked colloidal particles of the present invention, the substitution ratio of crosslinkers may be controlled to 2% to 50% of the total number of functional groups of the polysaccharide, and 2% to 98% of the crosslinkers may be configured such that one terminal of the crosslinker remains unreacted and is exposed on the surface, thereby enabling precise tuning of the surface charge of the polysaccharide-crosslinked colloidal particles.

Accordingly, the squeezable polysaccharide-crosslinked colloidal particle platform technology of the present invention can be finely engineered with diverse physicochemical properties such that it simultaneously serves as one or more types of modifiers selected from the group consisting of: a colloidal stability imparting modifier for in vivo administration, a squeezable modifier for tissue penetration, a hydrodynamic size modifier, a surface charge modifier, an anti-opsonization agent (opsonization inhibitor), a blood circulation half-life regulator, a pharmacokinetic modulator, an extravasation modulator, a blood capillary permeability modulator, a modifier for blood circulation, a modifier for lymphatic circulation, a modulator for avoidance of phagocytosis by macrophages, a modifier for body location tracking for MRI, a modifier for intravenous administration, a modifier for oral administration, a regulator for in vivo distribution and excretion, and a drug delivery vehicle for drug loading and release.

The invention is described in detail below.

The entire disclosure of Korean Patent Application No. 10-2021-0104405 is incorporated herein by reference.

The inventors have discovered that, in an aqueous solution, complex branched dextran or its derivatives form spherical nanoparticles through intramolecular and/or intermolecular crosslinking at the hydroxyl (-OH) groups of glucose building blocks using a crosslinker. 1 to 3 molecules of dextran or dextran derivatives can form such nanoparticles. These dextran-crosslinked nanoparticles exhibit excellent colloidal stability without aggregation and possess squeezability. Furthermore, it was found that reactive functional groups derived from the crosslinker, which are exposed on the surface of the dextran-crosslinked nanoparticles, can be utilized to modify the surface of the nanoparticles and to conjugate drugs for in vivo applications. By varying the type and/or degree of substitution of these surface-exposed functional groups, the surface charge of the nanoparticles can be freely controlled (see Examples 1-5 and 1-6). Moreover, the number of functional molecules (e.g., drugs) conjugated to the dextran-crosslinked nanoparticles can also be tuned by adjusting the reaction stoichiometry (see Example 1-7). It was also confirmed that the hydrodynamic diameter of the dextran-crosslinked nanoparticles increases with the molecular weight of the dextran used in the synthesis, allowing for control of the size of the resulting nanoparticle-bioconjugate complex for in vivo administration (see Example 1-10).

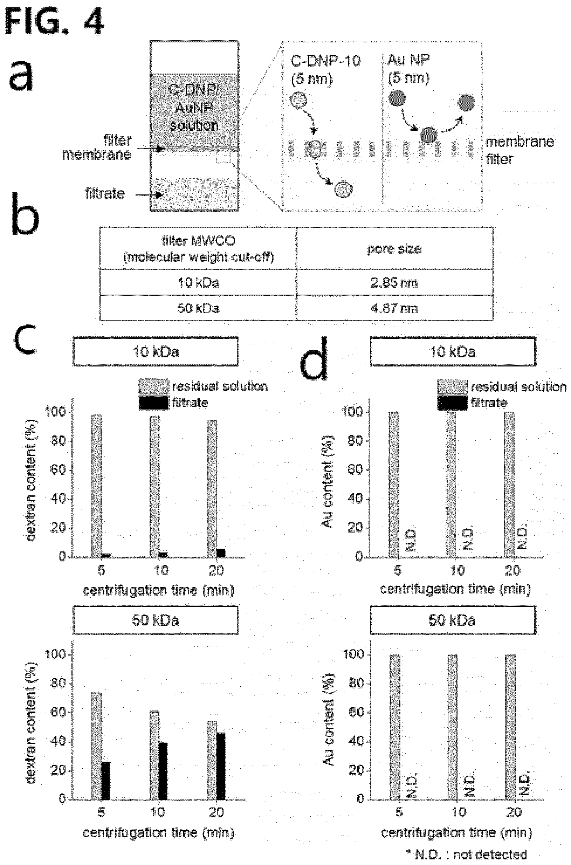

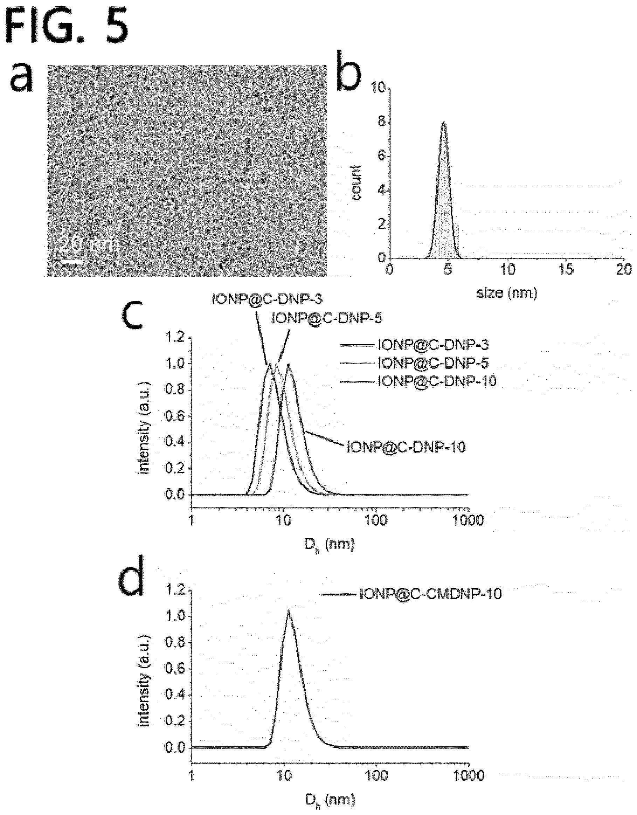

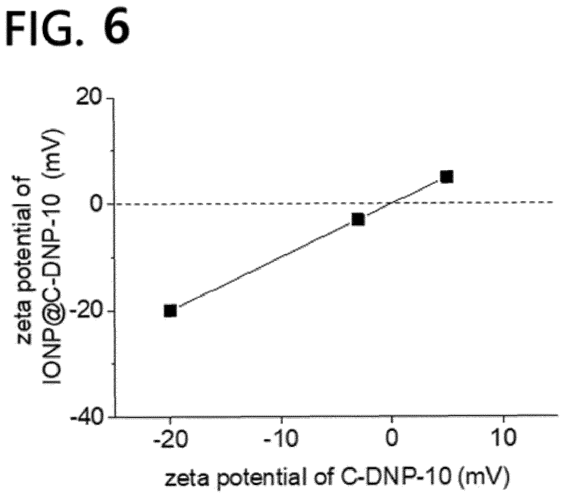

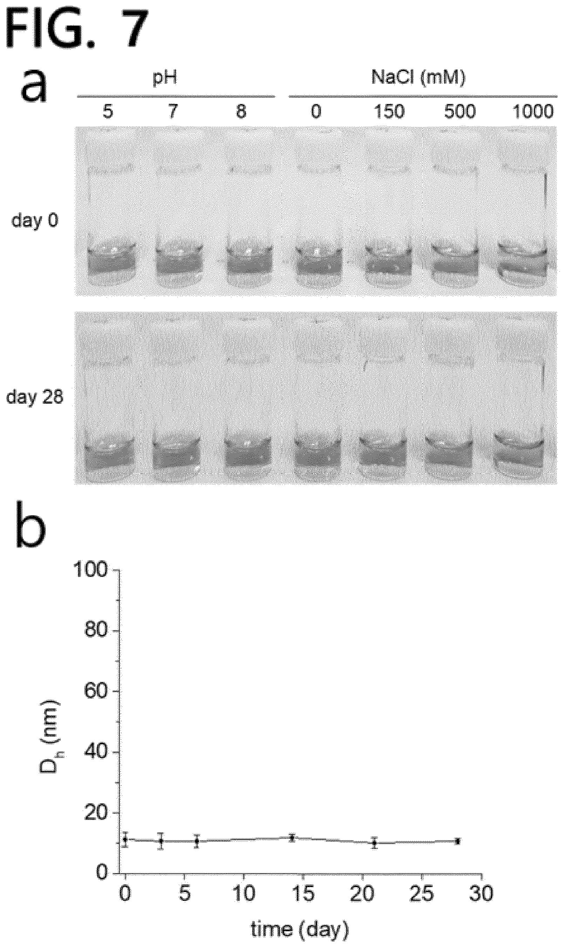

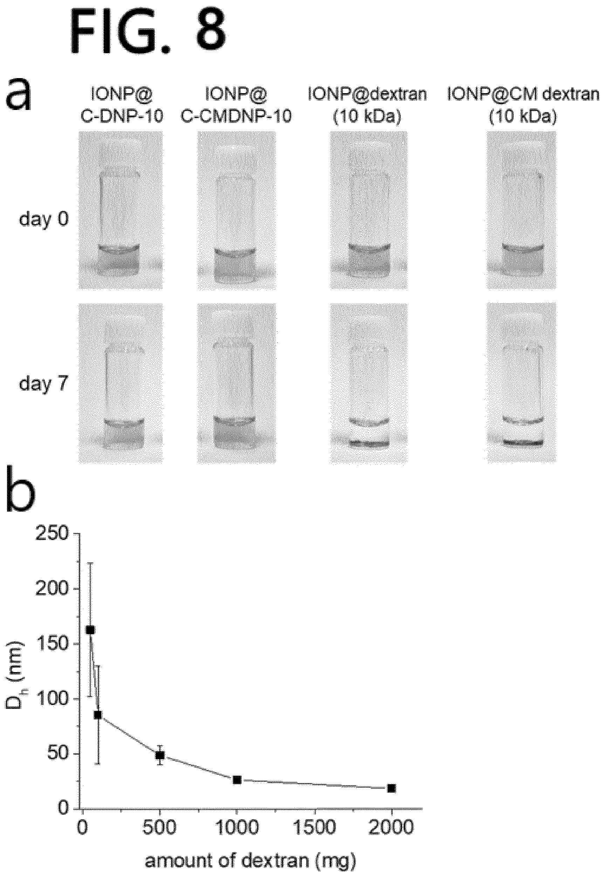

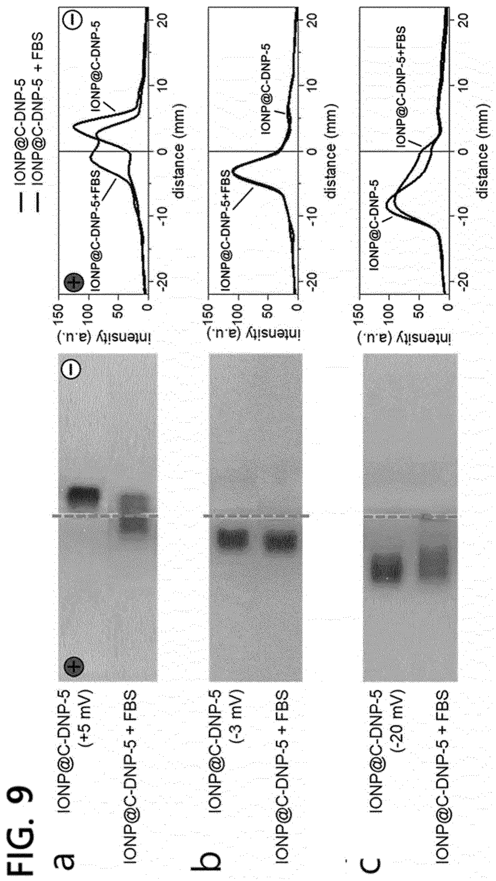

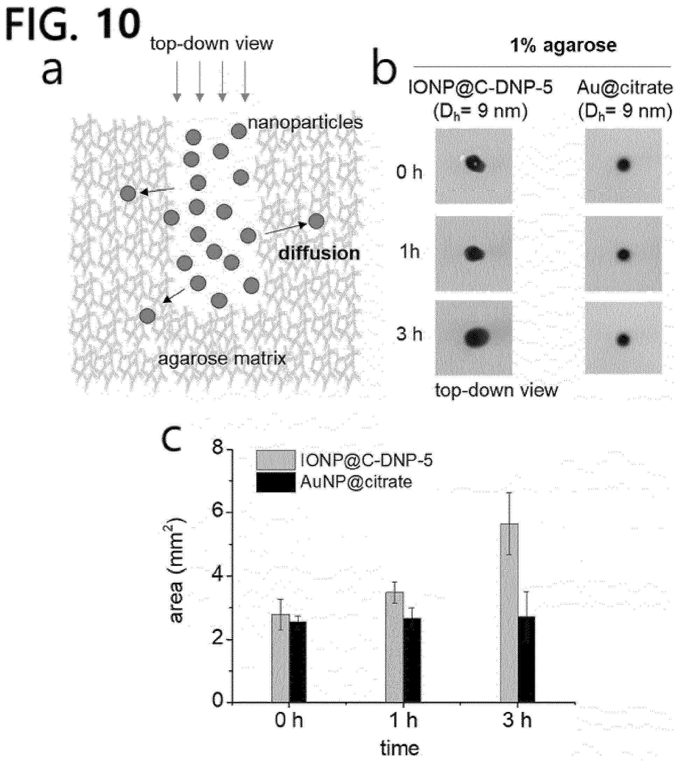

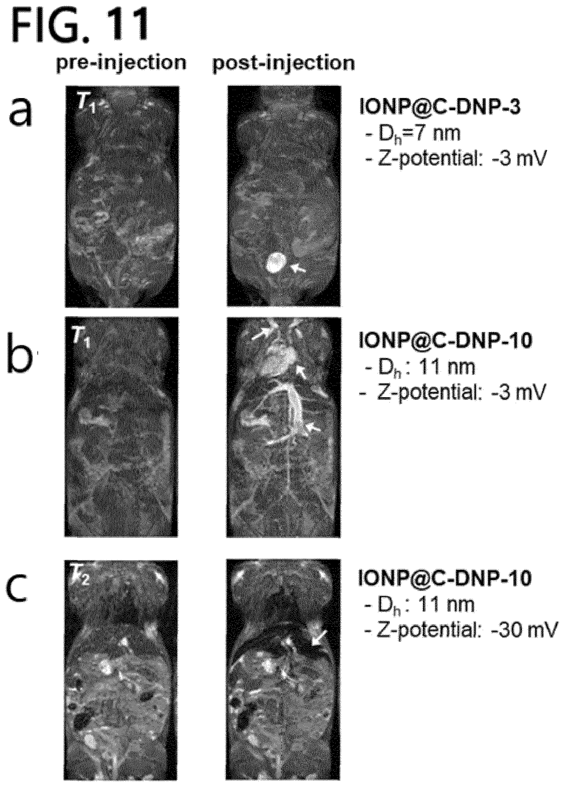

Specifically, it was discovered that the dextran-crosslinked nanoparticles (C-DNPs) exhibit superior squeezability, compared to conventional inorganic nanoparticles. This property facilitates their ability to traverse in vivo biological environments, i.e., biological microstructures, which constitute the targeted passage routes (see Examples 1-8 and FIG. 4). The squeezability of C-DNP-coated iron oxide nanoparticles and the resulting diffusion and mobility in biological tissues were also determined (Examples 1-16 and FIG. 10). Furthermore, in various embodiments, iron oxide nanoparticles coated with C-DNPs of different molecular weights were synthesized and analyzed, and it was found that the larger the molecular weight of the dextran used in the synthesis of C-DNPs, the larger the hydrodynamic size, thus enabling size tunability (Examples 1-10 and FIG. 5); By controlling the surface charge of C-DNPs, it is possible to control the surface charge of iron oxide nanoparticles coated with C-DNPs (Example 1-11 and FIG. 6); and C-DNP-coated iron oxide nanoparticles have excellent colloidal stability by dispersing for a long time without precipitation due to aggregation under various physiological conditions (salt, pH) (example 1-12 and FIG. 7); The use of squeezable C-DNPs with coordination-capable crosslinker-derived functional groups exposed on the surface enables the coating of iron oxide nanoparticles with 20 times less amount than using uncrosslinked dextran and excellent colloidal stability (Examples 1-13 and FIG. 8); In the case of C-DNP-coated iron oxide nanoparticles, the anti-opsonization effect can be controlled by controlling the surface charge of the nanoparticles (Examples 1-15 and FIG. 9); and the pharmacokinetic behavior of C-DNP-coated iron oxide nanoparticles can be controlled by controlling the C-DNP size and surface charge (Examples 1-17 and FIG. 11).

Based on the results of Example 1, Example 2 describes the development of a targeted drug delivery system. Specifically, Toll-like receptors (TLRs), which are applicable to cancer therapy, were selected as the target. A chemical conjugation process was developed to link TLR agonists to the Cdex platform, and the therapeutic potential of the resulting constructs was confirmed in both cell-based and animal models. Preliminary in vitro and in vivo toxicity studies demonstrated the safety of the developed agents.

Furthermore, in Example 3, it was confirmed that the dextran-crosslinked nanoparticles, which comprises 1 to 3 molecules of dextran or dextran derivatives that are crosslinked intra- and/or intermolecularly at the -OH groups of the glucose building blocks, used as modifiers in Example 1, can be extended to form polysaccharide-crosslinked colloidal particles by using 1 to 3 molecules of branched polysaccharides or 2 to 30 molecules of cyclic polysaccharides, crosslinked at the hydroxyl groups of their constituent monosaccharide building blocks.

The present invention is based on these findings.

The in vivo administrable complex of the present invention comprises:
(a) a polysaccharide crosslinked colloidal particle formed by intramolecular and/or intermolecular crosslinking of 1 to 3 branched polysaccharides or 2 to 30 cyclic polysaccharides at hydroxyl groups of their monosaccharide building blocks using a crosslinker; and
(b) a functional nanoparticle or a drug for in vivo administration that is bound to surface-exposed functional groups derived from the crosslinker on the colloidal particle,
wherein the type and number of the crosslinker-derived functional groups conjugated to the functional nanoparticle or drug are controlled.

The polysaccharide cross-linked colloidal particle and/or the in vivo administrable complex is characterized by being squeezable without aggregation, allowing mobility and biodistribution within in vivo structures.

In one specific embodiment of the in vivo administrable complex of the present invention, the polysaccharide crosslinked particle-drug conjugate (PDC) exhibits a defined drug-to-particle ratio (DPR), analogous to the drug-to-antibody ratio (DAR) used in ADCs. The DPR represents the average number of drug molecules conjugated per polysaccharide crosslinked colloidal particle. For example, the present invention contemplates an average DPR of 1 to 10, preferably 1 to 8, more preferably 1 to 6, even more preferably 1 to 4, and particularly 1 to 3 in cases where the drug is a hydrophobic small molecule (to prevent aggregation of the PDC) or a peptide therapeutic (to modulate immunogenicity of the PDC). Uniform DPR values are advantageous in improving the predictability of the pharmacological and physicochemical behavior of the PDC.

The in vivo administrable complex of the present invention enables controlled delivery of functional nanoparticles or drugs to tumor tissues and/or renal clearance through the use of the squeezable polysaccharide crosslinked colloidal particles, which are capable of deforming and navigating within biological structures.

The administrable complex may be administered via various routes, including injection, and is not limited by the site or method of administration, provided that it is suitable for in vivo application.

As used herein, the term "squeezability" refers to the property of being able to occupy less space,

Further, the expression "squeezable within in vivo structures" or "squeezable to migrate within in vivo structures" refers to the capability of a particle to traverse spatial constraints that would otherwise be impassable based on its hydrodynamic diameter alone, by virtue of its squeezable nature and dynamic change in hydrodynamic size.

Non-limiting examples of in vivo structures through which the squeezable colloidal particles and/or in vivo administrable complexes may migrate include intercellular spaces within tissues, such as blood capillaries, lymphatic vessels, gastrointestinal epithelium, and other organ structures, specifically the microscale interstitial spaces and extracellular matrix (ECM) between constituent cells of those tissues.

Furthermore, according to the present invention, a composition for modifying a substance intended for in vivo administration (hereinafter, 'target material' or 'target substance') comprises polysaccharide-crosslinked colloidal particles formed by intramolecular and/or intermolecular crosslinking of 1 to 3 branched polysaccharides or 2 to 30 cyclic polysaccharides at hydroxyl groups of their monosaccharide building blocks using a crosslinker. The polysaccharide-crosslinked colloidal particles are engineered to (i) resist enzymatic hydrolysis in vivo, (ii) remain dispersed in biological environments without aggregation, (iii) be squeezable to allow interstitial passage within in vivo biological structures, (iv) avoid interactions with a biological system or biological responses (i.e., bio-inertness), and (v) be filterable through the kidneys. Importantly, the type and number of crosslinker-derived functional groups exposed on the surface of the polysaccharide-crosslinked colloidal particles are controlled to enable binding to the target material.

As a result, the polysaccharide-crosslinked colloidal particles are compressible (squeezable) and capable of interstitial movement in vivo, bind to the target material via a uniform number of surface-exposed crosslinker-derived functional groups, and improve dispersion stability in biological fluids through hydration of hydrophilic functional groups exposed on the particle surface.

That is, the polysaccharide-crosslinked colloidal particles and/or in vivo administrable complexes modified therewith are water-dispersible. In particular, the hydration of surface-exposed hydrophilic functional groups provides or enhances colloidal stability of the in vivo administrable complex in biological fluids.

Accordingly, the present invention addresses the issue of aggregation, caused by poor colloidal stability under physiological conditions (e.g., salt, pH), which is typically observed with conventional in vivo administrable functional nanoparticles (e.g., iron oxide nanoparticles) or drugs (e.g., poorly soluble drugs, TLR agonists). Such aggregation leads to unpredictable pharmacokinetics and undesirable tissue deposition.

As used herein, "biological fluids" refer to intracellular or extracellular fluids. Extracellular fluids include blood, lymph, and interstitial fluid surrounding cells.

The transport of substances between biological fluids occurs through capillary circulation driven by two opposing forces: hydrostatic pressure and osmotic pressure.

At the arterial end of capillaries, hydrostatic pressure (~30 mmHg) exceeds osmotic pressure (~21 mmHg), allowing plasma fluid to infiltrate tissues. During this process, oxygen and nutrients diffuse outward through the capillary wall, and the extravasated fluid contributes to the interstitial fluid.

In the midsection of capillaries, where the hydrostatic pressure decreases and becomes equal to the osmotic pressure, there is no net movement of water within the blood. However, at the venous end of the capillary bed, the hydrostatic pressure further decreases while the osmotic pressure-maintained by plasma electrolytes and proteins-exceeds the hydrostatic pressure. As a result, interstitial fluid reenters the capillaries from the surrounding tissue, and metabolic waste and carbon dioxide are taken up from the tissue into the bloodstream. Interstitial fluid present in tissues retains all components of blood plasma except red blood cells and plasma proteins.

The amount of fluid that exits the blood capillaries and the amount that returns is generally equivalent, with only a small portion absorbed through the lymphatic system.

In connection with this physiological context, the polysaccharide-crosslinked colloidal particles of the present invention can be engineered to be filtered through the kidneys but not to permeate through the walls of normal blood capillaries, thereby being cleared primarily via lymphatic vessels.

The normal blood capillaries that the polysaccharide-crosslinked colloidal particles of the present invention are unable to permeate possess a continuous endothelium with tight junctions and a basal lamina. Accordingly, since the polysaccharide-crosslinked colloidal particles of the present invention are engineered to be filtered by the kidneys, they can be filtered through the blood capillaries of the liver and spleen.

The polysaccharide-crosslinked colloidal particles of the present invention, which are used as drug modifiers or drug carriers, are characterized by being engineered with a hydrodynamic diameter of 2 to 10 nm, preferably 8 nm or less, more preferably 6 nm or less, and a surface charge in the range of -20 mV to 0 mV by exposing -COOH functional groups on the surface, such that they are filtered through the kidneys but do not permeate the vascular walls of normal blood capillaries, and are instead discharged exclusively via the lymphatic vessels.

When used as a drug modifier or drug carrier, the polysaccharide-crosslinked colloidal particles can extend the residence time at the site of local administration, thereby inducing expansion of the interstitial space, anatomical space distension, and/or lymph node enlargement at the administration site. Furthermore, the extended retention time at the targeted administration site can enhance the efficacy of the drug (see Examples 2 and 4).

In addition, the present invention enables control over the in vivo distribution and elimination of functional nanoparticles or drugs intended for in vivo administration, through modification by the polysaccharide cross-linked colloidal particles. Specifically, by tuning the hydrodynamic diameter and/or surface charge of the polysaccharide cross-linked colloidal particles, the pharmacokinetics of the functional nanoparticles or drugs can be regulated.

Since the polysaccharide-crosslinked colloidal particles of the present invention are designed with a hydrodynamic diameter of 10 nm or less, preferably 8 nm or less, more preferably 6 nm or less, and a surface charge ranging from -20 mV to 0 mV, they can be eliminated through urinary excretion following systemic circulation via renal filtration. As a result, more than 90% of the particles can be excreted within 48 hours, preferably within 24 hours.

The polysaccharide-crosslinked colloidal particles of the present invention are engineered to be easily excreted in urine and are not subject to hepatic metabolism, thereby exhibiting no hepatotoxicity.

For example, when the hydrodynamic diameter of the polysaccharide-crosslinked colloidal particles and/or the in vivo administrable complex is 10 nm or less, preferably 8 nm or less, more preferably 6 nm or less, and the surface charge is adjusted within the range of -20 mV to 0 mV, they can be filtered by the kidneys but do not permeate the vascular walls of normal blood capillaries and are discharged only through the lymphatic system. Accordingly, in the case of local administration, urinary excretion is facilitated, and the potential for adverse effects caused by prolonged retention or long-term accumulation can be mitigated (see Examples 3 and 4).

For example, by adjusting at least one parameter selected from the molecular weight of the polysaccharide (e.g., dextran or dextran derivatives such as carboxymethyl dextran), the length of the polysaccharide main chain, the type of crosslinker used during the crosslinking reaction, the amount and rate of crosslinker addition during synthesis, any subsequent chemical modification of functional groups, the molecular weight of the resulting polysaccharide cross-linked colloidal particles, and their surface charge, it is possible to control the final size and charge of the polysaccharide cross-linked colloidal particles. Through such control, the particles can be engineered to avoid or enable phagocytosis by macrophages, and to be excreted via either renal or hepatic clearance, resulting in elimination through urine or feces. Moreover, desirable circulation half-lives and tailored in vivo distribution and excretion pharmacokinetics may be achieved.

Additionally, the present invention allows modulation of the swelling degree of the polysaccharide-crosslinked colloidal particles through the adjustment of crosslinking density, thereby enabling control over the pharmacokinetics of in vivo administrable complexes that have been modified with such polysaccharide cross-linked colloidal particles. Furthermore, the in vivo administrable complexes modified with polysaccharide-crosslinked colloidal particles can be formulated such that the particles are embedded within the aqueous interior of predefined carriers-such as exosomes, liposomes, or polymeric micelles-that determine their in vivo behavior and/or distribution.

### [In vivo Structures Navigable in a Squeezable State]

The polysaccharide-crosslinked colloidal particles of the present invention are capable of passing within in vivo structures in a squeezable state and, therefore, can be used as a squeezable modifier for tissue penetration.

Non-limiting examples of in vivo structures through which the dextran-crosslinked nanoparticles and/or the in vivo administrable complexes modified therewith according to the present invention may migrate in a squeezable state include blood capillaries, lymphatic vessels, the gastrointestinal tract, and intercellular microspaces or intracellular matrices constituting various organ tissues.

In multicellular organisms, cells differentiate morphologically and functionally and are typically organized into homogeneous populations that perform specific functions; such organized cellular assemblies are referred to as "tissues."

Animal tissues are classified morphologically and functionally into epithelial tissue, connective tissue, cartilage, bone, blood and lymph, muscle tissue, and nervous tissue.

Epithelial tissue covers body surfaces and the luminal surfaces of digestive and respiratory tracts, as well as the cavities such as the peritoneal and pericardial cavities, forming continuous sheets of one or more layers of tightly packed cells with minimal intercellular space. In some cases, epithelial tissue invaginates to form glandular structures composed of secretory cells. Specialized epithelia also exist, including sensory epithelia for vision, hearing, and equilibrium, as well as structures such as hair and nails.

The four tissue types-connective tissue, cartilage, bone, and blood/lymph-are collectively referred to as supporting tissues. Supporting tissues contain abundant extracellular matrix, and connective tissue such as bone, cartilage serve to maintain the form and integrity of the body and internal organs. The extracellular matrix consists of fibers and ground substance, within which cells are embedded and scattered. Blood and lymph are considered supporting tissues because their plasma and lymph fluid serve as the ground substance, and fibrin as the fibrous component.

Muscle tissue is composed of contractile cells known as muscle fibers, which are elongated and slender in shape and capable of generating movement through contraction.

Nervous tissue consists of neurons and glial cells, and is responsible for the wired transmission of biological signals. In higher animals, the brain and spinal cord constitute the central nervous system (CNS), while the extensions from the CNS form the peripheral nervous system (PNS). The CNS is composed of nervous tissue supplemented by blood vessels and connective tissue, while the PNS comprises nerve cells, fibers, and Schwann cells (a type of glial cell) that ensheath the fibers.

In summary, the polysaccharide crosslinked colloidal particles and/or the in vivo administrable complexes of the present invention are squeezable and allow for precise modulation of their swelling degree, overall size, and/or surface charge. Therefore, they can be engineered to be mobile within various in vivo structures, including the extracellular matrix (ECM) of connective tissues forming blood capillaries, lymphatic vessels, gastrointestinal tracts, and various internal organs, and/or the interstitial spaces within such structures.

The total length of blood vessels in the human body is approximately 120,000 kilometers, circulating blood throughout the body in under a minute. Capillaries, which connect arterioles and venules, account for about 90% of all vasculature (approximately 95,000 km) and are connected to every organ, enabling the delivery of nutrients and oxygen. Capillaries have diameters of 30 to 40 µm, and the smallest ones are around 8 µm, allowing the passage of only a single red blood cell at a time. Structurally, they consist of a single layer of endothelial cells, facilitating efficient exchange of substances with adjacent cells.

The extracellular matrix (ECM) filled with various in vivo structures and interstitial fluid, the space between plasma and cells, provides a space for water, ions, and small solutes to continue to exchange across the gaps between cells that make up blood capillaries.

The gaps pores between endothelial cells of blood capillaries are estimated to be approximately 5-7 nm in diameter (~60 kDa), and this size can determine the permeability of different molecules or proteins of varying sizes. Thus, the ability of a drug, especially a nanoscale substance, to traverse the capillary wall significantly influences its systemic absorption and efficiency of targeted delivery.

In the case of the blood-brain barrier (BBB), the intercellular gaps between capillary endothelial cells are narrower and sealed by tight junctions compared to those in ordinary vasculature. Therefore, crossing the BBB requires highly selective permeability or active transport mechanisms. These characteristics allow the BBB to protect the brain and central nervous system from potential threats and pathogens in the bloodstream by isolating the cerebral environment.

Circulating blood is filtered through the kidneys, where waste is excreted into the bladder and eliminated via urine. The glomerular filtration barrier of the kidney typically permits the passage of substances approximately 6-8 nm in size. Although, in theory, materials within or below this size range should pass through, hard materials such as inorganic nanoparticles require a hydrodynamic diameter of 5.5 nm or less to effectively cross the glomerular barrier. Therefore, both size and rigidity of the substance are key factors in determining renal excretion.

Accordingly, the in vivo administrable complexes of the present invention, which are designed to be squeezable during movement within in vivo structures, may be administered intravenously and engineered for selective delivery to target tissues by controlling vascular permeability and systemic absorption. Furthermore, the renal excretion rate and clearance can be tuned to adjust the circulation time or systemic elimination level. This feature offers advantages in utilizing the polysaccharide crosslinked colloidal particles of the invention, particularly as part of in vivo administrable complexes, in the diagnosis and/or treatment of specific diseases.

### [Polysaccharide Crosslinked Colloidal Particles Using Branched or Cyclic Polysaccharides]

The polysaccharide crosslinked colloidal particles can be formed by crosslinking 1 to 3 branched polysaccharide molecules or 1 to 30 cyclic polysaccharide molecules, with one or more types of crosslinkers, in an aqueous solvent.

In this case, hydroxyl (-OH) groups of the monosaccharide building blocks comprising the branched or cyclic polysaccharides are modified with a first crosslinker, and then intramolecular and/or intermolecular crosslinking is induced between spatially adjacent modified functional groups, either directly or via a second crosslinker, to form the polysaccharide-crosslinked colloidal particles. When the first and/or second crosslinker is an amine-based crosslinker, amine groups derived from the crosslinker may remain exposed on the surface of the resulting colloidal particles.

As the molecular weight of the branched or cyclic polysaccharides used in the synthesis increases, the hydrodynamic size of the resulting polysaccharide-crosslinked colloidal particles also increases. Accordingly, the hydrodynamic diameter of the particles can be tuned within a range of 2 to 20 nm.

Polysaccharides are a group of compounds in which multiple monosaccharides of identical or different structures are linked by enzyme-sensitive glycosidic bonds, and are widely found in the cell walls of animals, plants, and microorganisms.

These polysaccharides may carry neutral, positive, or negative charges, may have linear or branched molecular structures, and may vary in molecular weight from several hundred to several thousand Daltons. Due to these physicochemical properties, polysaccharides significantly influence the biodistribution of carrier drug molecules in vivo.

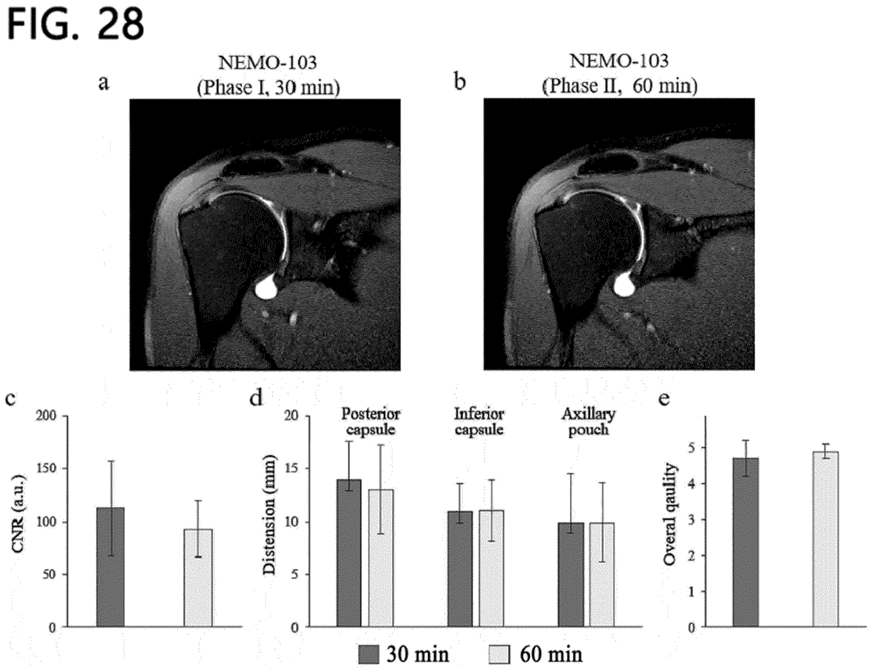

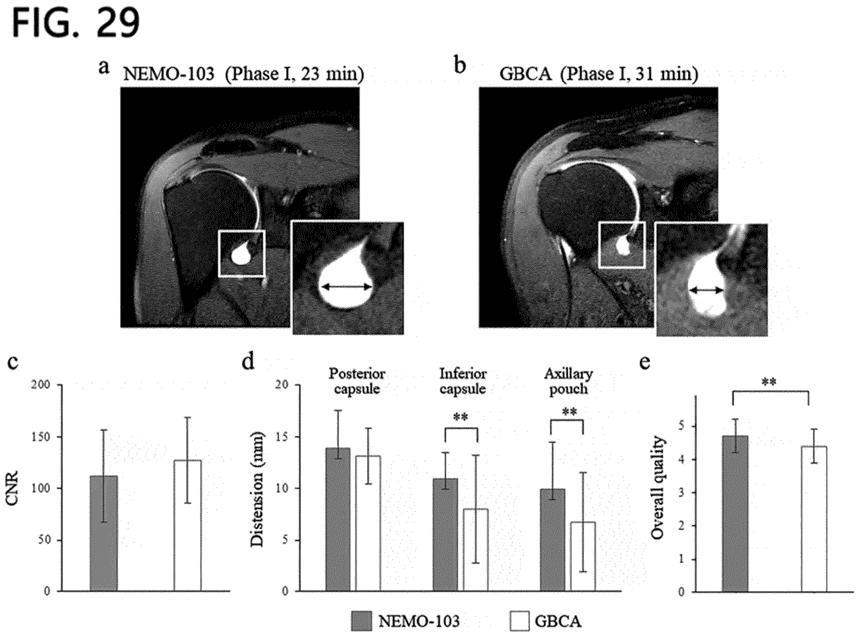

The polysaccharide-crosslinked colloidal particles synthesized using various branched or cyclic polysaccharides, when administered in vivo (e.g., via intravenous injection), were confirmed to be excreted in the urine through renal filtration after entering the vasculature of the nephrons via systemic circulation. This excretion was validated as an MRI signal observed in the bladder (see Example 3, FIGS. 28 and 29).

The branched or cyclic polysaccharides to be crosslinked may be homopolysaccharides or heteropolysaccharides.

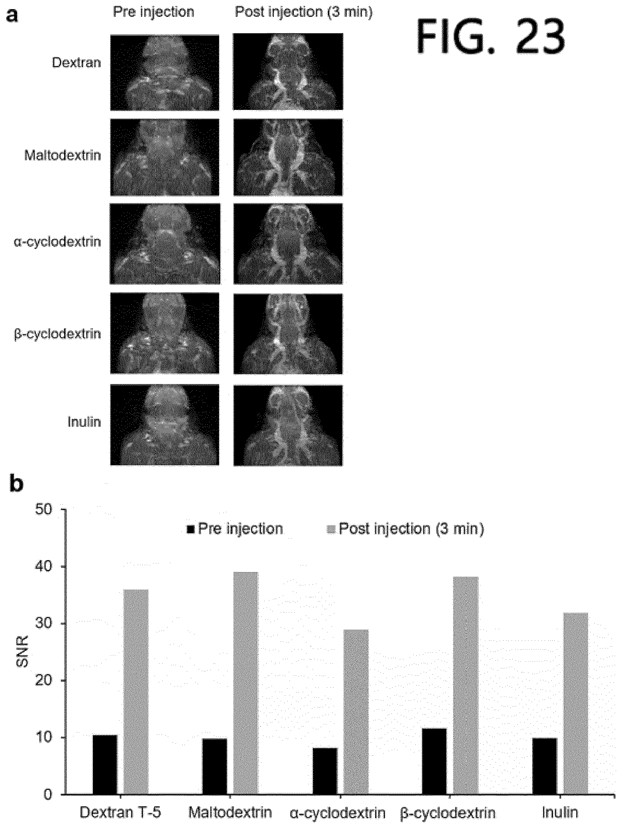

Non-limiting examples of branched or cyclic polysaccharides include, as illustrated in FIG. 23, dextran, cyclodextrin, maltodextrin, and inulin.

Dextran is a polysaccharide derived from the condensation of glucose and is a complex branched glucan, as shown in the following structural formula. It is a microbially derived branched poly-α-D-glucoside predominantly composed of glycosidic bonds at the C-1 → C-6 positions.

The main chain of the polymer is composed of α(1→6)-linked glucose monomers, and the branching points are connected via α(1→3) glycosidic bonds.

Dextran was initially discovered as a microbial product in wine, but its large-scale production became feasible only after processes using bacterial cultures were developed. Dextran is currently synthesized from sucrose by specific lactic acid bacteria belonging to the genus *Lactobacillus.*

Dextran is approved by the U.S. Food and Drug Administration (FDA) as a biocompatible material.

As used herein, the term "dextran" includes various derivatives thereof. Non-limiting examples of dextran derivatives include carboxymethyl dextran (CM-dextran), dextran sulfate, and diethylaminoethyl dextran (DEAE-dextran).

There is an increasing industrial demand for dextran molecules of various specific molecular weights for diverse applications. For example, dextran with a molecular weight ranging from 70,000 to 100,000 Da is used as a plasma substitute. Dextran of approximately 40,000 Da is most likely used to improve blood circulation by reducing blood viscosity and inhibiting erythrocyte aggregation. Smaller dextran sulfates, such as those around 10,000 Da, are used as iron transport agents or anticoagulants.

Inulin is an energy-storage polysaccharide found in plants of the *Compositae* family and consists of a linear chain of fructosyl groups linked by β-2,1 glycosidic bonds and terminated by an α-D-1,2 glucopyranoside ring at the reducing end. Inulin is used as an excipient in pharmaceutical formulations under the GRAS regulatory classification, and its water-soluble form is utilized to test renal glomerular filtration. A specific semicrystalline particulate form known as delta inulin exhibits immunomodulatory properties and has been used as a vaccine adjuvant. Although inulin is not digestible by humans, it can be metabolized by bifidobacteria in the gut, providing not only a probiotic effect but also additional benefits such as colon-targeted drug delivery. Modifications of inulin for medical use typically involve periodate oxidation followed by functionalization via amination reactions or reactions with anhydrides.

The polysaccharide crosslinked colloidal particles of the present invention, which are formed by intramolecular and/or intermolecular crosslinking of 1 to 3 branched polysaccharides or 2 to 30 cyclic polysaccharides via a crosslinker at the hydroxyl (-OH) groups of their constituent monosaccharide building blocks, may be regarded as a type of drug, in which the synthesis method and resulting physicochemical properties-such as size, surface charge, and morphology-can be precisely controlled.

According to the present invention, the polysaccharide crosslinked colloidal particles used as modifiers for substances intended for in vivo administration are formed by intramolecular and/or intermolecular crosslinking of 1 to 3 complex branched polysaccharides or 2 to 30 cyclic polysaccharides in an aqueous medium via the hydroxyl groups of their monosaccharide building blocks using a crosslinker, thereby enabling various improvements over conventional polymer coatings widely used for nanomaterials and pharmaceutical agents (see Examples 1-1 to 1-13 and FIG. 8).

According to the present invention, polysaccharide-crosslinked colloidal particles compactly formed via intramolecular and/or intermolecular crosslinking of complex branched or cyclic polysaccharides in an aqueous solution at the hydroxyl groups of their monosaccharide building blocks can be readily controlled to exhibit desired hydrodynamic size and swelling degree. As such, they allow predictable modulation of mobility within biological fluids and can be engineered to be squeezable for transport within various anatomical structures. For example, in the polysaccharide-crosslinked colloidal particles of the present invention, the degree of substitution of the crosslinker may be controlled to 2% to 50% of the available functional groups on the polysaccharide, and 2% to 98% of the crosslinker molecules may be configured such that one terminal group does not participate in crosslinking and remains exposed on the outer surface. Through such control, the swelling behavior and compressibility of the colloidal particles can be precisely tuned as desired.

Additionally, by adjusting at least one of the following parameters-namely, the molecular weight of the polysaccharide, the length of the polysaccharide backbone, the type of crosslinker used during crosslinking, the amount and rate of crosslinker administration during synthesis, and the type and degree of subsequent functional group modification-it is possible to finely control the overall size and surface charge of the resulting polysaccharide-crosslinked colloidal particles and/or the in vivo administrable complexes thereof, thereby imparting desired blood circulation time, biodistribution, and excretion pharmacokinetics.

For renal excretion, the average molecular weight of the dextran or dextran derivatives used may be 10,000 Da or less, and the molecular weight of spherical dextran-crosslinked nanoparticles formed by crosslinking dextran-based molecules through a crosslinker may be 35,000 Da or less.

The polysaccharide-crosslinked colloidal particles of the present invention are nanoparticles in which complex branched polysaccharides are intramolecularly and/or intermolecularly crosslinked at the -OH functional groups of their monosaccharide building blocks in an aqueous solution. The branched polysaccharides may form dimers or trimers through intermolecular crosslinking, or a single branched polysaccharide molecule may be intramolecularly crosslinked via a crosslinker to form the colloidal particle. The degree of crosslinking and/or the molecular weight of the branched polysaccharide can be adjusted to control the compressibility, and preferably, the particles may be compact and spherical.

In particular, polymer solutions increase in viscosity with increasing concentration, and this also applies to aqueous solutions containing polysaccharide-crosslinked colloidal particles. According to the Mark-Houwink-Sakurada (MHS) equation, which relates the viscosity of a polymer solution to the molecular weight of the polymer, it is well known that viscosity is directly proportional to molecular weight. Excessive viscosity in injectable formulations may increase the risk of vascular occlusion or vascular damage due to high injection pressure. Therefore, for drug formulations utilizing the dextran-crosslinked nanoparticles as modifiers for intravenous administration, it is preferable to use dextran-based molecules with an average molecular weight of 10,000 Da or less, more preferably around 5,000 Da or less. When such low-molecular-weight dextran molecules are crosslinked to form spherical dextran-crosslinked nanoparticles with a molecular weight of approximately 15,000 Da or less, an injectable formulation with appropriate viscosity for in vivo administration can be achieved.

For example, when the average molecular weight of the branched or cyclic polysaccharides is adjusted to be relatively low, the resulting polysaccharide-crosslinked colloidal particles of the present invention can be used to prepare the in vivo administrable complex at high concentration, thereby allowing precise control of the formulation viscosity through dilution adjustment, and enabling a reduction in the injection volume relative to the desired dose of the functional nanoparticles or therapeutic agents to be administered.

For instance, when dextran-based molecules with an average molecular weight of 10,000 Da or less are crosslinked in an aqueous phase at the -OH groups of glucose building blocks, 1 to 3 dextran-based molecules are crosslinked intramolecularly and/or intermolecularly to form spherical nanoparticles. Accordingly, dextran-crosslinked nanoparticles prepared from dextran-based molecules with an average molecular weight of 10,000 Da or less may have a molecular weight of 35,000 Da or less. The in vivo administrable complex modified therewith may be engineered as a nanostructure having a hydrodynamic diameter of 10 nm or less, preferably 5 nm or less, for renal excretion. Generally, a nanostructure must have a hydrodynamic diameter of 6 to 8 nm or less in order to be naturally excreted through the kidney.

Therefore, by designing various synthesis methods for polysaccharide-crosslinked colloidal particles including dextran-crosslinked nanoparticles, and applying these particles for various modifications, it is possible not only to control the biodistribution and excretion profile of the target functional nanoparticles or drugs without causing immune rejection or toxicity, but also to ensure that, in certain cases, even if the target materials bound to the polysaccharide-crosslinked colloidal particles are co-excreted, they remain stable in plasma without aggregation, are not metabolized or degraded in vivo, and can be recovered from urine or feces for potential reuse. In certain embodiments, the drug may be released or separated from the polysaccharide-crosslinked colloidal particle after in vivo administration, and each component may follow a distinct excretion pathway. In such cases, the separated polysaccharide-crosslinked colloidal particle may remain unmetabolized or undegraded in the body and may be excreted via urine or feces and subsequently recovered for reuse. For example, the polysaccharide-crosslinked colloidal particles dissociated from the in vivo administrable complex after administration may be absorbed into the circulatory system and excreted through the kidneys into the urine without extravasation through the vascular wall.

In the present invention, the functional groups derived from the crosslinker and exposed on the surface of the polysaccharide-crosslinked colloidal particles may be the terminal functional groups of the crosslinker itself or modified/substituted versions thereof. For example, the crosslinker-derived functional groups exposed on the surface may include functional groups in which at least a portion of the terminal groups of the crosslinker are substituted with other functional groups.

In the present invention, the hydrophilic functional groups may be derived from: (i) functional groups of branched or cyclic polysaccharides that did not participate in the crosslinking reaction; (ii) terminal functional groups of crosslinkers that did not participate in crosslinking; and/or (iii) terminal groups of crosslinkers exposed after the reaction and subsequently modified.

Non-limiting examples of crosslinker-derived or hydrophilic functional groups exposed on the surface of the polysaccharide-crosslinked colloidal particles include amino groups, carboxyl groups, hydroxyl groups, and/or thiol groups. Reactive functional groups such as amine, thiol, carboxyl, and hydroxyl groups facilitate not only surface modification but also chemical conjugation with biopharmaceuticals and various types of small-molecule drugs, including ligands that specifically bind to cell receptors, antibodies or antibody fragments, antigenic peptides, and nucleic acids (DNA, RNA, or their fragments). In some cases, they may be cleaved in the acidic tumor microenvironment (pH ≤ 7) or by hydrolytic enzymes, thereby releasing active drug compounds.

Non-limiting examples of acid-labile linkages that can be cleaved under acidic conditions (pH ≤ 7), such as those found in tumor tissues, include carbonate and ester bonds.

For example, a polysaccharide-crosslinked colloidal particle-drug complex, in which a drug payload is conjugated to the particle via a cleavable linker, may include a peptide linker susceptible to cleavage by: (i) extracellular matrix (ECM)-degrading enzymes such as matrix metalloproteinases (MMPs), which are secreted by tumor cells during local invasion and metastasis; or (ii) intracellular hydrolytic enzymes that cleave peptide bonds.

Furthermore, the in vivo administrable complex of the present invention may comprise molecules exhibiting targeting functionality (hereinafter, 'targeting molecules') that are conjugated via (i) crosslinker-derived surface functional groups of the polysaccharide-crosslinked colloidal particles and/or (ii) surface functional groups of the functional nanoparticles or drugs. The targeting molecules may include, but are not limited to, antibodies or their antigen-binding fragments thereof, repebodies, and aptamers, thereby enabling the in vivo administrable complex to emulate the platform technology of antibody-drug conjugates (ADCs).

When the exposed functional group on the surface of the polysaccharide-crosslinked colloidal particles is positively charged (e.g., an amine group), cytotoxicity similar to that of other cationic polymers may arise. This issue can be mitigated by substituting some or all of the amine groups with carboxyl, methyl, or ethyl groups.

Non-limiting examples of hydrophilic functional groups capable of forming coordination bonds with metal ions (e.g., iron ions) include amine, thiol, carboxyl (carboxylate and carboxylic acid), and hydroxyl groups.

The terminal hydroxyl, amine, and non-coordinating carboxylate groups of the polysaccharide-crosslinked colloidal particles confer water solubility to in vivo administrable complex of the present invention. Accordingly, hydration of the hydrophilic groups exposed on the surface of the polysaccharide-crosslinked colloidal particles enhances the dispersion stability of the in vivo administrable complex in body fluids. As a result, in vivo administrable complex modified with the polysaccharide-crosslinked colloidal particles can remain stably dispersed in body fluids without precipitation or aggregation, even when a large amount of drug is conjugated, thereby retaining their intended function.

Furthermore, the present invention enables the surface charge of the polysaccharide-crosslinked colloidal particles and that of the modified target substance (e.g., a functional nanoparticle or drug) to be freely controlled by adjusting the type and/or degree of substitution of the functional groups derived from the crosslinker and exposed on the surface of the particles.

In addition, by precisely tuning the molecular weight, size, and surface charge of the polysaccharide-crosslinked colloidal particles, the overall size and charge of the particles or the resulting in vivo administrable complexes can be tailored to meet the desired pharmacokinetic profile, including blood circulation time, renal clearance, and hepatic clearance.

According to the present invention, the polysaccharide-crosslinked colloidal particles obtained via crosslinking reactions and, optionally, further modification of the terminal groups of the crosslinkers exposed after the reaction, can bind to functional nanoparticles or drugs for in vivo adminstration through coordination bonding, covalent bonding, hydrogen bonding, and/or electrostatic interactions via the functional groups exposed on their surface. In particular, the ability of the crosslinker-derived functional groups on the surface of the polysaccharide-crosslinked colloidal particles to coordinate with metals facilitates the surface modification of various functional metal nanoparticles that are otherwise difficult to functionalize, thereby allowing the tuning of their physicochemical properties. For example, divalent or trivalent iron ions may coordinate with carboxylic acid or carboxylate groups derived from the crosslinker on the surface of the polysaccharide-crosslinked colloidal particles. Therefore, the polysaccharide-crosslinked colloidal particles of the present invention can be used to functionalize the surface of metal or metal oxide nanoparticles (e.g., iron oxide nanoparticles) via coordination or covalent bonding.

### [Composition for Modifying a Nanoparticle or Drug for In Vivo Administration, and Method for Preparing the Same]

According to the present invention, a composition for modifying a target substance for in vivo administration comprises a polysaccharide-crosslinked colloidal particle formed by intra- and/or inter-molecular crosslinking of 1 to 3 branched polysaccharides or 2 to 30 cyclic polysaccharides at -OH functional groups of the constituent monosaccharide building blocks in an aqueous solution using a crosslinker.

Meanwhile, the modification composition comprising the polysaccharide-crosslinked colloidal particle and the in vivo administrable complex modified therewith may, in a non-limiting example, be prepared by a method comprising the following steps, and may be extended to use branched or cyclic polysaccharides instead of dextran or dextran derivatives (see Example 3):
Step 1 of preparing an aqueous solution of dextran or a dextran derivative;
Step 2 of dropwise adding an epoxide and an alkaline aqueous solution at room temperature;
Step 3 of dropwise adding divalent or higher-valent polyamines at room temperature to form dextran-crosslinked nanoparticles bearing terminal amine groups;
Step 4 of precipitating the resulting product;
Step 5 of redispersing the precipitated product in water;
   optionally, Step 6 of recovering the dextran-crosslinked nanoparticles bearing terminal amine groups by dialysis;
Step 7 of adding an organic acid anhydride to the dextran-crosslinked nanoparticles bearing terminal amine groups, so as to substitute at least a portion or all of the terminal amine groups with carboxylic acid and/or carboxylate groups;
   optionally, Step 8 of purifying a solution of dextran-crosslinked nanoparticles bearing carboxylic acid and/or carboxylate groups to obtain water-dispersible dextran-crosslinked nanoparticles;
   optionally, Step 9 of adding an iron precursor (e.g., iron chloride) or an aqueous solution of iron oxide nanoparticles to the water-dispersible dextran-crosslinked nanoparticles prepared in the previous step, to obtain (i) dextran-crosslinked nanoparticles having a shell formed of divalent or trivalent iron ions, or (ii) complexes in which the surface of iron oxide nanoparticles is surface-modified with the dextran-crosslinked nanoparticles; and
   optionally, Step 10 of purifying or concentrating the nanostructures obtained in the previous step by ultrafiltration.

The number of dextran molecules subjected to crosslinking in the dextran-crosslinked nanoparticles can be controlled to be the same or different, depending on the synthesis conditions and/or purification process.

In Step 2, the epoxide used to modify the hydroxyl groups of the glucose building blocks is not particularly limited as long as it enables subsequent reaction with a crosslinker. Preferably, the epoxide is a halo alkyl oxirane, such as epichlorohydrin.

In Step 3, the polyamine may be replaced with any crosslinker capable of forming a covalent bond with the epoxide-derived functional groups that have modified the hydroxyl groups of the glucose building blocks. Such variants also fall within the scope of the present invention.

Steps 2 and 3 are economically advantageous because the crosslinking reaction at the hydroxyl groups of the glucose building blocks proceeds without requiring any additional expensive catalyst.

In Step 4, a large amount of an organic solvent having a dielectric constant between 15 and 50 may be added to induce precipitation of the product.

According to the present invention, an in vivo administrable complex in which a functional nanoparticle or drug is modified with a polysaccharide-crosslinked colloidal particle is also a type of drug that must ultimately be eliminated from the body. Once administered into the body, the drug elimination process begins. The primary pathways for drug elimination include (1) hepatic metabolism, (2) biliary excretion, and (3) urinary excretion. Elimination encompasses both biotransformation (i.e., drug metabolism) and excretion. Excretion refers to the removal of the intact drug from the body. A high proportion of nanoparticles retained in organs such as the liver and spleen indicates unsatisfactory excretion performance.

For example, the polysaccharide-crosslinked colloidal particles of the present invention can be designed and synthesized such that, upon in vivo administration, they are predominantly collected in the urine without accumulation in normal tissues, thereby enabling rapid elimination from the body exclusively via the urinary excretion pathway, without undergoing hepatic metabolism or biliary excretion. Alternatively, depending on the intended use, they may be designed and synthesized to avoid urinary excretion and instead follow hepatic metabolism and biliary excretion routes.

Accordingly, the in vivo administrable complex modified with the polysaccharide-crosslinked colloidal particle according to the present invention can be engineered such that (i) it remains stable without aggregation in physiological pH-buffered solutions and blood plasma, (ii) it is not phagocytosed by macrophages by adjusting the surface charge to be neutral (i.e., close to zero), and (iii) it is excreted via the urinary excretion pathway by precisely designing and synthesizing the polysaccharide-crosslinked colloidal particle to have a compact hydrodynamic diameter smaller than the renal filtration threshold. Therefore, the polysaccharide-crosslinked colloidal particle and the in vivo administrable complex comprising the same may be collected in the urine at a recovery rate of 80% or more, preferably 90% or more, and more preferably 95% or more relative to the administered dose.

In Step 9, non-limiting examples of hydrophilic functional groups that can coordinate with iron ions include hydroxyl, carboxylic acid, carboxylate, and amine groups.

In addition, all disclosures of Korean Patent Application No. 10-2021-0104405 are hereby incorporated into the present invention and this specification in their entirety, particularly with respect to dextran-crosslinked nanoparticles having a shell composed of divalent or trivalent iron ions.

The polysaccharide-crosslinked colloidal particles having a shell composed of divalent or trivalent iron ions, as engineered through Step 9, may be designed and synthesized to function as T1 MRI contrast agents that generate bright signals in MRI imaging. Accordingly, after administration in vivo, the position of the in vivo administrable complexes modified with the polysaccharide-crosslinked colloidal particles may be tracked by MRI imaging.

The polysaccharide-crosslinked colloidal particles of the present invention may be engineered and synthesized to prolong the retention of contrast enhancement during MRI, thereby enabling longer scan times and enhancing the spatial resolution of MRI. This feature allows high-resolution imaging of the entire vascular system in vivo. Accordingly, the polysaccharide-crosslinked colloidal particles of the present invention are capable of visualizing microvasculature that is clinically significant but difficult to observe using conventional contrast agents. For example, when imaging the rat brain using dextran-crosslinked nanoparticles at a spatial resolution of 0.078 mm × 0.078 mm × 0.078 mm, microvasculature with a thickness of approximately 0.078 mm could be clearly visualized. Given that the spatial resolution of the most commonly used 3 Tesla (3T) MRI scanners in clinical settings is approximately 1 mm, the resolution achieved using the dextran-crosslinked nanoparticles of the present invention represents an approximately 13-fold improvement.

In summary, the polysaccharide-crosslinked colloidal particles of the present invention may serve as T1 or T2 MRI contrast agents when divalent or trivalent iron ions are coordinated to the crosslinker-derived functional groups exposed on the particle surface.

In addition, iron oxide nanoparticles engineered to function as T1 or T2 MRI contrast agents may be surface-modified with dextran-crosslinked nanoparticles in Step 9. This modification renders the nanoparticles squeezable, enabling them to move within in vivo structures without aggregation upon in vivo administration, thereby maintaining their functionality as T1 or T2 MRI contrast agents (see Example 1-17, FIG. 11).

Therefore, the in vivo administrable complexes modified with polysaccharide-crosslinked colloidal particles according to the present invention can serve as MRI contrast agents. After administration in vivo, their location can be monitored by MRI imaging to assess: phagocytosis by macrophages, metabolic degradation, circulation in the bloodstream, delivery to parenchymal cells via blood capillaries, tissue accumulation, renal excretion into urine, excretion into feces, absorption into the vascular system, leakage through vessel walls, potential for recovery and reuse via excretion, and intracellular uptake.

### ["Stealth" Effect]

The polysaccharide-crosslinked colloidal particles of the present invention can be used as a modifier that imparts a "stealth" effect, similar to PEGylation of drugs, and drugs (including functional nanoparticles) modified with the polysaccharide-crosslinked colloidal particles of the present invention can evade immune recognition, thereby enhancing the delivery and efficacy of diagnostic and/or therapeutic agents.

Because the polysaccharide-crosslinked colloidal particles of the present invention are hydrophilic (water-attracting) and can form a hydrated shell around the particle, the in vivo administrable complex of the present invention can be produced by coupling functional nanoparticles or drugs intended for in vivo administration to surface-exposed crosslinker-derived functional groups of the polysaccharide-crosslinked colloidal particles in an aqueous sovent, and can exhibit a stealth effect during in vivo distribution without aggregation.

When the surface of a functional nanoparticle is modified with the polysaccharide-crosslinked colloidal particle of the present invention, or when a hydrophobic drug with potential for aggregation in vivo is distributed on the surface of the polysaccharide-crosslinked colloidal particle via covalent or coordination bonds, the number of drugs bound per unit particle is preferably adjusted to 5 or fewer, such that immune recognition and clearance by the immune system are reduced due to the stealth effect, thereby increasing both the efficacy and duration of the drug within the body.

When the polysaccharide-crosslinked colloidal particle of the present invention is attached to the surface of a functional nanoparticle, or when a hydrophobic drug is distributed on the surface of the polysaccharide-crosslinked colloidal particle via covalent or coordination bonding, the polysaccharide-crosslinked colloidal particle extends outward from the in vivo administrable complex of the present invention and attracts surrounding water molecules. The polysaccharide-crosslinked colloidal particle of the present invention forms a hydration layer around the in vivo administrable complex or the functional nanoparticle, which constitutes the core mechanism of the "stealth" effect.

The polysaccharide-crosslinked colloidal particles of the present invention exert a "stealth" effect through the following mechanisms of action:
(1) Reduced opsonization: Opsonization is the process by which particles are marked for clearance by the immune system. The hydration layer formed by the polysaccharide-crosslinked colloidal particles of the present invention inhibits the binding of opsonins-proteins such as antibodies and complement factors-to the particle surface, thereby reducing opsonization.
(2) Avoidance of phagocytosis: By reducing opsonization, the polysaccharide-crosslinked colloidal particles help the in vivo administrable complex or the functional nanoparticles of the present invention avoid recognition and engulfment by phagocytes, a type of immune cell responsible for consuming foreign particles.
(3) Prolonged circulation time: Stealth particles can remain in systemic circulation for an extended period without being recognized and eliminated by the immune system. Moreover, due to their tunable size and squeezable characteristics, the extravasation or vascular penetration behavior of these particles can be controlled, thereby enabling longer circulation times and enhancing targeted drug delivery.
(4) Minimized immunogenicity: The polysaccharide-crosslinked colloidal particles of the present invention reduce the overall immunogenicity of the in vivo administrable complex or functional nanoparticles, thereby lowering the likelihood of triggering immune responses. This is particularly important for preventing rapid clearance of subsequent doses due to immune recognition following repeated administration.
(5) Reduced recognition by the mononuclear phagocyte system (MPS): The MPS, primarily located in the liver and spleen, is responsible for clearing foreign materials from the bloodstream. The polysaccharide-crosslinked colloidal particles of the present invention are better able to evade recognition by this system.

In summary, due to the stealth effect of the polysaccharide-crosslinked colloidal particle of the present invention as described above, the in vivo administrable complex of the present invention exhibits reduced clearance and extended circulation time. As a result, the drug can reach and act on the target site (enhanced drug efficacy), the prolonged systemic circulation permits a reduction in dosing frequency and amount (lower dosage requirement), and the minimized interaction with the immune system may reduce immune-related side effects (reduced adverse effects).

### [Use of Polysaccharide-Crosslinked Colloidal Particles as in Vivo Administrable Modifiers]

The polysaccharide-crosslinked colloidal particles of the present invention can be used to modify the surface of functional nanoparticles or the drugs through crosslinker-derived functional groups exposed on their surface, such as a controlled number of amine groups and/or - COOH groups.

The polysaccharide-crosslinked colloidal particles of the present invention can provide a drug modality that is drained into lymphatic vessels upon injection into a tissue site, rather than via intravenous administration.

The squeezable polysaccharide-crosslinked colloidal particles of the present invention are capable of binding to target substances via the surface-exposed crosslinker-derived functional groups, and thus may be used as modifiers for target substances such as functional nanoparticles or drugs, as exemplified below.

### 1. Modifiers for In Vivo Introduction / Administration

In this specification, the terms "in vivo introduction" and "in vivo administration" are used interchangeably.

The term "body fluid" in this specification refers to an in vivo environment, and includes, for example, extracellular fluids such as blood, lymph, and interstitial fluid, as well as intracellular fluids.

The polysaccharide-crosslinked colloidal particles of the present invention can function as in vivo administrable modifiers by imparting or enhancing dispersion stability and colloidal stability in body fluids to the target substances ,such as functional nanoparticles or drugs, via hydration of hydrophilic functional groups exposed on their surface. Accordingly, by utilizing the polysaccharide-crosslinked colloidal particle platform technology, which allows for precise control of various physicochemical properties, it is possible to regulate, for example, the blood concentration of functional nanoparticles or drugs within an appropriate range.

### 2. Colloidal Stability-Imparting Modifier

Colloidal stability is a critical requirement for the biomedical application of nanomaterials. When the surface of nanomaterials-such as hydrophobic drugs or functional nanoparticles-becomes unstable under physiological conditions (e.g., salt concentration, pH), they may undergo aggregation driven by van der Waals forces. Such aggregation may undesirably alter the physicochemical properties or pharmacokinetic profile of the nanomaterials, or result in their deposition in tissues and subsequent failure of clearance.

In contrast, in vivo administrable complexes in which functional nanoparticles or drugs are modified with the polysaccharide-crosslinked colloidal particles of the present invention can improve colloidal stability in biological fluids through the hydration of hydrophilic functional groups exposed on the surface of the polysaccharide-crosslinked colloidal particle.

As demonstrated in FIG. 8, in vivo administrable complexes comprising functional nanoparticles or drugs modified with the polysaccharide-crosslinked colloidal particles of the present invention exhibited superior dispersion stability in solution under various salt concentrations and pH conditions, compared to control complexes modified with conventional dextran or dextran derivatives. Furthermore, as shown in FIG. 9, these complexes also maintained colloidal stability in blood.

Therefore, the polysaccharide-crosslinked colloidal particles of the present invention can be used as colloidal stability imparting modifiers for in vivo administration of target substances.

### 3. Hydrodynamic Size Modifier

The pharmacokinetic properties in vivo of the in vivo administrable complex, in which nanomaterials such as functional nanoparticles or drugs are modified with the polysaccharide-crosslinked colloidal particles of the present invention, are determined not by the intrinsic size of the nanomaterials themselves, but by the total hydrodynamic diameter that includes the coating layer of the polysaccharide-crosslinked colloidal particles after modification.

Accordingly, the polysaccharide-crosslinked colloidal particles of the present invention can be used as hydrodynamic size modifiers for target substances such as functional nanoparticles or drugs.

By employing polysaccharide-crosslinked colloidal particles precisely engineered to have a desired hydrodynamic diameter, the present invention enables fine-tuning of the total hydrodynamic diameter of the in vivo administrable complex modified with the polysaccharide-crosslinked colloidal particles through adjustment of the molecular weight of the colloidal particles.

For example, the total hydrodynamic diameter of the in vivo administrable complex of the present invention can be regulated by using polysaccharide-crosslinked colloidal particles having various molecular weights, thereby adjusting the hydrodynamic size of the particles (see Examples 1-1 to 1-4, Example 1-10, and FIG. 5).

In addition, in the in vivo administrable complex in which functional nanoparticles or drugs are modified with the polysaccharide-crosslinked colloidal particles of the present invention, the colloidal particles that contribute to the total hydrodynamic diameter are spherical nanoparticles formed by intramolecular and/or intermolecular crosslinking of 1 to 3 branched polysaccharides or 2 to 30 cyclic polysaccharides in an aqueous solvent via -OH groups of their monosaccharide building blocks with a crosslinker. In the polysaccharide-crosslinked colloidal particles of the present invention, the crosslinker substitution ratio can be controlled in the range of 2% to 50% relative to the number of functional groups on the polysaccharides. Further, 2% to 98% of the crosslinkers may be configured such that one terminal end is not involved in crosslinking and remains exposed on the surface, thereby enabling precise control over the swelling degree of the colloidal particles. Accordingly, not only the polysaccharide-crosslinked colloidal particles themselves, but also the in vivo administrable complexes modified therewith, can be engineered to have a uniform size distribution.

Accordingly, the present invention enables the imparting of desired in vivo pharmacokinetic properties to in vivo administrable complexes modified with the polysaccharide-crosslinked colloidal particles by precisely controlling the type and molecular weight of the branched or cyclic polysaccharides used in the modifier, and/or the type and amount of the crosslinkers.

Nanoparticles exhibit size-dependent organ-specific uptake and biodistribution in the human body. In particular, nanoparticles with a diameter of 50 nm or greater are rapidly sequestered by Kupffer cells in the liver, leading to their predominant accumulation in hepatic tissue.

However, even small nanoparticles may undergo unintended aggregation in the absence of adequate dispersibility, which can result in extensive clearance by the reticuloendothelial system (RES).

In this regard, in an in vivo administrable complex in which functional nanoparticles or drugs are modified with the polysaccharide-crosslinked colloidal particles according to the present invention, the colloidal particles expose hydrophilic functional groups on their surface to the aqueous environment. As a result, the nanoparticles do not aggregate in vitro or in vivo, and exhibit excellent dispersibility, colloidal stability, and/or storage stability. This leads to rapid and uniform distribution in the bloodstream and contributes to consistent therapeutic effects due to uniform particle size. Moreover, the complex maintains a uniform hydrodynamic diameter of 100 nm or less, which reduces phagocytic uptake by the hepatic reticuloendothelial system (RES) and thereby prolongs the circulation time in blood. In some cases, the complex may be engineered to avoid systemic accumulation and instead be excreted through renal filtration without undergoing hepatic metabolism.

For example, nanoparticles with a hydrodynamic diameter of approximately 1 to 30 nm may circulate in the blood for an extended period without being phagocytosed. Furthermore, to enable renal excretion via the kidneys, the particle diameter generally must be 6 to 8 nm or smaller. If the diameter exceeds this range, renal excretion becomes difficult. Accordingly, when the in vivo administrable complex in which functional nanoparticles or drugs are modified with the polysaccharide-crosslinked colloidal particles according to the present invention is intended for renal excretion via urine, the polysaccharide-crosslinked colloidal particles can be engineered to have a total hydrodynamic diameter of 8 nm or less, preferably 6 nm or less, and a uniform size distribution (see FIG. 11A).

Conversely, when renal excretion via urine is suppressed, the circulation time in the bloodstream can be extended, thereby maintaining high blood drug concentrations for a prolonged period. Additionally, it can enhance delivery efficiency to the reticuloendothelial system (RES), thereby improving drug delivery to RES-enriched organs such as the liver and spleen. When the in vivo administrable complex in which functional nanoparticles or drugs are modified with the polysaccharide-crosslinked colloidal particles according to the present invention is designed to prolong circulation time in the bloodstream or increase delivery efficiency to the RES, it may be engineered to have a total hydrodynamic diameter of 8 nm or greater with a uniform size distribution (see FIGS. 11B and 11C).

When the polysaccharide-crosslinked colloidal particles are designed and synthesized to maintain colloidal stability through hydration in biological fluids without undergoing opsonization, the in vivo administrable complex in which functional nanoparticles or drugs are modified therewith may be configured to remain undegraded in vivo, be instead filtered through the vasculature to the kidney and collected in the bladder, enabling the majority of the complex to be excreted via urine (see FIG. 11A). Accordingly, the complex may not only be recyclable but may also be utilized to collect information from tissues at the administration site or from the bloodstream via adsorption or other means. This is based on the observation that, in the in vivo administrable complex in which functional nanoparticles or drugs are modified with the polysaccharide-crosslinked colloidal particles according to the present invention, the polysaccharide-crosslinked colloidal particles exhibit excellent colloidal stability without aggregation, thereby maintaining a compact hydrodynamic diameter smaller than the renal filtration threshold and demonstrating superior excretion capacity in vivo. For example, such a complex can be precisely engineered to have an average hydrodynamic size of 10 nm or less, preferably 8 nm or less, while maintaining colloidal stability without aggregation in vivo, thus enabling complete renal excretion without structural alteration.

### 4. Surface Charge Modifier

The polysaccharide-crosslinked colloidal particles of the present invention can be used as surface charge modifiers for target substances such as functional nanoparticles or drugs.

The surface characteristics of the polysaccharide-crosslinked colloidal particles can be finely controlled by adjusting the type and/or density of surface-exposed functional groups. Accordingly, the in vivo administrable complex in which functional nanoparticles or drugs are modified with the polysaccharide-crosslinked colloidal particles of the present invention can also have its surface properties precisely engineered.

Meanwhile, the surface modification efficiency can be estimated by measuring the chemical quantity of the functional groups, the increase in surface charge, or the enhancement in surface hydrophilicity. One method commonly used to assess surface modification is to measure the zeta potential of an aqueous suspension containing nanoparticles. The zeta potential reflects the surface potential of the particles and is influenced by both the composition of the particles and the properties of the dispersion medium. The primary reason for measuring zeta potential is to predict colloidal stability. Interparticle interactions play a critical role in determining colloidal stability. Zeta potential measurements provide a quantitative evaluation of such interactions. Zeta potential is an index of the repulsive forces between particles, and most aqueous colloidal systems are stabilized by electrostatic repulsion. The greater the repulsive force between particles, the less likely they are to approach each other and form aggregates. Because surface charge can prevent nanoparticle aggregation, nanoparticles having a zeta potential of +10 mV or greater are generally considered stable in aqueous solutions. However, nanoparticles bearing high surface charge may exhibit reduced stability in electrolyte-rich solutions, such as biological fluids, due to ion-mediated aggregation. It has been reported that when amines (positively charged) and carboxylates (negatively charged) coexist on the surface, forming a zwitterionic state in which the net zeta potential is near neutral, the nanoparticles remain stable both in aqueous solutions and under physiological conditions such as in blood.

Furthermore, upon in vivo administration, nanomaterials interact with biomolecules such as proteins via electrostatic or non-specific binding mediated by their surface charge. Such interactions may result in the formation of protein coronas, which increases the likelihood of recognition by immune cells. Accordingly, the ability to modulate the surface charge of nanomaterials is crucial.

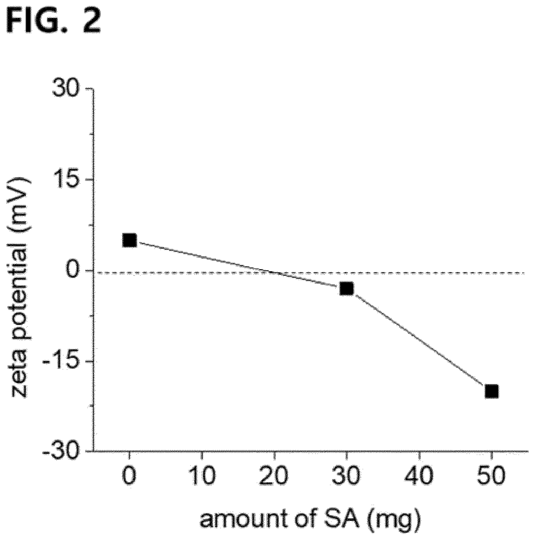

As described above, the polysaccharide-crosslinked colloidal particles of the present invention allow for precise control of surface charge by modulating the type and/or density of crosslinker-derived terminal functional groups exposed on the particle surface (see FIG. 2).

For example, a combination of positively charged amine groups and negatively charged carboxylate groups can generate a zwitterionic surface state with near-neutral charge (see Examples 1-5 and 1-6).

Within the range where the crosslinker-derived terminal functional groups do not cause toxicity and colloidal stability is maintained in biological fluids, the surface charge (zeta potential) of the polysaccharide-crosslinked colloidal particles may range from -30 mV to +10 mV.

Therefore, the present invention enables precise control over the molecular weight and/or surface charge of the polysaccharide-crosslinked colloidal particles used in the modifier, thereby imparting the resulting in vivo administrable complexes with desirable pharmacokinetic properties in vivo.

In addition to aqueous stability, the surface charge of nanoparticles significantly affects their interaction with endogenous proteins. In general, nanoparticles larger than 30 nm administered intravenously are readily recognized by the immune system and subsequently cleared from the bloodstream via phagocytic uptake by macrophages. Even for smaller nanoparticles, those bearing excessive positive or negative surface charges may adsorb blood components, primarily serum proteins, thereby increasing their effective size and promoting clearance by phagocytes.

For example, in the in vivo administrable complexes modified with the polysaccharide-crosslinked colloidal particles according to the present invention, the surface charge of the complexes may be governed by the surface charge of the polysaccharide-crosslinked colloidal particles. This surface charge significantly influences the pharmacokinetics and behavior of intravenously administered nanomaterials. Specifically, when serum proteins bind nonspecifically to the nanomaterial via electrostatic interactions-i.e., opsonization-nanomaterial-protein complexes are formed, which promote phagocytic uptake and organ accumulation via the mononuclear phagocyte system (MPS). Even if the original nanomaterials possess a size amenable to renal filtration, their association with proteins increases their hydrodynamic size beyond the renal clearance threshold, thereby preventing urinary excretion. To avoid such unintended organ accumulation and enable renal clearance, it is essential to impart anti-opsonization properties to the nanoparticles.

Accordingly, the in vivo administrable complexes modified with the polysaccharide-crosslinked colloidal particles of the present invention can be engineered such that their surface charge, derived from the colloidal particle, is finely tuned to fall within the range of -30 mV to +10 mV. This tuning minimizes non-specific adsorption of serum proteins and allows modulation of blood circulation time and renal excretion capability (see FIG. 11).

The in vivo administrable complex of the present invention may also be engineered to promote or evade opsonization by modulating the surface charge of the polysaccharide-crosslinked colloidal particles, which serve as modifiers, such that the surface charge is positive, negative, or zwitterionic (i.e., exhibiting both positive and negative charges).

The desired surface charge can be achieved by modulating the crosslinker substitution ratio on the polysaccharide-crosslinked colloidal particles to be within 2% to 50% relative to the total number of functional groups on the polysaccharide. In addition, 2% to 98% of the crosslinkers may be designed such that one terminal group remains unreacted in the crosslinking process and is instead exposed on the surface of the particle.

As shown in Example 1-17 and FIG. 11, when a 4 nm iron oxide nanoparticle is surface-modified with a 3 nm dextran-crosslinked nanoparticle having a surface charge of -3 mV, the resulting surface-modified particle exhibits a surface charge of -3 mV, a total hydrodynamic diameter of 8 nm or less, and is not phagocytosed by macrophages. Due to its renal clearance capability, renal excretion can be visualized in T1 MR imaging (see FIG. 11A).

When a 4 nm iron oxide nanoparticle is surface-modified with a 5 nm dextran-crosslinked nanoparticle also having a surface charge of -3 mV, the resulting particle has a surface charge of -3 mV and a total hydrodynamic diameter greater than 8 nm. In this case, the iron oxide nanoparticles remain in the bloodstream for an extended period, which is advantageous for prolonged vascular imaging. Accordingly, in T1 MR imaging, cardiovascular structures such as the heart, aorta, and carotid artery can be clearly visualized even 30 minutes post-injection (see FIG. 11B).

In contrast, when a 4 nm iron oxide nanoparticle is surface-modified with a 5 nm dextran-crosslinked nanoparticle having a surface charge of -30 mV, the resulting particle exhibits a surface charge of -30 mV and a total hydrodynamic diameter greater than 8 nm. These nanoparticles are readily taken up by macrophages and accumulate in the liver (see FIG. 11C), making them suitable for liver imaging in T2 MR applications.

### 5. Opsonization Inhibitors (Anti-Opsonization)

The polysaccharide-crosslinked colloidal particles of the present invention can be used as opsonization inhibitors for modifying target materials such as functional nanoparticles or drugs.

The surface charge of the polysaccharide-crosslinked colloidal particles of the present invention can be engineered to range from -30 mV to +10 mV, thereby preventing nonspecific binding to plasma proteins such as albumin.

To evaluate whether the surface charge modulation of the polysaccharide-crosslinked colloidal particles, which serve as modifiers in the present invention, can effectively control actual binding with serum proteins-that is, the anti-opsonization effect-an in vivo administrable complex of the present invention, which are surface-modified with dextran-crosslinked nanoparticles having adjusted surface charges, was mixed with serum proteins (fetal bovine serum, FBS). Changes in hydrodynamic diameter or surface charge upon binding with serum proteins were assessed using electrophoresis (see Example 1-15). As shown in FIG. 9, depending on the type of dextran-crosslinked nanoparticles exhibiting different surface charges, i.e., depending on the final surface charge, the prevention of serum protein binding was confirmed by analyzing electrophoretic band shifts and broadening.

Opsonins are phagocytosis-promoting factors present in serum, including immunoglobulins and other plasma proteins that bind to foreign particles and promote their uptake by phagocytes. Bacteria opsonized by such proteins are rapidly destroyed by macrophages.

When nanoparticles are exposed to actual in vivo environments-such as the bloodstream, interstitial fluid, or the extracellular matrix-proteins adsorb nonspecifically onto the nanoparticle surface, forming a "protein corona." This corona effect leads to opsonization, whereby proteins coat the nanoparticle surface, which ultimately determines the nanoparticles' apparent size, colloidal stability, surface characteristics, cellular uptake efficiency, in vivo biodistribution, intracellular localization, pharmacokinetics, tissue distribution, and toxicity.

Nanomedicines may be rapidly cleared from the bloodstream via the mononuclear phagocyte system (MPS), thus reducing or nullifying their therapeutic efficacy. The corona effect may also impair the targeting capability of conjugates in which a targeting moiety is bound to the nanoparticle surface, due to alterations in surface properties. Moreover, nanoparticle-protein interactions have been reported to induce hypersensitivity reactions in certain patients.

In contrast, the polysaccharide-crosslinked colloidal particles of the present invention may be used to modify functional nanoparticles or drugs for in vivo administration as opsonization inhibitors, thereby addressing the above-described issues by adjusting the final surface charge of the resulting in vivo administrable complexes For example, by combining surface-exposed functional groups derived from the crosslinker-such as amine groups imparting a positive charge and carboxyl groups imparting a negative charge-the polysaccharide-crosslinked colloidal particles of the present invention may be engineered to exhibit a zeta potential of 0 mV, and thus may serve as anti-opsonization modifiers for in vivo administration that inhibit phagocytic uptake by macrophages.

### 6. Blood Circulation Half-Life Regulator

The polysaccharide-crosslinked colloidal particles of the present invention can be used as blood circulation half-life regulators for target substances such as functional nanoparticles or drugs.

The polysaccharide-crosslinked colloidal particles of the present invention can regulate the circulation time in the bloodstream and/or prevent immune phagocytosis of the resulting in vivo administrable complexes modified therewith by adjusting the type of cross-linker and/or the number of functional groups derived from the cross-linker exposed on the surface.

The half-life of a nanodrug in systemic circulation is closely related to how quickly and efficiently it extravasates from the microvasculature into the target tissue microenvironment, and thus modulation of circulation time is critical for effective drug delivery. One of the most influential factors determining the circulation half-life of nanoparticles is nonspecific adsorption due to binding with plasma proteins. To reduce nonspecific binding with plasma proteins, polyethylene glycol (PEG) or PEGylation is commonly used. However, in the present invention, instead of PEG, the surface of the functional nanoparticles is coated with polysaccharide-crosslinked colloidal particles, which effectively block the aforementioned corona effect and thereby reduce nonspecific interactions with the mononuclear phagocyte system (MPS).

### 7. Pharmacokinetic Modulator

The polysaccharide-crosslinked colloidal particles of the present invention may be used as a pharmacokinetic modulator for modifying target substances such as functional nanoparticles or drugs.

The in vivo administrable complex modified with the polysaccharide-crosslinked colloidal particles of the present invention may be engineered to provide desired circulation time, biodistribution, or excretion profile- such as renal clearance pharmacokinetics-by controlling the overall size and surface charge of the colloidal particles. This may be achieved by adjusting at least one parameter selected from: the molecular weight of the branched or cyclic polysaccharide, the length of the polysaccharide backbone, the type of crosslinker used during the crosslinking reaction, the amount and rate of crosslinker administration during synthesis, and any additional chemical modification of functional groups. In particular, the in vivo pharmacokinetic characteristics of the in vivo administrable complex modified with the polysaccharide-crosslinked colloidal particles of the present invention may be tailored by modulating the molecular weight and surface charge of the colloidal particles (see Example 1-17 and FIG. 11).

Whether the in vivo administrable complex undergoes phagocytosis by macrophages following in vivo administration, metabolic degradation, circulatory half-life, extravasation across capillary walls for parenchymal delivery, tissue accumulation, renal clearance into urine, systemic absorption into the vascular compartment following in vivo administration, or potential collection and reuse via urinary excretion- all of these can be controlled by adjusting the size and surface charge of the in vivo administrable complex modified with the polysaccharide-crosslinked colloidal particles of the present invention.

The enhanced permeation and retention (EPR) effect, wherein organic or inorganic nanoparticles tend to accumulate more selectively in cancerous or diseased tissues with loose architecture than in normal tissues with dense structures, can also be achieved by controlling the size of the in vivo administrable complexes according to the present invention.

For their growth and survival, cancer cells rely on altered biochemical regulatory mechanisms that are distinct from those of normal cells. Notably, even under normoxic conditions, cancer cells predominantly depend on glycolysis rather than oxidative phosphorylation. Through cytoplasmic glycolysis, energy is generated, and pyruvate is converted into lactate. The lactate is secreted from the cells, contributing to an acidic tumor microenvironment, which promotes degradation of the extracellular matrix, tissue invasion, and metastasis, thereby enhancing tumor malignancy.

Accordingly, the polysaccharide-crosslinked colloidal particles and/or the in vivo administrable complexes modified therewith can be engineered to be squeezable for transport within in vivo structures, thereby maximizing the EPR effect in terms of tumor tissue penetration.

Furthermore, customized in vivo administrable complexes exhibiting desired pharmacokinetic and pharmacodynamic properties can be designed by adjusting various parameters associated with the polysaccharide-crosslinked colloidal particles used as modifiers-such as the molecular weight of the polysaccharide, the length of the polysaccharide backbone, the type of crosslinker used, the amount and rate of crosslinker addition during synthesis, and/or combinations involving additional chemical functionalization.

### 8. Blood Capillary Permeability Modulator

The polysaccharide-crosslinked colloidal particles of the present invention can be used as a blood capillary permeability modulator for modifying target substances such as functional nanoparticles or drugs.

In general, drug distribution refers to the reversible process by which a drug leaves the bloodstream and enters the extracellular fluid and tissues.

Capillary structures vary widely in terms of the extent to which their basement membranes are exposed due to slit junctions between endothelial cells. In the liver and spleen, large discontinuous fenestrations in the capillaries expose much of the basement membrane, allowing the passage of large plasma proteins. In contrast, brain capillaries are continuous and lack slit junctions. Instead, tightly packed endothelial cells form tight junctions that constitute the blood-brain barrier (BBB). To enter the brain, a drug must either cross the endothelial cells of the central nervous system (CNS) capillaries or be actively transported.

Typically, drug molecules administered intravenously rapidly disappear from the plasma and enter the interstitial fluid of tissues. Factors influencing this process include intrinsic physiological parameters such as cardiac output, local blood flow, capillary permeability, and tissue volume, as well as physicochemical properties of the drug, such as molecular size, relative lipophilicity, and binding affinity to plasma and tissue proteins.

For example, the polysaccharide-crosslinked colloidal particles and/or the in vivo administrable complexes modified therewith according to the present invention may be engineered to exhibit blood capillary impermeability, as demonstrated by their excretion via the kidneys into the urine following intravenous administration without leaking through vascular walls or entering interstitial fluid (see FIG. 11A). Specifically, the polysaccharide-crosslinked colloidal particles and/or the in vivo administrable complexes modified therewith are formed such that one end of the crosslinker having a hydrophilic functional group, or a hydrophilic group derived therefrom, is exposed to the aqueous environment. Accordingly, they behave as hydrophilic drugs and are designed to be impermeable not only to cellular membranes but also to the slit junctions between endothelial cells of blood capillaries-that is, they are engineered to exhibit capillary impermeability.

### 9. Modifiers for Blood/Lymph Circulation and/or Excretion, and Modifiers for Lymphatic Administration

The polysaccharide-crosslinked colloidal particles of the present invention can be used as a modifier for blood circulation, a modifier for lymph circulation, and/or a modifier for lymphatic administration for target substances such as functional nanoparticles or drugs.

The circulatory system is responsible for the flow of body fluids such as blood or lymph, which deliver nutrients, oxygen, and energy to organs throughout the body, and also transport carbon dioxide and metabolic waste generated from physiological activities to the respiratory or urinary systems for excretion.

Blood circulation is driven by cardiac motion. Circulating blood performs various functions including oxygen transport, nutrient delivery, removal of metabolic waste products, maintenance of body temperature, and hormone transport. A substantial amount of ions, nutrients, organic wastes, dissolved gases, and water diffuse through the capillaries, and most of these are reabsorbed back into the capillaries. The amount of fluid leaving the capillaries and the amount returning to them is nearly the same, with only a small fraction being absorbed via the lymphatic vessels.

The lymphatic system is an essential component of the immune system, comprising organs that generate and process specialized white blood cells that combat infections and cancer. These organs include the thymus, bone marrow, spleen, tonsils, appendix, and Peyer's patches in the small intestine. The lymphatic system consists of: (i) lymphatic vessels with thin walls, (ii) lymph nodes, and (iii) two major collecting ducts.

Lymphatic vessels are distributed throughout the body and are larger than capillaries-which are the smallest vessels connecting arteries and veins-but smaller than the smallest veins. Most lymphatic vessels are equipped with valves, similar to those in veins, to ensure unidirectional flow of lymph toward the heart. Lymphatic vessels collect lymph, a type of interstitial fluid, from tissues throughout the body and transport it to lymph nodes. The lymph is then returned to the venous circulation via two major collecting ducts. Compared to capillaries, lymphatic vessels are more permeable, allowing them to absorb macromolecules-including antigens and cells-more readily than blood capillaries.

Lymph originates from the interstitial fluid that diffuses through the thin walls of capillaries into the spaces between cells. While most of this fluid is reabsorbed by the capillaries, the remainder enters the lymphatic vessels and is ultimately returned to the venous circulation. Lymph also contains (i) proteins, minerals, nutrients, and other substances that influence tissue function, and (ii) a variety of other materials such as damaged cells, cancer cells, and foreign substances (e.g., bacteria and viruses) that have entered the tissue interstitium.

Lymph is a pale yellow fluid that contains fewer proteins but more lipids than blood, and is rich in lymphocytes and white blood cells. As it circulates throughout the body via the lymphatic vessels, lymph delivers nutrients to cells and removes waste products. Lymphocytes play a critical role in immune responses, defending the body against invading pathogens such as bacteria and viruses.

Lymph nodes serve as collection centers for lymph and are distributed throughout the body along lymphatic vessels, where they connect with lymphatic channels.

All lymph passes through strategically located lymph nodes, where damaged cells, cancer cells, and foreign substances are filtered out. Lymph nodes also contain specialized white blood cells-such as lymphocytes and macrophages-that phagocytose and destroy damaged cells, cancer cells, and infectious microorganisms. Therefore, one of the key functions of the lymphatic system is to remove damaged cells from the body and prevent the spread of infections and cancer. Certain lymph nodes are clustered near the surface of the body, such as in the neck, armpits, and groin, while others are located deep within internal regions like the abdomen.

The lymph nodes discharge their contents into the collecting ducts, which in turn empty into the two subclavian veins located beneath the clavicles. These subclavian veins converge to form the superior vena cava, a major vein that returns blood from the upper body to the heart.

In summary, blood circulates throughout the body via arteries and returns through veins. During this process, a portion of the fluid remains between cells as interstitial fluid-an extracellular fluid that is filtered from the plasma through capillaries within tissues and fills the spaces between cells. When this interstitial fluid enters the lymphatic capillaries, it is referred to as lymph. Lymph flows into the lymphatic vessels at a very slow rate and eventually reenters the bloodstream. As lymph exits the capillary walls and bathes the tissue cells, it removes waste products from within the tissues.

In detail, this bodily fluid-lymph-circulates throughout the body via the lymphatic vessels, supplying nutrients to each cell and transporting waste materials such as carbon dioxide (CO₂) that has diffused out of the cells, as well as damaged cells, cancer cells, and bacteria. After completing this process, the lymph returns to the bloodstream at the junction of the internal jugular vein and the subclavian vein. As the lymph flows through the lymph nodes, lymphocytes within the lymph nodes interact with foreign substances, removing and destroying them. This is why lymph nodes often become swollen during acute inflammation, as they actively filter and respond to external invading substances.

Accordingly, the polysaccharide-crosslinked colloidal particles of the present invention may be engineered for administration to distal regions of the body such that they are absorbed into lymphatic vessels or directly administered into lymphatic vessels. In such cases, they can flow into lymphatic vessels and be absorbed into the circulatory system without being cleared by lymphocytes in lymph nodes. Likewise, drugs or functional nanoparticles modified with the polysaccharide-crosslinked colloidal particles of the present invention may also be administered to distal body regions or directly into lymphatic vessels, whereby they enter the lymphatic vessels and are absorbed into the circulatory system without being cleared by lymphocytes in lymph nodes.

Furthermore, when the polysaccharide-crosslinked colloidal particles and/or functional nanoparticles of the present invention serve as MRI contrast agents, their absorption or administration into lymphatic vessels enables MRI imaging of abnormalities in the lymphatic circulation pathway, such as lymphatic obstruction, lymphedema, enlarged lymph nodes, lymphadenitis, lymphoma, and metastasis of tumors from other organs to adjacent lymph nodes.

In addition, the polysaccharide-crosslinked colloidal particles of the present invention may impart or enhance biocompatibility to target substances such as functional nanoparticles or drugs, which would otherwise elicit immune rejection responses such as allergic reactions or toxicity upon administration into the body.

The polysaccharide-crosslinked colloidal particles of the present invention allow precise control of surface properties through the type and/or density of functional groups exposed on their surface. Accordingly, in vivo administrable complexes modified with the polysaccharide-crosslinked colloidal particles of the present invention, whose surface properties are precisely controlled, may likewise exhibit finely controlled surface characteristics. For example, the polysaccharide-crosslinked colloidal particles of the present invention, and/or drugs or functional nanoparticles modified therewith, may be engineered such that, after in vivo administration, they are not retained in tissues but are instead absorbed into the circulatory system and subsequently excreted in urine via renal filtration. Moreover, since the polysaccharide-crosslinked colloidal particles of the present invention can be engineered to be excreted in intact form without metabolic degradation along the routes of absorption, distribution, and/or excretion following in vivo administration, they may also be suitable for reuse or recycling. By utilizing this principle, the circulation and excretion of functional nanoparticles and drugs modified with the polysaccharide-crosslinked colloidal particles may be controlled via blood and lymphatic flow. Furthermore, through adsorption in vivo, they may collect and eliminate biological information (e.g., proteins in the target tissue or bloodstream) or hazardous substances such as heavy metals.

In summary, the polysaccharide-crosslinked colloidal particles of the present invention may be engineered (i) as modifiers for blood circulation, (ii) as modifiers that, after in vivo administration, flow into lymphatic vessels and are absorbed into the circulatory system, or (iii) as modifiers for lymphatic administration. Therefore, in vivo administrable complexes modified with these particles may likewise be engineered for blood circulation, for absorption into the circulatory system via lymphatic vessels, and/or for administration via lymphatic vessels.

For example, the polysaccharide-crosslinked colloidal particles of the present invention may serve as modifiers that prevent in vivo administrable complexes modified therewith from being phagocytosed by macrophages or metabolically degraded after in vivo administration, and, when necessary, enable their excretion via the kidneys into the urine.

Accordingly, the polysaccharide-crosslinked colloidal particles of the present invention may be introduced into the lymphatic circulation, absorbed into the circulatory system, and/or reused after in vivo administration. As a result, in vivo administrable complexes comprising drugs or functional nanoparticles modified with the polysaccharide-crosslinked colloidal particles may likewise be introduced into the lymphatic circulation, absorbed into the circulatory system, and rendered suitable for reuse.

### 10. Modulator for Avoidance of Phagocytosis by Macrophages

The polysaccharide-crosslinked colloidal particles of the present invention may be used as a modulator for avoidance of phagocytosis by macrophages of target substances such as functional nanoparticles or drugs.

Nanoparticulate drug formulations have the potential to selectively deliver drugs to diseased sites or to enhance the local drug concentration at such sites. However, most nanoparticulate drug formulations, such as artificial nanocarriers, liposomes, and polymeric nanoparticles, face the limitation of being rapidly cleared from the bloodstream before reaching the diseased site due to phagocytosis by the reticuloendothelial system, a component of the body's immune system.

The reticuloendothelial system is also referred to as the mononuclear phagocyte system (MPS). It is composed of cells located throughout the human body that are capable of absorbing specific substances. These cells constitute part of the body's defense mechanism.

Reticuloendothelial cells originate from precursor cells in the bone marrow. These precursor cells develop into monocytes, which are phagocytic cells released into the bloodstream. While some monocytes remain in circulation, most migrate into tissues where they differentiate into much larger phagocytes known as macrophages. Most macrophages are stationary cells that remain within tissues, filtering and destroying foreign substances. However, some macrophages may become detached and circulate within the bloodstream or interstitial spaces.

The morphology and nomenclature of macrophages within tissues vary depending on their anatomical location. Reticular cells are found in the lymph node sinuses, spleen, and bone marrow, whereas histiocytes are commonly found in subcutaneous tissues. Microglia are resident in neural tissues, alveolar macrophages are located in the pulmonary alveoli, and Kupffer cells are present in the liver. Through phagocytosis, macrophages constitute the first line of defense against harmful particles that enter the body.

According to the present invention, polysaccharide-crosslinked colloidal particles formed by intra- and/or inter-molecular crosslinking of 1 to 3 branched polysaccharides or 2 to 30 cyclic polysaccharides at the -OH groups of their monosaccharide building blocks using a crosslinker possess structural characteristics that allow them to be controlled by various modulatory factors such that they are neither phagocytosed by macrophages nor bound by opsonin proteins in the bloodstream. Accordingly, the in vivo administrable complexes modified with the polysaccharide-crosslinked colloidal particles of the present invention can be engineered to exhibit desired blood circulation and renal clearance profiles.

Because the polysaccharide-crosslinked colloidal particles of the present invention can be engineered to avoid phagocytosis by macrophages during in vivo administration, biodistribution, and elimination. the in vivo administrable complexes modified with the polysaccharide-crosslinked colloidal particles of the present invention are not absorbed by macrophage-mediated phagocytosis in reticuloendothelial system organs such as the liver, spleen, bone marrow, and lymph nodes.

In one embodiment of the present invention, it was confirmed by MRI that dextran-crosslinked nanoparticles engineered to function as T1 MRI contrast agents were predominantly excreted via the kidneys into the urine after in vivo administration. It was also found that, after in vivo administration, the particles could be predominantly collected in the urine rather than in the feces, and that the particles could be collected in the urine even after intra-articular injection. From these findings, it was demonstrated that the polysaccharide-crosslinked colloidal particle platform technology of the present invention can be engineered to avoid phagocytosis by macrophages and metabolic degradation in the course of in vivo administration, biodistribution, and elimination.

Furthermore, in one embodiment of the present invention, it was confirmed by MRI that certain dextran-crosslinked nanoparticles engineered to function as T1 MRI contrast agents, when administered intravenously, intra-articularly, or into the spinal canal, did not distribute to the liver, spleen, bone marrow, or lymph nodes. This was further confirmed by the observation that the nanoparticles were fully recovered in the urine without structural alteration.

Accordingly, the polysaccharide-crosslinked colloidal particles of the present invention can be engineered to avoid phagocytosis by macrophages and, therefore, may be used as modulators for avoidance of phagocytosis by macrophages for target substances.

### 11. Modifier for Body Location Tracking for MRI

The polysaccharide-crosslinked colloidal particles of the present invention may be used as body location tracking modifiers for MRI of target substances such as functional nanoparticles or drugs.

In the in vivo administrable complexes modified with the polysaccharide-crosslinked colloidal particles of the present invention, (i) functional nanoparticles for in vivo administration, which are bound to surface-exposed reactive groups derived from crosslinkers, and/or (ii) the polysaccharide-crosslinked colloidal particles themselves may be engineered to function as MRI contrast agents.

Accordingly, the in vivo administrable complexes modified with the polysaccharide-crosslinked colloidal particles, which are engineered to function as MRI contrast agents, can be used for positional tracking in MR angiography, MR arthrography, MR cisternography, MR myelography, MR lymphangiography, MR cholangiopancreatography, or MRI contrast imaging of the brain or abdomen.

Magnetic resonance imaging (MRI) is a technique that acquires anatomical, physiological, and biochemical information of the body in image form by utilizing the relaxation phenomena of nuclear spins of hydrogen atoms in water under a magnetic field. It is currently one of the non-invasive, real-time imaging diagnostic tools that enables visualization of internal organs in living humans or animals.

In the fields of life sciences and medicine, magnetic resonance imaging (MRI) is widely and precisely utilized by employing a method that involves injecting substances from outside the body to increase image contrast. These substances are referred to as contrast agents, and they allow clear differentiation of target areas in the MRI by enhancing signal contrast using superparamagnetic or paramagnetic materials. The contrast between tissues in MRI images arises from differences in the relaxation process, in which the hydrogen nuclear spins of water within tissues-excited by external energy-return to equilibrium at varying rates depending on the tissue type. Contrast agents influence this relaxation process, creating differences in relaxation times between tissues and inducing changes in MRI signals, thereby enhancing the contrast between tissues more distinctly.

Enhanced contrast through the use of contrast agents allows specific organs and tissues to be visualized more clearly by increasing or decreasing their signal intensity relative to surrounding regions. A contrast agent that increases the signal intensity of the target tissue relative to its surroundings is referred to as a "positive" contrast agent (T1 contrast agent), whereas one that decreases the signal intensity is referred to as a "negative" contrast agent (T2 contrast agent). More specifically, MRI contrast agents are categorized into T1 contrast agents, which exploit the high-spin state of paramagnetic materials, and T2 contrast agents, which utilize the magnetic inhomogeneity surrounding ferromagnetic or superparamagnetic materials.

The ratio of the spin-spin relaxivity coefficient (r₂) to the spin-lattice relaxivity coefficient (r₁), commonly referred to as the r₂/r₁ ratio, is a key index for determining whether a contrast agent is more suitable for T1 or T2 MRI applications. Typically, T1 contrast agents exhibit an r₂/r₁ ratio of about 1 to 2, while T2 contrast agents desirably have an r₂/r₁ ratio of 5 or higher.

To date, commercially available MRI contrast agents include paramagnetic compounds used as "positive" contrast agents and superparamagnetic nanoparticles used as "negative" contrast agents. Currently, iron oxide nanoparticles such as SPIO (Superparamagnetic Iron Oxide) are employed as T2 contrast agents. T2 imaging produces a negative contrast, in which the target region appears darker than surrounding tissue, offering limited contrast enhancement and presenting a drawback known as the blooming effect, where the contrast-enhanced area appears larger than its actual size. In contrast, T1 contrast agents produce positive contrast, allowing the target region to appear brighter in the image. T1 contrast agents commonly utilize high-spin materials, and gadolinium complexes, which possess seven unpaired electrons in their 4f orbitals, are typically used for this purpose.

Iron oxide used as a superparamagnetic material can be categorized based on particle size. Particles with diameters of 50 nm or more are referred to as SPIO, whereas those with smaller diameters are classified as USPIO (Ultrasmall Superparamagnetic Iron Oxide). Due to their smaller size, USPIO particles are less susceptible to phagocytosis by macrophages in the bloodstream, thereby exhibiting prolonged circulation time, which can be leveraged to detect vascular abnormalities. Additionally, USPIOs can be administered via rapid injection at lower doses compared to SPIOs. Feridex and Resovist, previously used in clinical settings, are representative examples of SPIO agents.

Since the late 1990s, thermal decomposition methods for nanoparticle synthesis have enabled the development of techniques for producing uniform iron oxide nanoparticles with sizes ranging from 5 to 20 nm.

The polysaccharide-crosslinked colloidal particles of the present invention may be engineered to function as T1 MRI contrast agents that produce bright signals on MRI images by coordinating divalent or trivalent iron ions to hydrophilic groups derived from crosslinkers exposed on the surface of a spherical core, which is formed by crosslinking of 1 to 3 branched polysaccharides or 2 to 30 cyclic polysaccharides at hydroxyl groups of their monosaccharide building blocks using a crosslinker. Furthermore, the divalent or trivalent iron ions may remain exposed on the surface, facilitating access by water molecules and thereby accelerating proton relaxation of water molecules, which further enhances T1 MRI contrast effects.

Therefore, the iron-coordinated polysaccharide-crosslinked colloidal particles of the present invention, due to their characteristics as T1 MRI contrast agents, can provide imaging that visualizes their location when present in microvasculature, ureters, liver, spleen, articular cavities, spinal canal, and/or various organs in vivo. In addition, MRI may be used to determine whether the particles accumulate in tissue and/or extravasate through vascular walls. Accordingly, the iron-coordinated polysaccharide-crosslinked colloidal particles may serve as body location tracking agents.

The iron-coordinated polysaccharide-crosslinked colloidal particles of the present invention may also be engineered to allow MRI-based monitoring of their in vivo fate, including whether they are phagocytosed by macrophages, metabolically degraded, circulate in the bloodstream, are delivered to cellular parenchyma via capillary walls, accumulate in tissue, are excreted into urine via the kidneys or into feces, are absorbed into the vascular system after administration, extravasate through blood vessel walls, or are collectable via urine for potential reuse.

Moreover, the iron-coordinated polysaccharide-crosslinked colloidal particles of the present invention enable quality control of associated pharmaceutical compositions in animal studies by verifying, through in vivo biodistribution analyses based on the administration site and/or route, whether the compositions exhibit the intended pharmacokinetic behavior and whether they have been manufactured with uniform quality. Furthermore, the accumulated bioinformation may be used to precisely design in vivo administrable complexes tailored to patient characteristics (e.g., condition and/or medical history), disease type, route of administration, and pharmacological mechanism of a drug delivered via the polysaccharide-crosslinked colloidal particles of the present invention.

The iron-coordinated polysaccharide-crosslinked colloidal particles of the present invention, while functioning as contrast agents, may also be conjugated to drug carriers such as fragments, antibodies, aptamers, or artificial antibodies (e.g., repebodies) that include target antigen-binding domains. In such cases, the particles can be targeted to cells expressing the target antigen on their surface or to regions within the body that contain the target antigen, enabling visualization and quantification of such regions, as well as quantification of the distribution or concentration of the target antigen based on T1 MRI signal intensity. Furthermore, when an antibody specific to a tumor-associated antigen is used, enhanced therapeutic efficacy may be achieved, such as high tumor uptake and rapid clearance from healthy tissues.

### 12. Drug Delivery Vehicle for Drug Loading and Release

The polysaccharide-crosslinked colloidal particles of the present invention can exhibit high dispersibility, excellent colloidal stability in physiological fluids, solubility, and ease of preparation by controlling the type and density of crosslinker-derived functional groups exposed on their surface. Therefore, they can be used as drug delivery vehicles in various formulations.

As used herein, the term "drug" includes not only molecular-level pharmaceutical agents but also functional nanoparticles intended for in vivo administration, as long as they fall within the definition of drugs.

The polysaccharide-crosslinked colloidal particles of the present invention can bind not only to functional nanoparticles but also to non-particulate drugs through the crosslinker-derived functional groups exposed on their surface. These particles exhibit water dispersibility and colloidal stability and can be synthesized in aqueous solution. As such, aqueous suspensions of the polysaccharide-crosslinked colloidal particles possess high drug-loading capacity for drug administration.

Drugs can be loaded into the polysaccharide-crosslinked colloidal particles by two approaches. The first approach involves encapsulating the drug within the nanoparticle. The second approach involves conjugating the drug onto the nanoparticle. The drug-loading efficiency depends on factors such as the type and surface density of crosslinker-derived functional groups capable of interacting with the drug, the solubility of the drug within the polysaccharide-crosslinked colloidal particle, and the particle size. These parameters, in turn, are related to the type of crosslinker-derived functional group, the molecular weight of the polysaccharide-crosslinked colloidal particle, and the interactions between the drug and the particle.

The polysaccharide-crosslinked colloidal particles of the present invention can be engineered to possess various surface functional groups, such as amine, carboxyl, and thiol groups, or combinations thereof, to achieve a surface charge ranging from -30 mV to +10 mV. In this case, covalent or electrostatic interactions can be established not only with drugs such as indomethacin (IDM), aspirin, and doxorubicin, but also with a variety of growth factors and bioactive molecules, including oligonucleotides.

According to the present invention, the polysaccharide-crosslinked colloidal particles having crosslinker-derived functional groups exposed on their surfaces possess several advantages as drug delivery systems, including their biocompatible material composition, chemical stability, colloidal stability, and ease of control over surface charge and particle size. Therefore, the polysaccharide-crosslinked colloidal particles with surface-exposed crosslinker-derived functional groups according to the present invention can be used in various targeted therapies, including pulmonary delivery, cancer therapy, tumor therapy, and vaccination. A primary objective in designing nanoparticles for use as drug delivery systems is to control the particle size, surface area, and surface properties such that the nanoparticles can carry an appropriate amount of a drug, achieve site-specific action, and release the drug to exhibit the desired pharmacological activity.

The polysaccharide-crosslinked colloidal particles of the present invention can be engineered such that they do not induce appreciable levels of inflammation or coagulation under dynamic and quasi-static conditions in human blood, and thus may be suitable not only for local drug delivery but also for systemic drug delivery.

### 13. Modifier for Intravenous Administration

The polysaccharide-crosslinked colloidal particles of the present invention, when engineered to serve as modifiers for circulation in the bloodstream, can also function as modifiers for intravenous administration.

For example, the polysaccharide-crosslinked colloidal particles of the present invention may be engineered to exhibit suitable physicochemical properties-such as osmotic pressure and viscosity-required for intravenous injectable formulations. By controlling the average molecular weight of the polysaccharide to a lower value, the polysaccharide-crosslinked colloidal particles and/or the resulting in vivo administrable complexes can be prepared at a high concentration, enabling precise control over the viscosity of the intravenous formulation through dilution.

In addition, the polysaccharide-crosslinked colloidal particles of the present invention may be engineered to avoid clearance by the liver upon intravenous administration, and preferably to be excreted via the kidneys into the urine following circulation in the bloodstream. For instance, the polysaccharide-crosslinked colloidal particles of the present invention may be engineered to be excreted through the kidneys into the urine after intravenous injection, without leakage through vascular walls, phagocytic uptake by macrophages, or metabolic degradation. Conversely, when desired, the particles may be engineered such that, upon intravenous administration, they are cleared by the liver and spleen, thereby enabling targeted delivery of drugs to these organs.

Accordingly, the polysaccharide-crosslinked colloidal particles of the present invention are capable of circulating through the cerebrocardiovascular system after intravenous administration, thereby enabling the collection of information within the bloodstream during circulation. For example, when the polysaccharide-crosslinked colloidal particles are combined with iron ions, they may exert T1 MRI contrast-enhancing effects. In such cases, the particles may serve as contrast agents while circulating through at least one of the carotid artery, heart, aorta, inferior vena cava, cerebral vasculature, or hepatic vasculature, thereby allowing imaging of vascular structures and morphologies in specific tissues or organs, as well as analysis of blood flow and hemodynamic information.

### [In vivo Administrable Modifier for Functional Nanoparticles]

Colloidal stability is an essential property that must be guaranteed when functional nanoparticles are administered in vivo. Functional nanoparticles are typically several to tens of nanometers in size, which is significantly larger than molecular-scale substances on the order of angstroms. Due to this relatively larger size, they tend to aggregate easily under physiological conditions, resulting in increased particle size and loss of inherent physicochemical properties; in some cases, such aggregation may lead to undesirable tissue deposition. To prevent such phenomena, it is important to modify the surface of functional nanoparticles with hydrophilic materials to improve colloidal stability under diverse in vivo environments.

In the present invention, polysaccharide-crosslinked colloidal particles function as hydrophilic surface modifiers. Through crosslinker-derived functional groups exposed on their surface, they can modify the surface of functional nanoparticles intended for in vivo administration, thereby resolving the aforementioned issues.

According to the present invention, in vivo administrable complexes comprising functional nanoparticles may include, for example:
(i) an in vivo administrable complex in which the surface of a functional nanoparticle is modified with polysaccharide-crosslinked colloidal particles;
(ii) an in vivo administrable complex in which the surface of a functional nanoparticle is modified with polysaccharide-crosslinked colloidal particles, and the polysaccharide-crosslinked colloidal particles carry a drug; or
(iii) an in vivo administrable complex in which functional nanoparticles are modified with polysaccharide-crosslinked colloidal particles that have already been loaded with a drug.

The functional nanoparticles intended for in vivo administration may be inorganic nanoparticles, organic nanoparticles, or hybrid organic-inorganic nanoparticles. Non-limiting examples include iron oxide, gold, silver, quantum dots, and liposomes.

Inorganic nanoparticles such as metal oxides, quantum dots, and noble metals exhibit unique optical, magnetic, and electrical properties that distinguish them from organic nanoparticles. By leveraging these physicochemical properties, it is possible to achieve medically beneficial functions, such as precise observation, modulation, and control of biological phenomena at the molecular level. Specific examples include inorganic nanoparticles such as gold or gold nanoshells possessing photothermal or photodynamic therapeutic properties (i.e., inducing therapeutic effects by generating reactive oxygen species upon light exposure), iron oxide nanoparticles, and hafnium oxide nanoparticles.

Despite these promising features, inorganic nanoparticles administered in vivo are often recognized as foreign substances by the immune system, resulting in phagocytosis by macrophages and accumulation in organs, which makes excretion from the body difficult. When the composition of the inorganic nanoparticle includes heavy metals, such as those found in quantum dots, failure to eliminate them and their potential degradation within the body may lead to toxicity. Therefore, for nanoparticles to be used as contrast agents for disease diagnosis in vivo, it is essential to develop safe formulations that can be eliminated from the body without metabolic degradation.

For instance, the polysaccharide-crosslinked colloidal particles of the present invention can be engineered, via crosslinker-derived functional groups exposed on their surfaces, to coat functional nanoparticles intended for in vivo administration with various thicknesses and surface charges. As a result, the coated functional nanoparticles can evade phagocytic uptake and exhibit compact hydrodynamic diameters that enable renal filtration and/or prolonged circulation time in the bloodstream.

The polysaccharide-crosslinked colloidal particles of the present invention allow for uniform coating of the surfaces of functional nanoparticles, and the functional nanoparticles modified therewith can have their surface charge and/or degree of swelling finely tuned through the properties of the polysaccharide-crosslinked colloidal particles. This allows the retention time of the nanoparticles at the target site to be precisely controlled to ensure their intended biological performance.

### [In Vivo Administrable Modifier for Drugs]

As used herein, the term "drug" refers to any substance that is used for the diagnosis, cure, mitigation, treatment, or prevention of disease (excluding foods and medical devices), or that is intended to affect the structure or function of the body. For example, it includes any chemical or biological substance that exerts an effect on the body or its metabolism.

In this specification, the drug may be a monomolecular compound, peptide (amino acid), aptamer/RNA (oligomer), or protein (such as an antibody). Drugs having ultra-toxic properties with picomolar-level IC₅₀ values at the cellular level may be used as payloads; representative examples of such drugs include microtubulin inhibitors, DNA-damaging agents, and topoisomerase I (Top-1) inhibitors.

According to the present invention, in vivo administrable complexes comprising a drug may include: (1) a polysaccharide-crosslinked nanoparticle-drug conjugate in which the drug is bound to the polysaccharide-crosslinked nanoparticle via a degradable bond or linker as a payload; (2) an in vivo administrable complex in which polysaccharide-crosslinked nanoparticles are bound as a surface-modifying agent to the exposed surface of functional nanoparticles formed by aggregation of hydrophobic drugs; or (3) an in vivo administrable complex in which polysaccharide-crosslinked nanoparticles, acting as a cationic polymer, are electrostatically bound to anionic nucleic acid-based drugs such as nucleic acids, siRNA, or miRNA.

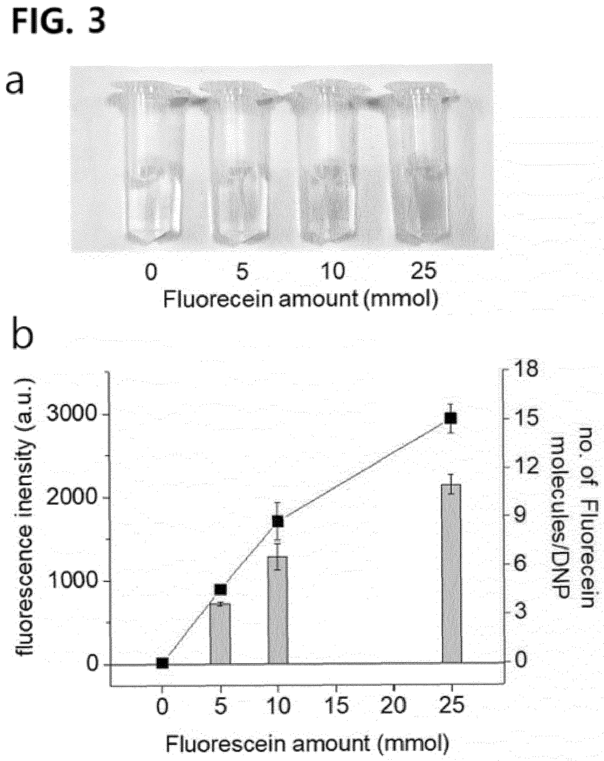

According to the present invention, polysaccharide-crosslinked colloidal particles formed by crosslinking 1 to 3 branched polysaccharides or 2 to 30 cyclic polysaccharides at the hydroxyl groups (-OH) of their constituent monosaccharide building blocks via a crosslinker can be engineered not only to impart colloidal stability in body fluids to poorly water-soluble or hydrophobic drugs, but also to function as carriers for drug delivery. As illustrated in FIG. 3, a fluorescent molecule that is otherwise insoluble in water was rendered water-dispersible upon conjugation and modification with a C-DNP (crosslinked dextran nanoparticle), and the number of such insoluble molecules conjugated can be adjusted. That is, to achieve effective therapeutic outcomes, the polysaccharide-crosslinked colloidal particles can be engineered to maintain a therapeutically effective concentration of the drug in the bloodstream and to release the bioactive substance at a controlled rate.

Additionally, the polysaccharide-crosslinked colloidal particles, after serving as drug delivery vehicles, can be engineered to be excreted through urine or feces without undergoing metabolic degradation in vivo.

Furthermore, according to the present invention, a polysaccharide-crosslinked colloidal particle-drug complex in which a drug is conjugated as a payload via a degradable acid-sensitive bond or linker to the polysaccharide-crosslinked colloidal particle can function as a prodrug. Once administered into the body, the complex can be engineered to release the active drug upon degradation in an acidic environment (pH ≤ 7) surrounding a tumor or via metabolic enzymes, thereby exerting its pharmacological effect, and can be flexibly designed according to the intended route of administration.

The transport of hydrophobic or poorly water-soluble drugs can be improved by the polysaccharide-crosslinked colloidal particles of the present invention, which is attributable to their small particle size, allowing for rapid dispersion in the bloodstream and targeted delivery to specific cells or tissues.

A notable advantage of the polysaccharide-crosslinked colloidal particle-drug complexes is that, even at high drug doses, they exhibit reduced cytotoxicity toward normal cells compared to conventional chemotherapy using the free drug alone, thereby enabling meaningful therapeutic effects.

Antibody-drug conjugates (ADCs) are novel drug platforms that selectively deliver potent cytotoxic payloads to tumor tissues by leveraging the high tissue selectivity of antibodies. In application of this principle, the present invention provides in vivo administrable complexes in which drugs and antibodies are conjugated to functional groups-preferably crosslinker-derived functional groups-exposed on the surface of the polysaccharide-crosslinked colloidal particles. As a result, ultra-potent payloads capable of inducing cancer cell death at picomolar (pM) concentrations can be selectively delivered to tumor tissues, while minimizing systemic drug exposure, thereby achieving both enhanced anticancer efficacy and improved safety.

### [Application of the Polysaccharide-Crosslinked Colloidal Particle Platform Technology of the Present Invention as a Drug Delivery Vehicle]

To maximize the pharmacological potential of a drug, it must be paired with a drug delivery system that is optimized for that specific drug.

According to the present invention, polysaccharide-crosslinked colloidal particle-drug complexes, in which a drug is conjugated as a payload via a biodegradable bond or linker to the polysaccharide-crosslinked colloidal particles, can be formulated into oral dosage forms, drugs absorbed through the oral or nasal mucosa, suppositories, or injectable formulations.

In the case of tablets or capsules, such dosage forms may exhibit greater stability in the gastric wall compared to liquid (solution) formulations, with more than 75% of the drugs demonstrating a sustained absorption duration of 1 to 3 hours.

The polysaccharide-crosslinked colloidal particles of the present invention offer advantages over other colloidal carriers in terms of higher stability, ease of manufacture, and tunable properties such as size, morphology, and surface charge, depending on the material and process parameters, enabling customized engineering for diverse applications. In addition, drug loading and release efficiency can be modulated based on the characteristics of the polysaccharide-crosslinked colloidal particles, as well as the concentration and nature of the drug.

Accordingly, the polysaccharide-crosslinked colloidal particles of the present invention have broad applicability and advantages in delivering various therapeutic agents, including anticancer drugs, peptide hormones, growth factors, and genetic materials such as DNA and RNA.

### 1. Tumor Penetration and Controlled Drug Release of Anticancer Drugs

The tumor penetration efficiency of nanodrugs varies depending on how strongly they interact with tumor tissues. For example, in the case of antibodies, when they bind strongly to tumor tissues, cellular internalization still occurs, but their penetration depth tends to be shallower compared to antibodies with weaker binding. Nanoparticles that are slightly larger than antibodies can exhibit a similar behavior.

Another critical factor for effective anticancer therapy is how to efficiently regulate the release of the drug payload from inside the nanoparticle during or following cellular uptake or extravasation. In general, when anticancer drugs are administered alone into the body, they exhibit very rapid pharmacokinetics (PK) and high cytotoxicity. Thus, determining the optimal drug concentration and exposure duration that minimizes cytotoxicity while maximizing therapeutic efficacy is essential. However, in the case of nanodrugs in which the drug is encapsulated or loaded, cytotoxicity concerns related to drug concentration may be alleviated. Additionally, the area under the curve (AUC) of drug release from nanodrugs-representing the concentration of drug released over time following administration-can be maintained at a relatively constant level, allowing the drug to be released gradually at low concentrations. In contrast, when free anticancer drugs are administered, the AUC typically shows a sharp peak followed by a steep decline in drug concentration over time. Nevertheless, due to the complexity and heterogeneity of tumor tissues, and because nanoparticle-based delivery does not always enhance therapeutic efficacy for all anticancer agents, the present invention provides a platform technology using squeezable polysaccharide-crosslinked colloidal particles to improve passive sustained drug release. Specifically, the invention enables the design of stimuli-responsive nanodrugs that release their payloads in response to physiological stimuli near the tumor microenvironment, such as local pH, redox state, or enzymatic activity. In addition to internal stimuli, nanodrugs may also be engineered to respond to external stimuli such as light, heat, magnetic fields, or ultrasound to achieve controlled release of the encapsulated drug.

### 2. Cellular Uptake and Intracellular Delivery Process

The squeezable polysaccharide-crosslinked colloidal particle platform technology of the present invention can be applied to the development of nanodrugs that enable effective active drug delivery, as opposed to passive delivery (e.g., enhanced permeability and retention, EPR), by specifically recognizing and binding to marker molecules expressed on the surface of cancer cells.

Nanoparticles may (i) release their payload drug into the local microenvironment after reaching the vicinity of the target cell, allowing the drug to enter the cell independently, (ii) enter the cell and subsequently release the encapsulated drug intracellularly, or (iii) be delivered to specific intracellular organelles-such as the nucleus, mitochondria, or endoplasmic reticulum-while retaining the payload, by trafficking through organelles such as endosomes. Accordingly, the polysaccharide-crosslinked colloidal particles of the present invention may be engineered into in vivo administrable complexes in which the drug is conjugated as a payload to the colloidal particles via degradable bonds or linkers, to facilitate such targeted intracellular delivery.

For example, when delivering small interfering RNA (siRNA) to cancer cells, efficient endosomal escape is essential. In this case, the surface charge of the polysaccharide-crosslinked colloidal particles may be modulated with cationic polymers such as poly(ethylene imine) to promote escape from endosomes and facilitate intracellular release of the siRNA.

### 3. Hepatic Metabolism and Hepatic Accumulation

Oral administration, being the most common route of drug delivery, offers the advantage of ease of use; however, it also carries the drawback of potential side effects due to the complex physiological processes that occur within the human body. Once a drug enters the body, it encounters a variety of metabolic enzymes whose primary function is to degrade or modify the chemical structure of exogenous substances. This enzymatic transformation facilitates the elimination of such substances from the body. As a result, most drugs undergo metabolic reactions to form metabolites, which can lead to diminished therapeutic efficacy of the parent drug and can, in turn, cause side effects and toxicity due to the metabolites.

By modifying the drug with the polysaccharide-crosslinked colloidal particles according to the present invention, the aforementioned problems associated with oral administration can be addressed.

### [Excretion of the In Vivo Administrable Complex According to the Present Invention]

The polysaccharide-crosslinked colloidal particles and/or in vivo administrable complexes according to the present invention may be engineered to exhibit pharmacokinetics that allow for complete excretion from the body without accumulation in normal tissues.

The in vivo administrable complexes of the present invention may be engineered to evade phagocytic uptake by macrophages and avoid metabolic degradation after in vivo administration, thereby enabling renal filtration and subsequent urinary excretion.

Furthermore, the in vivo administrable complexes of the present invention may be engineered to be absorbed into the vascular circulation following administration, and, once absorbed, to be excreted through renal filtration without extravasation, thereby allowing the excreted complexes to be collected via urine for potential reuse.

### ADVANTAGEOUS EFFECTS

The present invention provides a polysaccharide-crosslinked colloidal particle platform technology that not only serves as a carrier for drug delivery of functional nanoparticles or therapeutic agents intended for in vivo administration, but also can be strategically designed to control biodistribution and excretion within the body.

### BRIEF DESCRIPTION OF THE DRAWINGS/FIGURES

FIG. 1 illustrates the hydrodynamic diameters of C-DNP-3, C-DNP-5, and C-DNP-10 synthesized in Examples 1-1 through 1-4, measured by dynamic light scattering (DLS; measurement range: 1 nm to 1000 nm) (a), and the hydrodynamic diameter of C-CMDNP-10 (b).
FIG. 2 shows the surface charges of C-DNPs synthesized by adjusting the amount of SA to substitute the surface-exposed functional groups with carboxyl groups, as described in Examples 1-5-2 and 1-6.
FIG. 3 shows the visual fluorescence change (a) and the number of fluorescein molecules conjugated per C-DNP (b) for C-DNPs synthesized in Example 1-7 by adjusting the amount of NHS-Fluorescein to introduce and control the number of fluorescent molecules on the C-DNP surface.
FIG. 4 demonstrates the squeezability behavior of C-DNPs according to Example 1-8 (a). When filtered using 10 kDa and 50 kDa molecular weight cut-off (MWCO) filters (panel b), C-DNP-10 with a diameter of 5 nm passed through the filters (panel c), whereas gold nanoparticles (AuNPs) of similar size did not (panel d).
FIG. 5 illustrates the electron microscopy image of iron oxide nanoparticles (IONPs) (a); the observed size of the IONPs from electron microscopy (4 nm, b); the size of IONPs coated with C-DNP-3, C-DNP-5, and C-DNP-10 substituted with carboxyl groups according to Example 1-5-2 (c); and the size of IONPs coated with C-CMDNP-10 (d).
FIG. 6 illustrates the surface charge of iron oxide nanoparticles coated with C-DNPs having surface charges controlled according to Example 1-6.
FIG. 7 shows (a) the colloidal stability and (b) the change in hydrodynamic diameter over time of C-DNP-coated iron oxide nanoparticles under various physiological conditions (salt concentration and pH).
FIG. 8 illustrates (a) the colloidal stability and (b) the hydrodynamic diameter of iron oxide nanoparticles depending on the amount of dextran (molecular weight: 10 kDa) used for coating in Examples 1-13 and 1-14.
FIG. 9 shows electrophoresis results demonstrating the anti-opsonization effect of iron oxide nanoparticles coated with C-DNP-5 having surface charges of +5 mV (a), -3 mV (b), and -20 mV (c), according to one embodiment of the present invention.
FIG. 10 illustrates (a) the squeezability behavior of C-DNP-coated iron oxide nanoparticles according to Example 1-16. In 1% agarose gel, iron oxide nanoparticles coated with C-DNP-5 with a diameter of 9 nm exhibit squeezability, whereas gold nanoparticles of similar size coated with citrate do not (b, c).
FIG. 11 presents MRI images showing the pharmacokinetics of iron oxide nanoparticles coated with C-DNPs designed to exhibit the following properties through size and surface charge control as described in Example 1-11: hydrodynamic diameter of 7 nm and surface charge of -3 mV (a); hydrodynamic diameter of 11 nm and surface charge of -3 mV (b); and hydrodynamic diameter of 11 nm and surface charge of -30 mV (c).

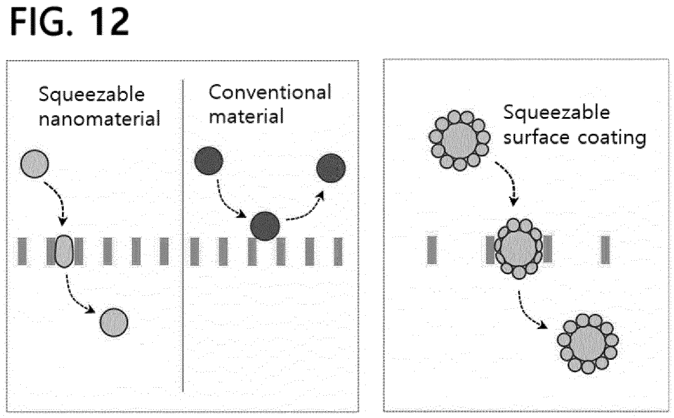

FIG. 12 is a conceptual diagram illustrating that the dextran-crosslinked nanoparticles and/or their in vivo administrable complexes are squeezable.
FIG. 13 shows (a) the conjugation chemistry used for combining Cdex with a TLR agonist, (b) the chemical structures of Cdex@TLR prepared by different conjugation chemistry, and (c) the ratio of TLR agonist bound per Cdex depending on the conjugation chemistry.
FIG. 14 illustrates (a) the hydrodynamic diameter and (b) the colloidal stability of Cdex@TLR_{A1} in 1 M NaCl solution.
FIG. 15 is an immunofluorescence image of NFκB p65 signaling after 2-hour treatment with two types of TLR agonists and their corresponding Cdex@TLR conjugates as described in Example 2-5.
FIG. 16 shows the result of Western blot analysis of NFκB p65 signaling after 4-hour treatment of cells with a TLR agonist.
FIG. 17 shows the immunofluorescence images of NFκB p65 signaling following 4-hour and 6-hour treatments of different TLR agonists.
FIG. 18 illustrates the cell viability, measured by CCK-8 assay, after 4-hour treatment of cells with Cdex, TLR_{A1} agonist, Cdex@TLR_{A1}, and Cdex@TLR_{B1} at various concentrations.
FIG. 19 shows (a) the experimental design for in vivo efficacy evaluation after administration of control, doxorubicin, TLR_{A1} agonist, Cdex@TLR_{A1}, Cdex@TLR_{A1}X2 (double dose), and Cdex@TLR_{B1} to a xenograft mouse model, and (b) the observed results thereof.
FIG. 20 shows the structural formulas of exemplary branched polysaccharides or cyclic polysaccharides that are suitable for use in the polysaccharide-crosslinked colloidal particles in Example 3.
FIG. 21 illustrates confirmation of the formation of crosslinked structures using a UV-Vis spectrophotometer for Dextran T-5 (a), Maltodextrin (b), α-cyclodextrin (c), β-cyclodextrin (d), and Inulin (e) in Examples 3-1 to 3-5.
FIG. 22 shows MRI phantom images of materials synthesized by introducing iron into the crosslinked structures of Dextran T-5 (a), Maltodextrin (b), α-cyclodextrin (c), β-cyclodextrin (d), and Inulin (e) in Example 3-10 and their *r₁* and *r₂* relaxivity values (f).
FIG. 23 shows T1-weighted MRI images obtained from mice intravenously injected with materials synthesized by introducing iron into crosslinked structures synthesized from Dextran T-5, Maltodextrin, α-cyclodextrin , β-cyclodextrin, and Inulin in Example 3-11 (a) and signal-to-noise ratios (SNRs) before and after administration (b).
FIG. 24 shows T1-weighted MRI images obtained from mice intravenously injected with materials synthesized by introducing iron into crosslinked structures synthesized from Dextran T-5, Maltodextrin, α-cyclodextrin, β-cyclodextrin, and Inulin in Example 3-12, thereby confirming renal excretion.
FIG. 25 compares the structure (a), MRI phantom image (b), and relaxivity (d) of the dextran-crosslinked nanoparticle-based T1 MRI contrast agent NEMO-103 of Example 4-2 with those of a representative clinical gadolinium-based contrast agent (GBCA), Dotarem (c, e).
FIG. 26 shows the imaging phase timeline of magnetic resonance arthrography (MRA) used in the clinical comparison study between the dextran-crosslinked T1 MRI contrast agent NEMO-103 (iron-based contrast agent, IBCA) and GBCA.
FIG. 27 illustrates the clinical comparison study protocol between NEMO-103 and GBCA in Example 4-3.
FIG. 28 shows MRA images captured at 30 minutes (a) and 60 minutes (b) after administration of NEMO-103 in humans, and a comparative analysis in terms of contrast-to-noise ratio (CNR) (c), distension (d), and overall image quality (e).
FIG. 29 shows MRA images captured at 30 minutes after administration of NEMO-103 (a) and a gadolinium-based contrast agent (GBCA, b) in humans, with comparative evaluation in terms of CNR (c), distension (d), and overall image quality (e).
FIG. 30 shows MRA images captured approximately 60 minutes after administration of NEMO-103 (a, c) and GBCA (b, e) in humans, with comparative assessment of CNR (c), distension (d), and overall image quality (e).

### Mode for carrying out the invention

Hereinafter, the present invention will be described in further detail with reference to specific examples. However, these examples are provided for the purpose of illustrating the technical features of the invention more clearly and are not intended to limit the scope of the invention in any way.

### Example 1:

### 1-1. Synthesis of C-DNP-3 Using 3 kDa Dextran

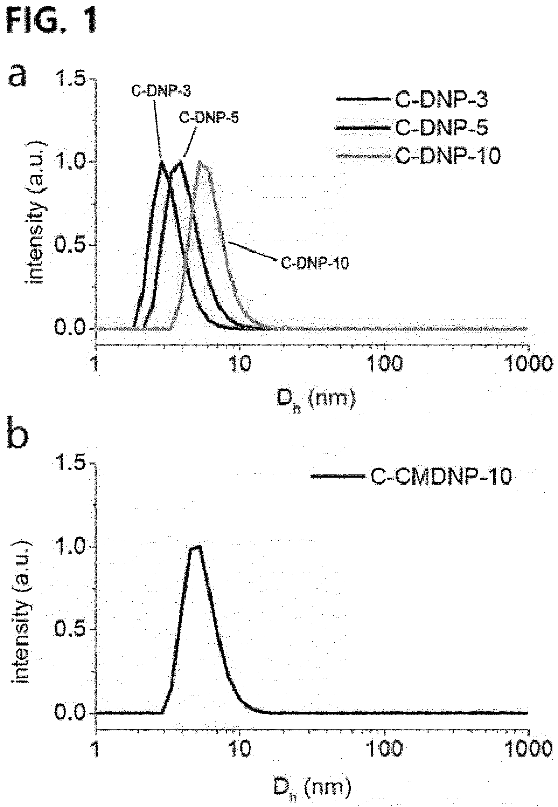

Dextran (molecular weight 3 kDa, 1 g) was dissolved in 4.2 mL of distilled water. To this solution, 8.3 mL of NaOH solution was added, followed by the addition of 3.3 mL of epichlorohydrin under stirring. Subsequently, 7 mL of diethylenetriamine (DETA) was added, and the reaction mixture was stirred for an additional 24 hours to yield C-DNP-3, a 3 kDa dextran-based crosslinked nanoparticle. The resulting product was purified by ultrafiltration. The hydrodynamic size, measured by dynamic light scattering (DLS), was 3 nm (FIG. 1a, blue).

### 1-2. Synthesis of C-DNP-5 Using 5 kDa Dextran

Except for replacing 3 kDa dextran with 5 kDa dextran, the synthesis was carried out under the same procedure as in Example 1-1, yielding C-DNP-5, a 5 kDa dextran-based crosslinked nanoparticle. The product was purified by ultrafiltration. The hydrodynamic size measured by DLS was 4 nm (FIG. 1a, green).

### 1-3. Synthesis of C-DNP-10 Using 10 kDa Dextran

Except for replacing 3 kDa dextran with 10 kDa dextran, the synthesis was carried out under the same procedure as in Example 1-1, yielding C-DNP-10, a 10 kDa dextran-based crosslinked nanoparticle. The product was purified by ultrafiltration. The hydrodynamic size measured by DLS was 5 nm (FIG. 1a, orange).

### 1-4. Synthesis of C-CMDNP-10 Using 10 kDa Carboxymethyl Dextran (CM-Dextran)

Except for replacing 3 kDa dextran with 10 kDa CM-dextran, the synthesis was carried out under the same procedure as in Example 1-1, yielding C-CMDNP-10, a 10 kDa CM-dextran-based crosslinked nanoparticle. The product was purified by ultrafiltration. The hydrodynamic size measured by DLS was 5 nm (FIG. 1b).

### 1-5. Substitution of Surface Functional Groups on C-DNP or C-CMDNP

### 1-5-1. Amine Group

The surface functional group of C-DNP or C-CMDNP synthesized in Examples 1-1 through 1-4 is an amine group.

### 1-5-2. Carboxyl Group

To 10 mL of C-DNP or C-CMDNP prepared in Examples 1-1 through 1-4, 30 mg of succinyl anhydride (SA) was added. The reaction mixture was stirred for 12 hours. The final product was purified by ultrafiltration.

### 1-5-3. Thiol Group

To 10 mL of C-DNP or C-CMDNP prepared in Examples 1-1 through 1-4, N-succinimidyl S-acetylthioacetate was added. The reaction mixture was stirred for 12 hours. The final product was purified by ultrafiltration.

### 1-5-4. Hydroxyl Group

To 10 mL of C-DNP or C-CMDNP synthesized in Examples 1-1 through 1-4, nitrous acid was added. The reaction mixture was stirred for 12 hours. The final product was purified by ultrafiltration.

### 1-6. Surface Charge Modulation of C-DNP and C-CMDNP

In 10 mL of C-DNP synthesized in Examples 1-1 through 1-3, when 30 mg of succinyl anhydride (SA) was added according to the procedure of Example 1-5-2, the measured surface charge was -3 mV. When 50 mg of SA was added, the surface charge was -20 mV. In the absence of added SA, the surface charge was +5 mV. Experiments conducted using C-DNP-3, C-DNP-5, and C-DNP-10 showed consistent results under the same substitution conditions, with respect to the amount of SA added and the resulting surface charge. In the case of 10 mL of C-CMDNP-10 synthesized in Example 1-4, which initially exhibited a surface charge of +5 mV, the addition of 30 mg of SA according to Example 1-5-2 yielded a surface charge of -4 mV. Representative results showing the change in surface charge of C-DNP-10 depending on the amount of SA added are presented in FIG. 2. These results demonstrate that increasing the amount of added SA leads to a more negative surface charge, thereby enabling tunable modulation of the surface potential of the colloidal particles.

### 1-7. Conjugation of Fluorescent Molecules into C-DNP and Control of the Conjugation density

1 mg of 5/6-carboxyfluorescein succinimidyl ester (NHS-fluorescein) was dissolved in 1 mL of DMSO. Aliquots of 5, 10, and 25 µL of this solution were added to 1 mg of C-DNP-5, which had surface amine groups according to Example 1-5-1 and a surface charge of +5 mV. After 24 hours of reaction, the fluorescein-introduced C-DNP-5 was purified by ultrafiltration. As shown in **Fig. 3****,** the number of fluorescein molecules conjugated to C-DNP-5 was 4 when reacted with 5 mmol of NHS-fluorescein/DMSO, 9 when reacted with 10 mmol, and 15 when reacted with 25 mmol. These results demonstrate that the number of molecules conjugated to C-DNP can be quantitatively controlled by adjusting the ratio of reactive moieties in the reaction.

### 1-8. Evaluation of Squeezability Property of C-DNP

C-DNP-10 (5 nm, size distribution ±11.2%, 10 mg) and gold nanoparticles (AuNPs, 5 nm, size distribution ±12%, 10 mg) dispersed in water were each placed in an ultrafiltration filter tube (regenerated cellulose membranes, manufactured by Merck Millipore; molecular weight cutoffs: 10 kDa (pore size: 2.85 nm), 50 kDa (pore size: 4.87 nm)). Centrifugation was performed at 1,000 rcf for 5, 10, and 20 minutes, respectively, and the amounts of C-DNP-10 and AuNPs that passed through the filters were measured (see FIGS. 4a and 4b). For C-DNP-10, the amount of dextran in the filtrate that passed through the filters was quantified using the phenol-sulfuric acid method. The quantities of C-DNP-10 that passed through the 10 kDa filter were 0.2 mg (2%) at 5 minutes, 0.29 mg (2.9%) at 10 minutes, and 0.57 mg (5.7%) at 20 minutes. The quantities that passed through the 50 kDa filter were 2.6 mg (26%) at 5 minutes, 3.93 mg (39.3%) at 10 minutes, and 4.58 mg (45.8%) at 20 minutes (see FIG. 4c). For gold nanoparticles, the amount of Au in the filtrate was analyzed using inductively coupled plasma (ICP), and no Au was detected in any of the filtrates through either the 10 kDa or 50 kDa filters at any of the time points (see FIG. 4d). Although C-DNP-10 has a size larger than the filter pore size, its detection in the filtrate indicates that it possesses squeezability.

### 1-9. Surface Coating of Inorganic Nanoparticles Using C-DNP/C-CMDNP

10 mg of 4 nm iron oxide nanoparticles (see FIGS. 5a and 5b) were dispersed in tetramethylammonium hydroxide (TMAOH) solution and stirred. The supernatant was removed by magnetic decantation, followed by two washes with 10 mL each of hexane and acetone. The washed iron oxide nanoparticles were then mixed with 100 mg of C-DNP-3, C-DNP-5, C-DNP-10, and C-CMDNP-10, each substituted with carboxyl groups according to Example 1-5-2, and stirred for 12 hours. The resulting C-DNP/C-CMDNP-coated iron oxide nanoparticles (IONP@C-DNP-3, IONP@C-DNP-5, IONP@C-DNP-10, IONP@C-CMDNP-10) were purified via ultrafiltration.

### 1-10. Hydrodynamic Size of Inorganic Nanoparticles Coated with C-DNP/C-CMDNP

The hydrodynamic sizes of IONP@C-DNP-3, IONP@C-DNP-5, and IONP@C-DNP-10 prepared in Example 1-9 were 7 nm, 9 nm, and 11 nm, respectively (see FIG. 5c). Because the carboxymethyl (CM) group is small (angstrom scale) and does not significantly affect the overall particle size (nanometer scale), the hydrodynamic size of IONP@C-CMDNP-10 prepared in Example 1-9 was also 11 nm (see FIG. 5d).

### 1-11. Surface Charge of Nanoparticles Coated with C-DNP Having Various Surface Charges

Using the C-DNP-3, C-DNP-5, and C-DNP-10 prepared in Example 1-6 with surface charges adjusted to -20 mV, -3 mV, and +5 mV at pH 7, the 4 nm iron oxide nanoparticles were surface-coated in accordance with Example 1-9. The surface charges of the coated nanoparticles were -20 mV when coated with -20 mV C-DNP, -3 mV when coated with -3 mV C-DNP, and +5 mV when coated with +5 mV C-DNP. This result indicates that the surface of the iron oxide nanoparticles was uniformly coated with C-DNP, as the resulting surface charges matched those of the C-DNP regardless of the original surface charge of the nanoparticles.

Representative data showing the surface charges of iron oxide nanoparticles coated with -20 mV, -3 mV, and +5 mV C-DNP-10 are provided in Fig. 6. Thus, the surface charge of coated nanoparticles can be controlled using C-DNPs with tunable surface charges.

### 1-12. Evaluation of Colloidal Stability of Iron Oxide Nanoparticles Coated with C-DNP and C-CMDNP

Solutions mimicking physiological conditions were prepared with varying salt concentrations (0, 0.15, 0.5, and 1.0 M NaCl) and pH values (5, 7, and 8). To each solution, 0.1 mg of iron oxide nanoparticles coated with C-DNP-10 or C-CMDNP-10 prepared in Example 1-9 (IONP@C-DNP-10, IONP@C-CMDNP-10) was added. Colloidal stability was monitored over 28 days. As shown in FIG. 7a, IONP@C-DNP-10 remained dispersed without aggregation or precipitation under all tested physiological conditions. In addition, as shown in FIG. 7b, no changes in hydrodynamic size were observed over the monitoring period, indicating excellent colloidal stability. Similarly, IONP@C-CMDNP-10 also showed excellent colloidal stability without precipitation, as illustrated in FIG. 8a.

### 1-13. Surface Coating of Inorganic Nanoparticles Using Non-crosslinked Native Dextran

10 mg of 4 nm iron oxide nanoparticles were dispersed in tetramethylammonium hydroxide (TMAOH) solution and stirred. The supernatant was removed by magnetic decantation, and the nanoparticles were washed twice with 10 mL of hexane and acetone, respectively. The washed iron oxide nanoparticles were mixed with 50, 100, 500, 1000, or 2000 mg of non-crosslinked native dextran (molecular weight: 10 kDa) and stirred for 12 hours. The dextran-coated nanoparticles were purified by ultrafiltration. The hydrodynamic sizes of the dextran-coated iron oxide nanoparticles were 160 nm, 85 nm, 49 nm, 26 nm, and 18 nm, respectively. As shown in FIG. 8b, the minimum hydrodynamic size of iron oxide nanoparticles achievable using native dextran (10 kDa) was 18 nm, requiring a minimum of 2000 mg of dextran. This is 20 times the amount of C-DNP-10 (100 mg) required for coating. In other words, when using non-crosslinked native dextran, 20 times more material is needed compared to C-DNP-10 to achieve colloidal stability of iron oxide nanoparticles. Furthermore, as shown in FIG. 8a, iron oxide nanoparticles coated with 100 mg of C-DNP-10 did not show precipitation during colloidal stability tests, whereas those coated with 100 mg of non-crosslinked native dextran showed precipitation within 7 days under physiological conditions. Thus, C-DNP enables coating with 1/20 the amount of dextran while ensuring superior colloidal stability.

This effect is attributed to the compact spherical structure of the dextran-crosslinked nanoparticles of the present invention, the strong coordination bonding between the crosslinker-derived functional groups (e.g., carboxyl or amine groups) and the iron oxide nanoparticle surface, and their excellent colloidal stability.

### 1-14. Surface Coating of Inorganic Nanoparticles Using Non-Crosslinked CM Dextran

10 mg of 4 nm iron oxide nanoparticles were added to tetramethylammonium hydroxide (TMAOH) solution and stirred. The supernatant was removed by magnetic decantation, and the nanoparticles were washed twice with 10 mL of hexane and acetone, respectively. The washed iron oxide nanoparticles were mixed with 2,000 mg of non-crosslinked carboxymethyl dextran (CM dextran, molecular weight: 10 kDa) and stirred for 12 hours. The CM dextran-coated nanoparticles were purified by ultrafiltration. The hydrodynamic size of the CM dextran-coated iron oxide nanoparticles was 18 nm. Additionally, as shown in Fig. 8a, iron oxide nanoparticles coated with 100 mg of C-CMDNP-10 did not show precipitation during colloidal stability tests, whereas those coated with 100 mg of non-crosslinked CM dextran showed precipitation within 7 days under physiological conditions.

### 1-15. Anti-opsonization Effect of C-DNP-coated Iron Oxide Nanoparticles

Magnetic nanoparticles coated with C-DNP-5 adjusted to surface charges of -20, - 3, and +5 mV, as prepared in Example 1-11, were mixed with fetal bovine serum (FBS) and incubated for 10 minutes. The mixtures were loaded onto 1% agarose gel and subjected to electrophoresis, and the migration of nanoparticles was visualized (FIG. 9). FIG. 9 compares the anti-opsonization effect of serum protein binding by evaluating band migration and broadening based on the surface charge of C-DNP-5-coated magnetic nanoparticles. Magnetic nanoparticles coated with C-DNP-5 with a surface charge of +5 mV migrated in the direction of the negative electrode during electrophoresis (FIG. 9a). However, when mixed with serum proteins, nonspecific adsorption increased the hydrodynamic size and caused surface charge randomization, leading to band broadening and altered migration direction (FIG. 9a). Nanoparticles coated with C-DNP-5 with a surface charge of -3 mV migrated slightly toward the positive electrode (FIG. 9b), and when mixed with serum proteins, no band broadening was observed, indicating that protein binding was inhibited and migration direction remained the same (FIG. 9b). Nanoparticles coated with C-DNP-5 with a surface charge of -20 mV showed significant migration toward the positive electrode (FIG. 9c), and mixing with serum proteins caused nonspecific binding, increasing the hydrodynamic size and resulting in band broadening (FIG. 9c).

Thus, as demonstrated in FIG. 9, the electrophoretic profiles of C-DNP-coated iron oxide nanoparticles confirm their anti-opsonization effect depending on surface charge modulation.

### 1-16. In Vivo Diffusion and Migration of C-DNP-coated Iron Oxide Nanoparticles in Tissue-like Matrix

IONP@C-DNP-5 (hydrodynamic size: 9 nm), prepared in Example 1-9, and citrate-coated gold nanoparticles (AuNP@citrate) with the same hydrodynamic size were each loaded (0.3 mg) into 1% agarose gel, which mimics tissue environments. Penetration into the agarose gel was photographed over time using a digital camera, and changes in area were measured (FIGS. 10a and 10b). The observed diffusion areas of IONP@C-DNP-5 were 2.5 mm² initially, 3.3 mm² after 1 hour, and 5.2 mm² after 3 hours (FIG. 10c). In contrast, AuNP@citrate showed initial area of 2.2 mm² and remained unchanged at 2.3 mm² at both 1 and 3 hours (FIG. 10c). Despite having the same hydrodynamic size as Au@citrate, IONP@C-DNP-5 penetrated the agarose matrix more rapidly, indicating enhanced diffusion and tissue mobility of C-DNP-coated nanoparticles.

### 1-17. Pharmacokinetic Analysis of C-DNP-coated Iron Oxide Nanoparticles by Tuning Particle Size and Surface Charge

As illustrated in FIG. 11, 4 nm iron oxide nanoparticles were coated with various C-DNPs and administered to animals, followed by time-dependent observation of MRI signal changes. Since 4 nm iron oxide exhibits both T1 and T2 contrast enhancement modes in MRI, these dual imaging modes were utilized for pharmacokinetic profiling.

An iron oxide nanoparticle coated with C-DNP-3 having a surface charge of -3 mV and hydrodynamic size of 3 nm exhibited a total hydrodynamic size of 7 nm. Because this hydrodynamic size is less than or equal to 8 nm and the surface charge is close to neutral (0 mV), the nanoparticle is not subject to phagocytosis by macrophages and can be excreted renally when administered intravenously. Accordingly, renal excretion of the C-DNP-3-coated iron oxide nanoparticle was confirmed in mice using T1-weighted MRI imaging (FIG. 11a). An iron oxide nanoparticle coated with C-DNP-10 having a surface charge of -3 mV and hydrodynamic size of 5 nm exhibited a total hydrodynamic size of 11 nm. Since its hydrodynamic size exceeds 8 nm and its surface charge is close to neutral, it is not readily phagocytosed and remains in the bloodstream for an extended period, making it suitable for prolonged vascular imaging (FIG. 11b). Therefore, even 30 minutes after intravenous injection in mice, cardiovascular structures such as the heart, aorta, and carotid arteries were clearly visualized by T1-weighted MRI. An iron oxide nanoparticle coated with C-DNP-10 having a surface charge of -30 mV and hydrodynamic size of 5 nm also exhibited a total hydrodynamic size of 11 nm. Given its size greater than 8 nm and a highly negative surface charge of -30 mV, the nanoparticle is readily phagocytosed by Kupffer cells in the liver after intravenous administration in mice, resulting in hepatic accumulation. This liver-specific accumulation was confirmed using T2-weighted MRI imaging (FIG. 11c).

### Example 2. Polysaccharide-Crosslinked Colloidal Particle-Drug Conjugate (Cdex@TLR as an Anticancer Agent)

The mechanism of tumor cell killing by TLR agonists is described in detail in the literature (Remaut et al., Eur. J. Pharm. Biopharm., 2022, 172, 16).

### 2-1. Preparation of Nanostructures (Cdex) Using Dextran T-10

A total of 180 µmol of Dextran T-10 (average molecular weight: 10,000 Da) was dissolved in 9 mL of distilled water, followed by the addition of epichlorohydrin and NaOH. Then, ethylenediamine was added, and the reaction mixture was stirred at room temperature (RT) for 24 hours. Subsequently, 25 mg of succinic anhydride was added to the reaction mixture, and succinylation was carried out for 24 hours at RT. The resulting product was purified by dialysis using a 10 kDa molecular weight cut-off (MWCO) membrane filter.

### 2-2. Drug Conjugation to Cdex (TLR 7/8 Agonist)

Among various commercially available TLR7 agonists, compounds containing amine groups that can react with the crosslinker-derived surface amine groups of Cdex (prepared in Example 2-1) were selected. Two TLR7 agonists (TLR_{A1} and TLR_{B1}) were chosen, which contain amine groups that do not interfere with the chemical site required for TLR7 binding.

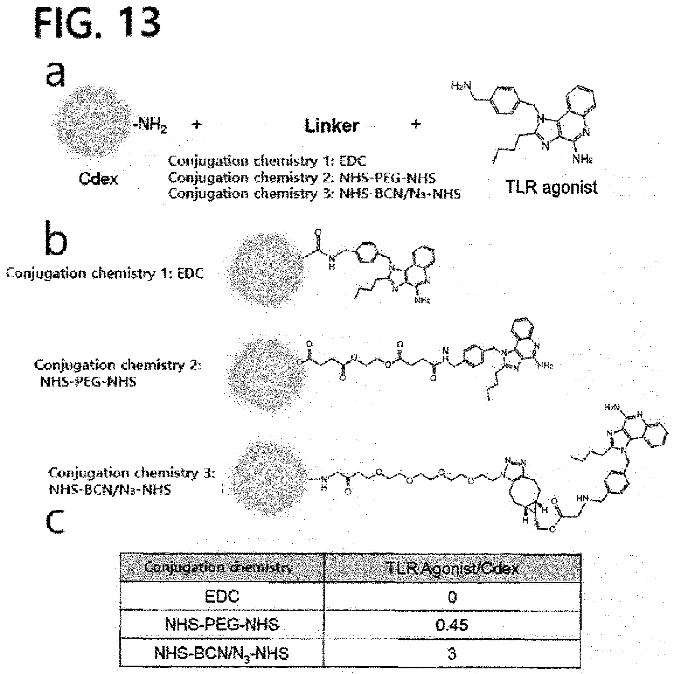

As illustrated in FIG. 13, the Cdex-TLR agonist conjugates (Cdex@TLR) were prepared using EDC (1-ethyl-3-(3-dimethylaminopropyl)carbodiimide), NHS-PEG-NHS (N-hydroxysuccinimide-polyethylene glycol-NHS), and BCN-NHS / N₃-NHS (azide-NHS) coupling chemistries (Fig. 13A, B). For the N₃-PEG-NHS conjugation, 30 mg of N₃-PEG-NHS was dissolved in 2 mL of DMSO and added to Cdex (Example 2-1), and the mixture was stirred for 2 hours at room temperature. Then, 10 mg of BCN-NHS and 10 mg of TLR_{A1} agonist were dissolved in 2 mL of DMSO and added to the mixture, followed by an additional 2-hour stirring. The product was purified by dialysis using a 10 kDa MWCO membrane. For NHS-PEG-NHS conjugation, 10 mg of TLRA1 agonist and 20 mg of NHS-PEG-NHS were added to Cdex and stirred for 2 hours at RT. The product was purified by dialysis using a 10 kDa MWCO membrane.

As a result, the number of TLR_{A1} agonists conjugated per Cdex was 3 for the NHS-BCN/N₃-NHS coupling method, and 0.45 for the NHS-PEG-NHS method. This indicates that the NHS-BCN/N₃-NHS chemistry provided the highest conjugation efficiency (FIG. 13c).

### 2-3. Quantification of Drug Conjugated to Cdex

The conjugated product from Example 2-2 was diluted to 0.5 µg/mL and loaded into a cuvette. The absorbance spectrum from 200 to 500 nm was measured using a UV-Vis spectrophotometer. While unconjugated Cdex showed no absorbance at 350 nm, the drug-conjugated Cdex exhibited a clear absorbance peak at 350 nm. This result enabled quantification of the drug content and determination of the number of TLRA1 agonist molecules per mole of Cdex. More than 3 TLR_{A1} agonist molecules were conjugated per Cdex.

### 2-4. Hydrodynamic Diameter and Colloidal Stability of Cdex@TLR

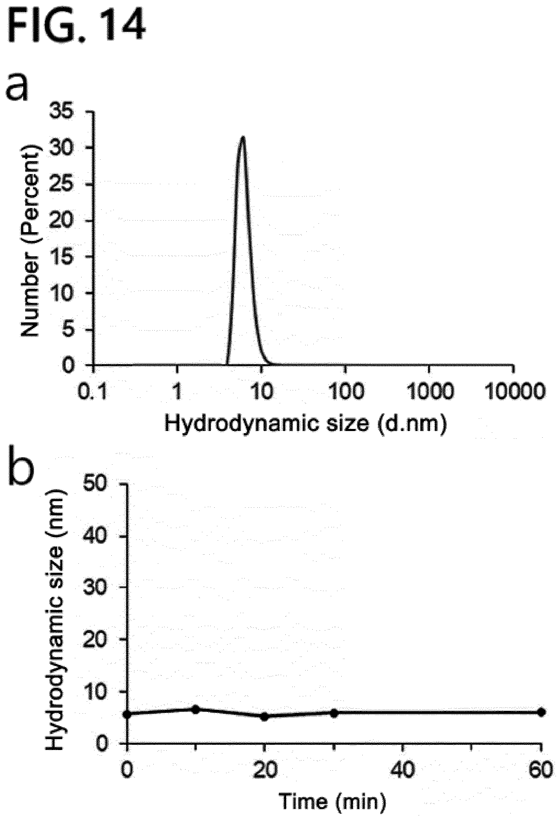

As shown in Fig. 14, the Cdex conjugated with TLR_{A1} agonist prepared in Example 2-2 exhibited a hydrodynamic diameter of 6 nm (FIG. 14a). The particle remained stably dispersed without aggregation even under harsh conditions of 1 M NaCl, which is significantly higher than physiological ionic strength (FIG. 14b).

### Example 2-5. In Vitro Efficacy Evaluation of Cdex@TLR for NF-κB p65 Signaling Activation

To evaluate the efficacy of the polysaccharide-crosslinked colloidal particle of the present invention as a drug delivery carrier, the ability to induce TLR (Toll-like receptor) signaling pathways (specifically, NF-κB signaling) in TLR-expressing cells was assessed.

To determine whether NF-κB signaling was activated upon treatment with Cdex@TLR prepared in Example 2-2, a representative immune cell line, RAW 264.7 macrophages, was employed to monitor TLR signaling activation.

Specifically, two Cdex@TLR samples, Cdex@TLR_{A1} and Cdex@TLR_{B1} (prepared in Example 2-2), as well as controls including Cdex alone and free TLR 7/8 agonists (TLR_{A1} and TLR_{B1}), were applied to RAW 264.7 cells. Immunofluorescence (IF) staining of NF-κB p65 was performed at the time points and concentrations specified in Table 1 to assess activation.

**[Table 1]**

| Treatment Group | Sample Name | Concentration (µM) | Incubation Time (h) | Detection |
|---|---|---|---|---|
| Control | Untreated | - | 2, 4, 6 | IF |
| | Cdex | 2.0, 3.0 | | |
| TLR 7/8 Agonist | TLR_{A1} | 6.0, 9.0 | | |
| | TLR_{B1} | | | |
| Cdex@TLR | Cdex@TLR_{A1}-3 | | | |
| | Cdex@TLR_{B1}-3 | | | |

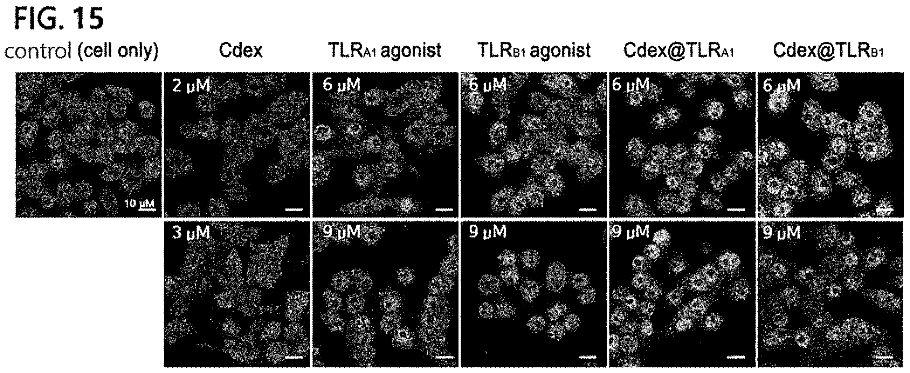

After 2 hours of treatment, immunofluorescence staining of NF-κB p65 revealed that, as shown in Fig. 15, in the untreated control and Cdex-treated cells, fluorescence was observed diffusely throughout the cytoplasm. In contrast, in the cells treated with TLR 7/8 agonists (TLR_{A1} and TLR_{B1}) and with Cdex@TLR_{A1} or Cdex@TLR_{B1}, strong NF-κB p65 fluorescence signals were observed predominantly in the nucleus compared to the cytoplasm. It is known that activation of the TLR signaling pathway leads to nuclear translocation of NF-κB p65, resulting in stronger fluorescence signals in the nucleus than in the cytoplasm. Since Cdex@TLR_{A1} and Cdex@TLR_{B1} exhibited stronger NF-κB p65 fluorescence than their respective free agonists, this suggests that these conjugates, Cdex@TLR_{A1} and Cdex@TLR_{B1} effectively induce TLR signaling activation. Moreover, fluorescence intensity did not significantly differ between treatment concentrations of 6 µM and 9 µM, indicating a saturation effect.

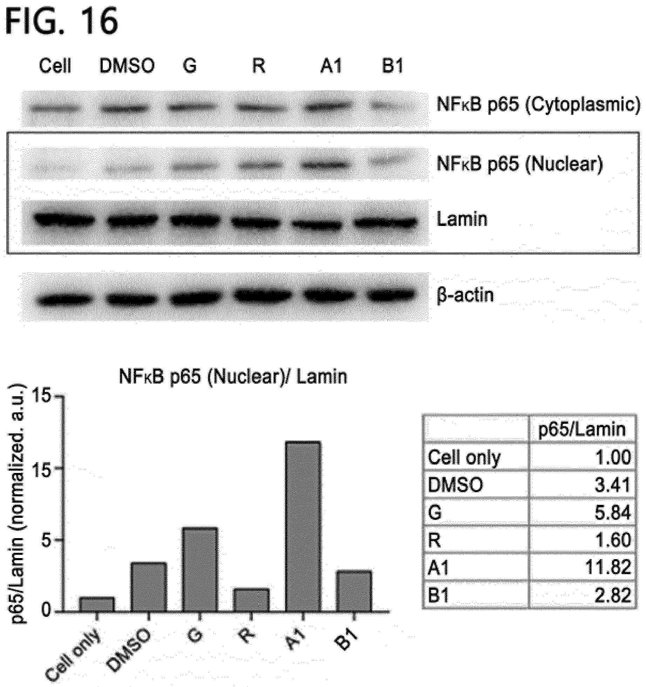

Among various TLR 7/8 agonists, the optimal agonist was selected based on the level of NF-κB p65 expression upon cellular treatment. Gardiquimod, Resiquimod, TLR_{A1}, and TLR_{B1} were each administered at 6 µM to RAW 264.7 macrophages for 4 hours. After incubation, the cells were lysed and fractionated into cytoplasmic and nuclear fractions, and NF-κB p65 expression was analyzed via Western blotting (Fig. 16). Lamin (nuclear) and β-actin (cytoplasmic) were used as housekeeping proteins for normalization. Among the four agonists, TLR_{A1} agonist showed the strongest expression of NF-κB p65 in the nuclear fraction. Compared to vehicle-treated controls (DMSO), Resiquimod and TLR_{B1} agonist showed relatively weaker nuclear NF-κB p65 expression.

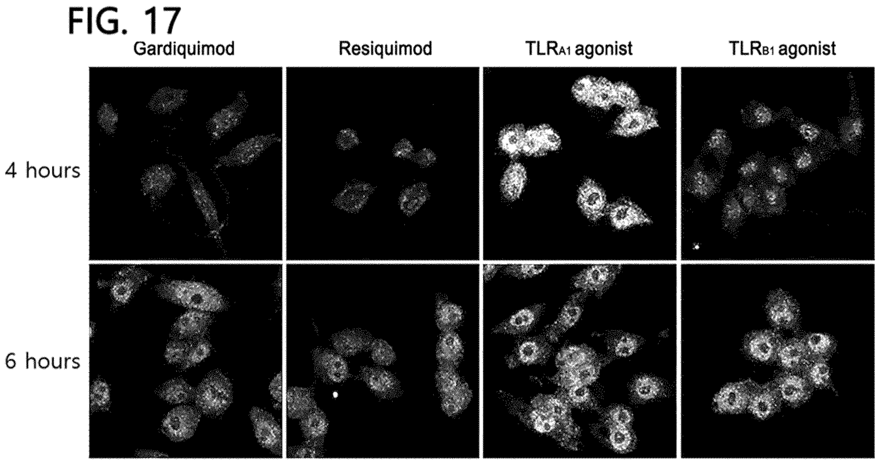

In a similar experiment, the same four agonists (Gardiquimod, Resiquimod, TLR_{A1}, and TLR_{B1}) were applied to RAW 264.7 macrophages at 6 µM for 4 and 6 hours, and NF-κB p65 expression was assessed via immunofluorescence (Fig. 17). TLR_{A1} agonist exhibited the highest nuclear NF-κB p65 expression at the 4-hour time point, confirming that it is the most potent inducer of TLR signaling activation under these conditions.

### Example 2-6. In Vitro Toxicity Assessment of Cdex@TLR

To evaluate cytotoxicity, two types of Cdex@TLR_{A1} and Cdex@TLR_{B1}-along with unconjugated Cdex and free TLR 7/8 agonists (TLR_{A1} and TLR_{B1}), were tested in vitro using varying concentrations and incubation times. The evaluation was conducted using RAW 264.7 macrophage cells which naturally express TLRs.

Specifically, treatment groups were categorized as untreated, low concentration, medium concentration, and high concentration. The samples were administered to cells, and the resulting cellular responses were evaluated using a Cell Counting Kit-8 (CCK-8) assay to determine cell viability across concentration and time points.

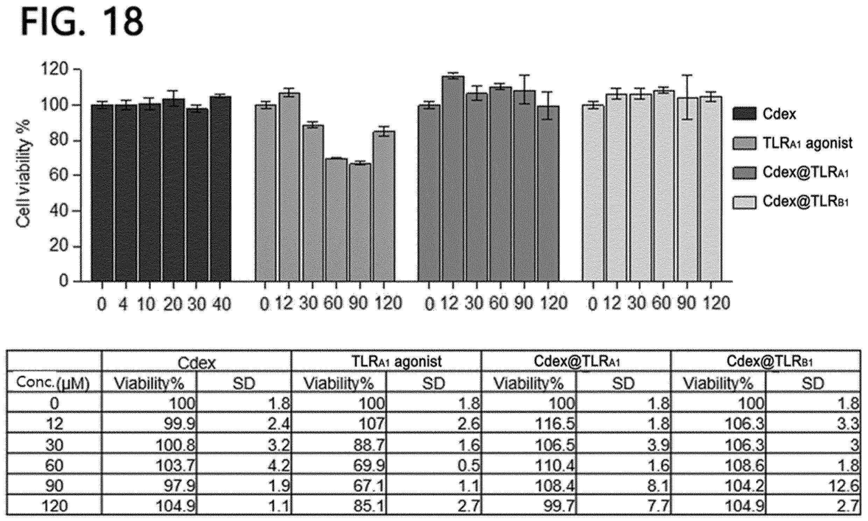

After 4 hours of treatment, CCK-8 assay results showed that Cdex alone exhibited no detectable cytotoxicity at any concentration tested. TLR_{A1} exhibited a reduction in cell viability to approximately 89% at a concentration of 30 µM. Both Cdex@TLR_{A1} and Cdex@TLR_{B1} showed no cytotoxicity within the concentration range tested (see FIG. 18).

### Example 2-7: In Vivo Antitumor Efficacy Assessment in a Cancer Xenograft Mouse Model

The antitumor efficacy of the test agents was evaluated by monitoring tumor volume changes following administration. The anti-cancer activity of Cdex@TLR_{A1} and Cdex@TLR_{B1} was confirmed via induction of an immune response and tumor cell death in a cancer xenograft mouse model.

BALB/c nude mice (6 weeks old) were used for the in vivo study. A breast cancer xenograft model was established by subcutaneous implantation of 1×10⁶ BT-474 human breast cancer cells mixed with Matrigel at a 1:1 volume ratio (total 100 µL) into the dorsal flank of each mouse.

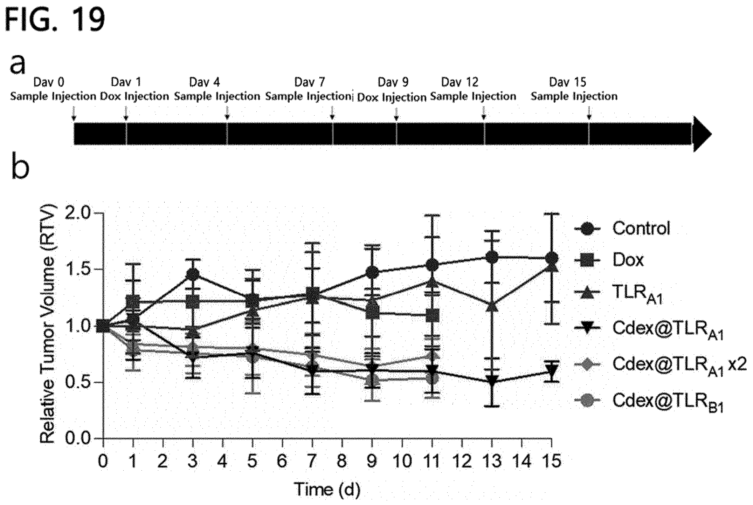

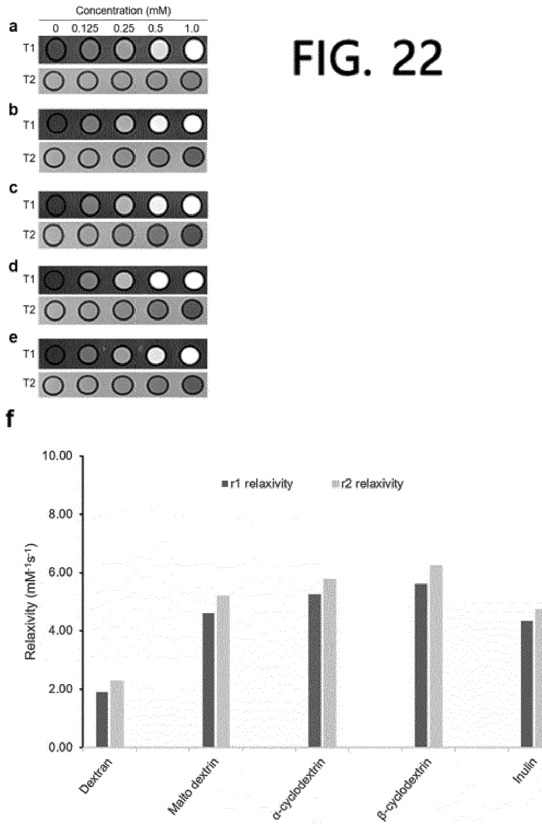

Once the tumor volume reached approximately 50 mm³, intratumoral injections of Cdex@TLR_{A1} and Cdex@TLR_{B1} were performed, and tumor volumes were measured over a 2-week period to evaluate efficacy. Dosing was carried out on days 0, 4, 7, 12, and 15 (see Fig. 22a). To enhance tumor cell killing, all groups except the negative control received Doxorubicin on days 1 and 9. A Doxorubicin-only group was also included for comparative purposes (see Fig. 19A and Table 2).

**[Table 2]**

| Group | Sample Name | TLR/Cdex | Concentration | Dose (µL) | 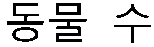 |
|---|---|---|---|---|---|
| 1 | Control (lXPBS) | | - | 20 | 3 |
| 2 | Doxorubicin (Dox) only | | 2 mg/kg | 20 | 4 |
| 3 | TLR_{A1} agonist | | 6 nmol/mL (0.3 nmol) | 20 | 3 |
| 4 | Cdex@TLR_{A1} | 2.4 | 6 nmol/mL (0.3 nmol) | 20 | 3 |
| 5 | Cdex@TLR_{A1} x2 | 2.4 | 12 nmol/mL (0.6 nmol) | 20 | 4 |
| 6 | Cdex@TLR_{B1} | 2.8 | 6 nmol/mL (0.3 nmol) | 20 | 3 |

Compared to the PBS control group and to groups receiving either free TLR_{A1} or doxorubicin alone, mice treated with Cdex@TLR_{A1}, Cdex@TLR_{A1} x2 (administered twice), and Cdex@TLR_{B1} exhibited a progressive reduction in relative tumor volume over time.

Compared to the control group (PBS injection), treatment with Cdex@TLR_{A1}, Cdex@TLR_{A1} x2, and Cdex@TLR_{B1} resulted in a time-dependent decrease in relative tumor volume, indicating a therapeutic effect against cancer. In contrast, the groups treated with doxorubicin alone or the TLR_{A1} agonist showed a slower rate of tumor growth, but no reduction in tumor size was observed.

### Example 3

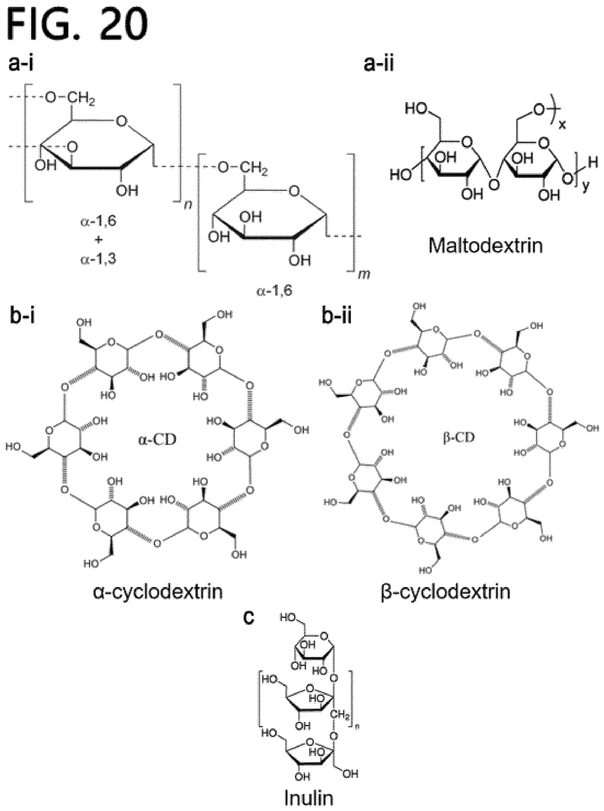

This example investigates whether crosslinking various polysaccharides including dextran (see FIG. 20) can form polysaccharide-crosslinked colloidal particles usable as T1 MRI contrast agents.

### 3-1. Synthesis of Nanostructures Using Dextran T-5

A total of 180 µmol of dextran T-5 (FIG. 20 (a-i), average molecular weight: 5,000 Da) was dissolved in 9 mL of distilled water, followed by the addition of epichlorohydrin and NaOH. Subsequently,diethylenetriamine was added, and the mixture was stirred at room temperature (RT) for 24 hours. The resulting product was purified using a 5 kDa molecular weight cut-off (MWCO) filter.

### 3-2. Synthesis of Nanostructures Using Maltodextrin

The procedure was identical to that described in Example 3-1, except that maltodextrin (FIG. 20 (a-ii), average molecular weight: 990 Da) was used in place of dextran T-5.

### 3-3. Synthesis of Nanostructures Using α-Cyclodextrin

The procedure was identical to that described in Example 3-1, except that α-cyclodextrin (FIG. 20 (b-i), average molecular weight: 970 Da) was used in place of dextran T-5.

### 3-4. Synthesis of Nanostructures Using β-Cyclodextrin

The procedure was identical to that described in Example 3-1, except that β-cyclodextrin (FIG. 20 (b-ii), average molecular weight: 1,100 Da) was used in place of dextran T-5.

### 3-5. Synthesis of Nanostructures Using Inulin

The procedure was identical to that described in Example 3-1, except that inulin (FIG. 20 (c), average molecular weight: 2,500 Da) was used in place of dextran T-5.

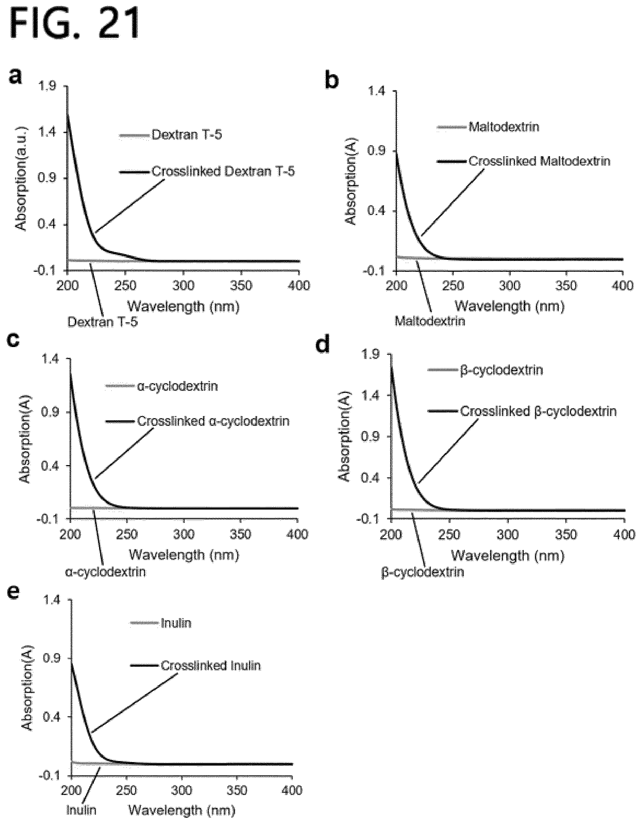

To confirm the crosslinking of the polysaccharides synthesized in Examples 3-1 to 3-5, each sample was prepared at an equal concentration, placed in a cuvette, and analyzed using a UV-Vis spectrophotometer in the 200-400 nm wavelength range. While typical polysaccharides do not exhibit absorbance in the visible range, nanostructures formed by crosslinking of polysaccharides scatter transmitted light and thereby exhibit an absorption phenomenon at shorter wavelengths. As shown in FIG. 21, the polysaccharides before crosslinking showed no absorbance in the observed range (FIG. 21, orange line), whereas the crosslinked polysaccharides exhibited absorption in the short-wavelength range (200-250 nm), confirming the formation of nanostructures via crosslinking of polysaccharides (FIG. 21, black line).

The nanostructures synthesized in Examples 3-1 through 3-5 contained surface amine groups introduced via diethylenetriamine. To quantify the number of surface amine groups per nanostructure, an o-phthalaldehyde assay was performed. The results showed that dextran T-5 contained 12.7 amine groups, maltodextrin 4.5, α-cyclodextrin 8.1, β-cyclodextrin 9.0, and inulin 8.0. This indicates that the synthesis method consistently introduces amine groups onto the surface of all nanostructures.

### 3-6. Introduction of Carboxyl Groups onto Nanostructures

To each of the nanostructures prepared in Examples 3-1 to 3-5, 25 mg of succinic anhydride was added and the mixture was stirred for 24 hours at room temperature to induce a succinylation reaction. The product was purified using a 5 kDa MWCO filter.

The number of residual amine groups in the nanostructures formed by crosslinkning of polysaccharides from Example 3-6 was measured using the o-phthalaldehyde assay. The number of residual amine groups per nanostructure was as follows: dextran T-5, 0.7; maltodextrin, 0.2; α-cyclodextrin, 0.2; β-cyclodextrin, 0.2; and inulin, 0.4. A value of less than or equal to 1 indicates that all amine groups were converted into carboxyl groups. Therefore, it was confirmed that the amine groups on the nanostructures derived from various polysaccharides were successfully replaced with carboxyl groups in this reaction.

### 3-7. Iron Loading onto Nanostructures

To the carboxylated nanostructures of Example 3-6, 45 µL of ferric chloride hexahydrate solution was added. The pH was adjusted to 8 using 2.5 M NaOH, and the mixture was reacted at room temperature for 1 hour. The product was purified using a 5 kDa MWCO filter to yield the iron-loaded nanostructures.

The iron content and polymer content of the iron-loaded nanostructures prepared in Example 3-7 were analyzed. Surface-bound iron was measured using inductively coupled plasma (ICP) analysis, and the content of each polymer was determined by the phenol-sulfuric acid method. The amount of iron bound per 1 mg of polymer was as follows: dextran T-5, 0.03 mg; maltodextrin, 0.026 mg; α-cyclodextrin, 0.033 mg; β-cyclodextrin, 0.026 mg; and inulin, 0.029 mg. These results confirmed that similar amounts of iron were introduced across different polymer-based nanostructures under the same reaction conditions.

### 3-8. Measurement of Hydrodynamic Diameter and Surface Charge of Iron-Loaded Nanostructures

The hydrodynamic diameter of the iron-loaded nanostructures obtained in Example 3-7 was analyzed using dynamic light scattering (DLS). The results indicated that dextran T-5 exhibited a diameter of 3.6 nm, maltodextrin 6.8 nm, α-cyclodextrin 2.9 nm, β-cyclodextrin 2.8 nm, and inulin 3.8 nm, confirming that the synthesized nanostructures possess similar hydrodynamic sizes. Surface charge measurements showed values of -3.01 mV for dextran T-5, - 6.62 mV for maltodextrin, -9.06 mV for α-cyclodextrin, -7.32 mV for β-cyclodextrin, and -2.83 mV for inulin. These results confirm that the synthesized iron-loaded nanostructures exhibit comparable surface charge and hydrodynamic diameter.

### 3-9. Viscosity Comparison of Nanostructures

The nanostructures from Example 3-7 were prepared at a concentration of 5 mg/mL, and viscosity measurements were performed at 25°C. The viscosity values of the crosslinked polymers were 1.09 mPa·s for dextran T-5, 1.43 mPa·s for maltodextrin, 1.26 mPa·s for α-cyclodextrin, 1.30 mPa·s for β-cyclodextrin, and 1.14 mPa·s for inulin, indicating similar viscosity profiles among the nanostructures.

### 3-10. T1 MRI Contrast Effect Evaluation of Iron Loaded Nanostructures

To evaluate the T1 MRI performance of the materials from Example 3-7, both the spin-spin relaxivity coefficient (r₂) and spin-lattice relaxivity coefficient (r₁) were measured, and the r₂/r₁ ratio was calculated. The r₂/r₁ ratio is a critical indicator in determining whether a contrast agent is suitable for T1 or T2 MRI. As shown in Fig. 22, 3.0 Tesla MRI analysis revealed the following: dextran T-5 exhibited an r₁ of 1.90 and r₂ of 2.27 (r₂/r₁ = 1.19)(a, f); maltodextrin exhibited r₁ = 4.60, r₂ = 5.19 (r₂/r₁ = 1.13)(b, f); α-cyclodextrin exhibited r₁ = 5.26, r₂ = 5.78 (r₂/r₁ = 1.10)(c, f); β-cyclodextrin exhibited r₁ = 5.62, r₂ = 6.24 (r₂/r₁ = 1.11)(d, f); and inulin exhibited r₁ = 4.33, r₂ = 4.73 (r₂/r₁ = 1.09)(e, f). These results indicate that all synthesized nanostructures possess r₂/r₁ ratios close to 1, thereby confirming their suitability as T1 MRI contrast agents.

### 3-11. In Vivo Contrast Efficacy Evaluation of Iron-Loaded Nanostructures

The nanostructures from Example 3-7 were administered intravenously to mice, and T1-weighted images were acquired using 9.4 Tesla MRI. Male Balb/c mice (5 weeks or older) were anesthetized and injected via the tail vein with the nanostructures. T1-weighted MRI images were obtained at various time points, and signal-to-noise ratio (SNR) was analyzed. As shown in FIG. 23, all iron-loaded nanostructures from Example 3-7 exhibited bright vascular signals (e.g., in the jugular vein) within approximately 3-5 minutes post-injection.

### 3-12. Renal Excretion of Iron-Loaded Nanostructures

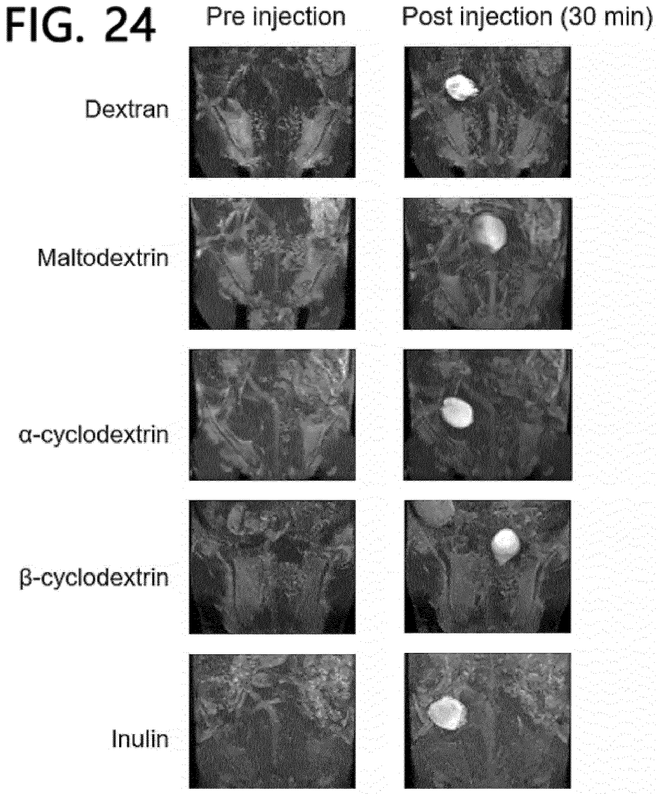

Following intravenous administration of the nanostructures from Example 3-7, T1-weighted MRI scans were performed for up to 1 hour using a 9.4 Tesla MRI to evaluate renal clearance. As shown in FIG. 24, T1 signals began to appear in the bladder within 30 minutes for most iron-loaded nanostructures. These observations indicate that the administered agents passed through the kidneys and were excreted into the bladder, confirming renal clearance.

### Example 4: Shoulder MR Arthrography Using the Dextran-Crosslinked T1 MRI Contrast Agent NEMO-103

### 4-1. Preparation of the Dextran-Crosslinked T1 MRI Contrast Agent NEMO-103

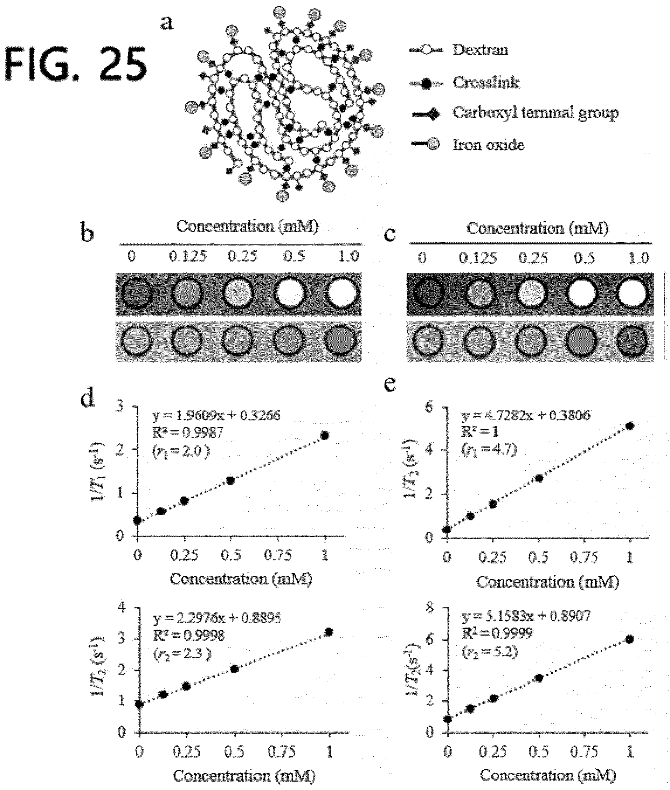

Dextran-crosslinked nanoparticles were synthesized by mixing an aqueous solution of 20 mM dextran T10 with sodium hydroxide solution and epichlorohydrin, followed by the addition of ethylenediamine. The resulting dextran-crosslinked nanoparticles were purified using a 10 kDa molecular weight cutoff (MWCO) filter. The terminal amine groups of the dextran-crosslinked nanoparticles were then substituted with carboxyl groups via reaction with succinic anhydride. After further purification using a 10 kDa MWCO filter, the carboxylated dextran-crosslinked nanoparticles were reacted with ferric chloride solution for 1 hour and subsequently purified with a 10 kDa MWCO filter to yield the dextran-crosslinked T1 MRI contrast agent NEMO-103 (FIG. 25a).

### 4-2. Phantom study

In a phantom study using a 3T MRI scanner, NEMO-103 exhibited bright T1 signal intensity on T1-weighted images, comparable to that of Dotarem^{®} (FIGS. 25b and 25c). The measured r₁ and r₂ relaxivity values of NEMO-103 were 2.0 mM⁻¹s⁻¹ and 2.3 mM⁻¹s⁻¹, respectively (FIGS. 25d and 25e). The r₂/r₁ ratio, which is an important parameter for T1 MRI contrast agents, was calculated to be 1.15. Given that an ideal T1 contrast agent exhibits an r₂/r₁ ratio close to 1, the observed ratio of 1.15 indicates that NEMO-103 is an ideal T1 MRI contrast agent. For comparison, the measured r₁ and r₂ values of Dotarem^{®} were 4.7 mM⁻¹s⁻¹ and 5.2 mM⁻¹s⁻¹, respectively, resulting in an r₂/r₁ ratio of 1.11.

### 4-3. Image Quality Assessment

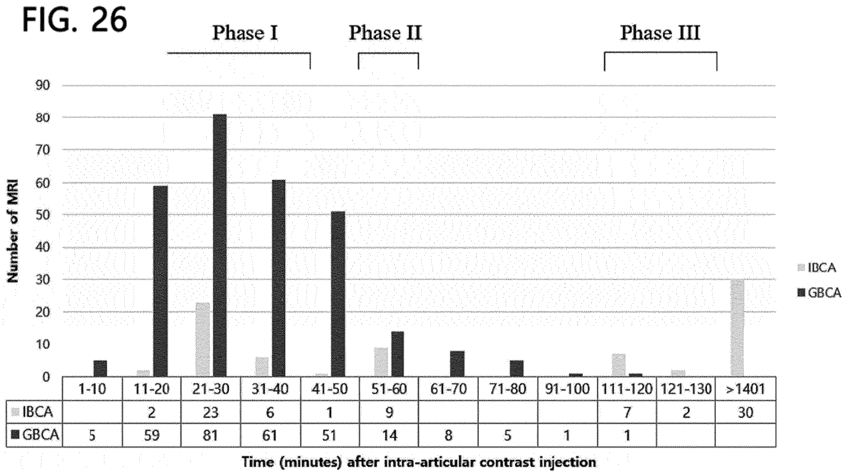

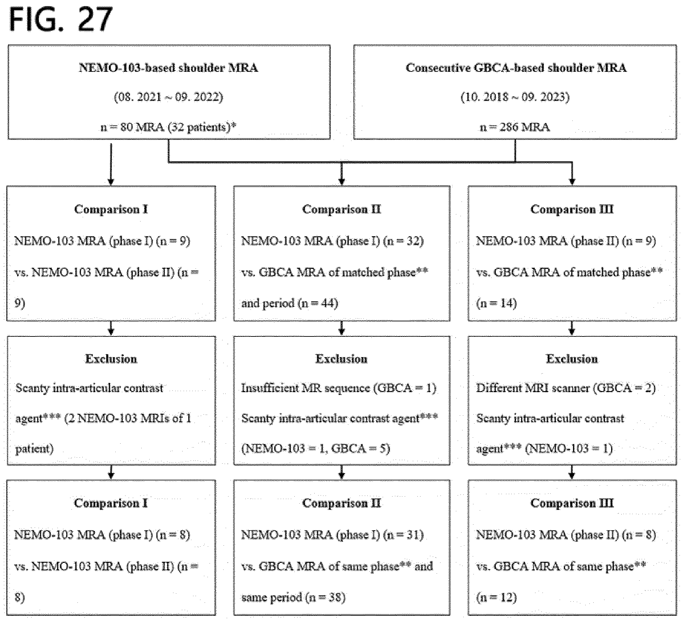

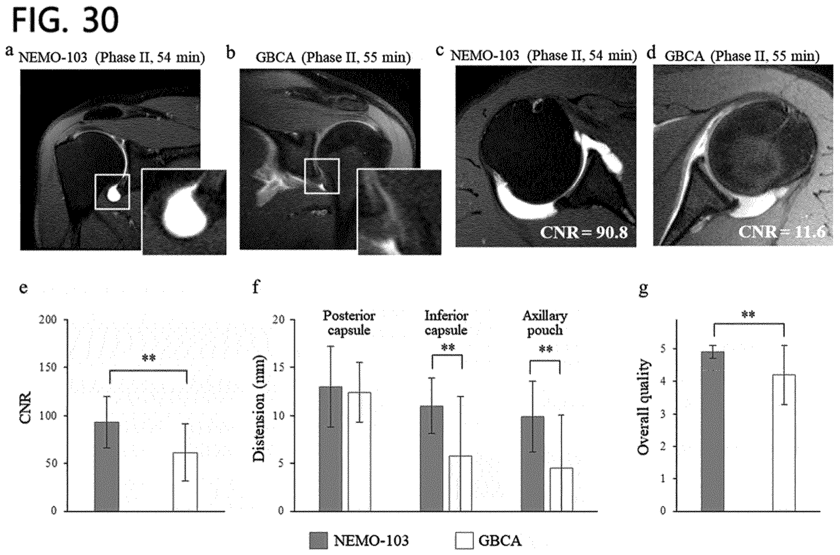

FIGS. 26 and 27 illustrate the phase design and image quality assessment method of a clinical comparative study between NEMO-103, a dextran-crosslinked T1 MRI contrast agent, and Dotarem^{®}, a representative clinical gadolinium-based contrast agent (GBCA) for T1 MRI. Phase I refers to MR arthrography (MRA) images acquired within 30 minutes after contrast agent administration. Phase II refers to MRA images acquired between 30 and 60 minutes post-administration, and Phase III refers to MRA images acquired between 110 and 130 minutes post-administration. In Comparison 1, no quantitative or qualitative differences were observed between NEMO-103-based MRA images acquired during Phase I and Phase II (FIG. 28). In Comparison 2, when comparing NEMO-103-based MRA images from Phase I with GBCA-based MRA images, no difference was observed in contrast-to-noise ratio (CNR); however, statistically significant differences were found in capsular distension (p < 0.05, FIG. 29). Specifically, NEMO-103-based MRA images showed higher scores in overall image quality. Although no significant difference in posterior capsular distension was observed between the two groups, NEMO-103-based MRA images showed superior distension in both the inferior capsule and the axillary pouch. In Comparison 3, which compared NEMO-103-based MRA images from Phase II with GBCA-based MRA images, more prominent differences were observed (FIG. 30). The CNR was significantly higher in the NEMO-103 group than in the GBCA group. In addition, more pronounced differences in inferior capsular and axillary pouch distension were observed. As a result, NEMO-103-based MRA images achieved superior outcomes in both inferior capsular and axillary pouch distension.

### 4-4. Visual Turing Test (VTT)

In the VTT for Comparison 1, radiologists were instructed to distinguish between NEMO-103-based MRA images obtained in Phase I and Phase II. The combined accuracy of the 8 testers was 46.8% (146/312), showing no statistically significant difference compared to random guessing (50.0%, 156/312; p = 0.423). In the VTT for Comparison 2, radiologists were instructed to differentiate between NEMO-103-based and GBCA-based MRA images acquired in Phase I. The combined accuracy of the 32 testers averaged 53.3% (624/1170), with no statistically significant difference compared to random guessing (50.0%, 585/1170; p = 0.107).

### [Discussion]

Amid growing safety concerns surrounding gadolinium-based contrast agents (GBCAs), iron (Fe)-based alternatives have emerged as a subject of active investigation. The present example evaluates the efficacy of NEMO-103 in comparison with a GBCA by assessing contrast-to-noise ratio (CNR) and MR arthrography (MRA) image quality. In direct intra-articular shoulder MRA, NEMO-103 (0.035 mg Fe/kg) and GBCA (0.037 mg Gd/kg) were each administered via direct joint injection, and the resulting CNR and visual Turing test (VTT) outcomes at 30 minutes post-injection (Comparison 2) demonstrated nearly equivalent image quality. Both contrast agents effectively delineated key anatomical structures, including tissue contrast, sharpness, rotator cuff tendons, labral structures, and adjacent cartilage. Furthermore, NEMO-103 maintained an enhanced CNR even at 120 minutes post-injection.

According to this example, the MRA image quality at 30 minutes (Phase I) and 60 minutes (Phase II) post-injection could not be distinguished in the visual Turing test, suggesting the potential extension of the imaging time window following injection. This may be advantageous for improving the operational efficiency of MR imaging facilities in clinical settings. According to current reports, particulate contrast agents with a hydrodynamic diameter of 3 nm are excreted from the joint cavity solely via lymphatic vessels due to their larger size and are not cleared through the venous system. Given that NEMO-103 has a larger hydrodynamic size (approximately 4.0 nm), it can only be excreted via lymphatic drainage, leading to prolonged retention within the joint cavity. In contrast, GBCAs (<1 nm) are rapidly cleared through both lymphatic and venous routes, thus limiting the time window available for image acquisition. However, in the MRA images taken 24 hours after administration, complete clearance of NEMO-103 from the joint cavity was confirmed, and no residual contrast agent was detected.

On the other hand, there was no statistically significant difference in posterior capsular distension between the MRA images based on NEMO-103 and those based on GBCA. This is presumed to be attributable to the patient's positioning during shoulder MRA. In the supine position, joint fluid tends to shift posteriorly, resulting in relatively less residual fluid in anatomical structures such as the undersurface of the subscapularis tendon and the axillary pouch. Over time, as the contrast agent is resorbed, this difference becomes more pronounced in the case of GBCA. Consequently, GBCA exhibited a relatively higher incidence of axillary pouch obliteration compared to NEMO-103. Moreover, the anterior aspect of the glenohumeral joint includes critical structures such as the subscapularis tendon and the anteroinferior glenoid labrum. Since multiple structures must be evaluated in this region, resorption of joint fluid and positional shifts caused by the supine posture may affect the conspicuity of lesions in this anterior compartment.

Unlike the long-established use of GBCAs, the novel contrast agent NEMO-103 requires safety evaluation. According to the clinical study report, treatment-emergent adverse events (TEAEs) were observed in 3 out of 32 subjects (9.4%) who underwent NEMO-103-based MRA, including two cases of localized injection-site reactions (muscle pain) and one case of hypertension. These adverse events were considered "unrelated to the investigational drug" in terms of causality, and all subjects recovered without medical intervention. Furthermore, no drug-related adverse events (ADRs), serious adverse events (SAEs), or severe adverse events occurred, and no subject discontinued participation due to these events. Evaluation of residual NEMO-103 in the liver and spleen at 24 hours post-injection confirmed complete clearance in all 30 subjects (100%) who underwent delayed imaging. The relative signal intensity (rSI) differences between the pre-contrast abdominal MRI and the 24-hour delayed MRI were all positive (liver: 13.2 ± 4.0; spleen: 5.5 ± 0.9), indicating elimination of the contrast agent. Two subjects did not undergo 24-hour delayed imaging that included liver and spleen evaluation.

Another advantage of NEMO-103 lies in its potential to eliminate reliance on conventional off-label use. Traditional GBCA-based MR arthrography requires a laborious dilution process of approximately 1:200, which increases the risk of contamination and infection, while also hindering standardization of contrast agent concentration. In contrast, NEMO-103 is supplied as a vial formulation pre-diluted to a concentration suitable for MR arthrography, thereby offering relative freedom from issues such as infection, contamination, and heterogeneity in contrast concentration.

As a retrospective study involving a limited number of patients, the present investigation has several limitations. First, since the subjects were not patients who underwent arthroscopic surgery, the diagnostic accuracy of NEMO-103-based shoulder MRA and GBCA-based shoulder MRA for individual lesions could not be evaluated with reference to surgical findings. However, by emphasizing the role of the contrast agent within the joint cavity-such as enhancement of contrast between surrounding structures and distension of the joint space-meaningful differences over time between MRA images using NEMO-103 and those using GBCA were observed. In addition, through a visual Turing test (VTT) involving a large number of testers, NEMO-103 demonstrated image quality comparable to that of GBCA from various perspectives. Future clinical trials may be designed to involve patients requiring surgical intervention, using operative records as the gold standard to evaluate the diagnostic accuracy of both contrast agents. Second, this study did not assess both contrast agents in the same subject. Individual variability may exist in joint cavity distension and contrast agent resorption. However, performing two MRAs using different contrast agents within a short time interval in the same patient presents a substantial challenge in study design. To overcome this limitation, the time interval between contrast agent injection and image acquisition was carefully reviewed, and phase matching was applied for comparative analysis. Third, it is noteworthy that the GBCA group was significantly older than the NEMO-103 group. Although not definitively proven, potential age-related differences in contrast agent resorption rates may exist. Nevertheless, considering that synovial fluid volume tends to increase with more severe degeneration and symptoms such as pain, it is plausible that the GBCA group, consisting of actual patients, had a greater synovial fluid volume than the relatively younger, volunteer-based NEMO-103 group.

In conclusion, NEMO-103-based MR arthrography of the shoulder is, at minimum, interchangeable with GBCA-based MR arthrography in terms of contrast-to-noise ratio (CNR), joint distension, image quality, and VTT performance. Moreover, it demonstrated enhanced resistance to contrast agent resorption over time, suggesting a potential extension of the feasible imaging time window.

## Claims

1. An in vivo administrable complex comprising:
(a) a polysaccharide-crosslinked colloidal particle formed by intra- and/or inter-molecular crosslinking of 1 to 3 branched polysaccharides or 2 to 30 cyclic polysaccharides at hydroxyl groups of constituent monosaccharide building blocks using a crosslinker; and
(b) a functional nanoparticle or a drug for in vivo administration, bound to a surface-exposed functional group derived from the crosslinker;
wherein the type and number of the crosslinker-derived functional groups available for conjugating the functional nanoparticle or the drug are controlled;
and wherein the polysaccharide-crosslinked colloidal particle and/or the in vivo administrable complex is engineered to be dispersible in vivo without aggregation, and to be squeezable, such that it is capable of migrating within in vivo structures due to its soft and deformable characteristics.

2. The in vivo administrable complex of claim 1,
wherein the polysaccharide-crosslinked colloidal particle is engineered to have a hydrodynamic diameter of 2 to 20 nm, preferably 10 nm or less, more preferably 8 nm or less, and to have surface-exposed carboxyl groups such that the surface charge is between -20 mV and 0 mV,
such that the particle is capable of undergoing renal clearance, is impermeable to vascular walls of normal blood capillaries, and is selectively drained into lymphatic vessels.

3. The in vivo administrable complex of claim 1,
wherein the polysaccharide-crosslinked colloidal particle is engineered to prolong residence time at the administration site, whereby enhancement of interstitial space and distension of anatomical structures at the administration site are induced.

4. The in vivo administrable complex of claim 1,
wherein, when the polysaccharide-crosslinked colloidal particle is formed by intra- and/or inter-molecular crosslinking of branched polysaccharides or cyclic polysaccharides at hydroxyl groups of constituent monosaccharide units using a crosslinker,
the ratio of the monosaccharide units crosslinked by the crosslinker is controlled to be from 2% to 60% relative to the total number of monosaccharide units, such that the particle is bio-inert by minimizing interactions with biological systems or biological responses, and/or is filterable through the kidney.

5. The in vivo administrable complex of claim 1,
wherein the substitution ratio of the crosslinker in the polysaccharide-crosslinked colloidal particle is controlled to be from 2% to 50% of the total number of reactive groups on the polysaccharide,
and wherein 2% to 98% of the crosslinkers are configured such that one terminal end remains unreacted and is exposed on the particle surface, such that the surface charge of the polysaccharide-crosslinked colloidal particle is controllable.

6. The in vivo administrable complex of claim 1,
wherein the delivery efficiency to tumor tissue and/or renal excretion potential is enhanced relative to the unmodified functional nanoparticle or drug, such that the in vivo administrable complex is capable of migrating within in vivo structures due to the squeezability of the polysaccharide-crosslinked colloidal particle.

7. The in vivo administrable complex of claim 1,
wherein the biodistribution and excretion profile of the functional nanoparticle or the drug is regulated by surface modification or conjugation with the polysaccharide-crosslinked colloidal particle.

8. The in vivo administrable complex of claim 1,
wherein the in vivo administrable complex is engineered to be either phagocytosed or not phagocytosed by macrophages, depending on surface modification or conjugation with the polysaccharide-crosslinked colloidal particle.

9. The in vivo administrable complex of claim 1,
wherein the in vivo structures comprise blood capillaries, lymphatic vessels, gastrointestinal tract, extracellular matrix (ECM), or intracellular matrix.

10. The in vivo administrable complex of claim 1,
wherein the complex comprises:
(i) a polysaccharide-crosslinked colloidal particle-drug conjugate in which a drug is conjugated directly or via a linker to the polysaccharide-crosslinked colloidal particle;
(ii) a functional nanoparticle whose surface is modified with a polysaccharide-crosslinked colloidal particle;
(iii) a functional nanoparticle modified with a polysaccharide-crosslinked colloidal particle, which in turn carries a drug; or
(iv) a functional nanoparticle modified by a drug-loaded polysaccharide-crosslinked colloidal particle.

11. The in vivo administrable complex of claim 1,
wherein the hydrodynamic diameter of the in vivo administrable complex is controlled by adjusting the coating thickness or the amount of the polysaccharide-crosslinked colloidal particle used for surface modification or conjugation.

12. The in vivo administrable complex of claim 1,
wherein the polysaccharide-crosslinked colloidal particle is stable in plasma without aggregation, is not metabolized or degraded in vivo after administration, and is recoverable from urine for potential reuse.

13. The in vivo administrable complex of claim 1,
wherein the total size and surface charge of the polysaccharide-crosslinked colloidal particle are controlled by adjusting at least one parameter selected from the group consisting of: the molecular weight of the polysaccharide, the length of the polysaccharide backbone, the type of crosslinker used, the amount and rate of crosslinker addition during synthesis, and the degree of subsequent chemical modification,
such that a desired blood circulation time or a desired biodistribution and excretion pharmacokinetic profile is achieved.

14. The in vivo administrable complex of claim 1,
wherein the crosslinker-derived functional groups exposed on the surface of the polysaccharide-crosslinked colloidal particle are selected from the group consisting of an amine group, a carboxyl group, a hydroxyl group, and a thiol group.

15. The in vivo administrable complex of claim 1,
wherein the polysaccharide-crosslinked colloidal particle is engineered to function as an anti-opsonization agent.

16. The in vivo administrable complex of claim 1,
wherein the surface of the functional nanoparticle is modified with a polysaccharide-crosslinked colloidal particle such that the functional nanoparticle is uniformly coated,
and wherein the resulting complex is engineered to have a compact hydrodynamic diameter that enables renal filtration and/or prolongs blood circulation time.

17. The in vivo administrable complex of claim 1,
wherein a targeting molecule is conjugated (i) via a crosslinker-derived functional group exposed on the surface of the polysaccharide-crosslinked colloidal particle or (ii) via a surface functional group of the functional nanoparticle or the drug.

18. The in vivo administrable complex of any one of claims 1 to 17,
wherein a magnetic resonance imaging (MRI) contrast effect is imparted by either:
(i) divalent or trivalent iron ions coordinated to crosslinker-derived functional groups exposed on the surface of the polysaccharide-crosslinked colloidal particle, such that a T1-weighted MRI contrast effect is produced; or
(ii) the functional nanoparticle being an iron oxide particle capable of serving as an MRI contrast agent.

19. The in vivo administrable complex of any one of claims 1 to 17,
wherein the polysaccharide-crosslinked colloidal particle is prepared by a method comprising:
Step 1 of preparing an aqueous solution of dextran or a dextran derivative;
Step 2 of dropwise adding an epoxide and an alkaline aqueous solution at room temperature;
Step 3 of dropwise adding divalent or higher-valent polyamines at room temperature to form dextran-crosslinked nanoparticles bearing terminal amine groups;
Step 4 of precipitating the resulting product;
Step 5 of redispersing the precipitated product in water;
optionally, Step 6 of recovering the dextran-crosslinked nanoparticles bearing terminal amine groups by dialysis;
Step 7 of adding an organic acid anhydride to the dextran-crosslinked nanoparticles bearing terminal amine groups, so as to substitute at least a portion or all of the terminal amine groups with carboxylic acid and/or carboxylate groups;
optionally, Step 8 of purifying a solution of dextran-crosslinked nanoparticles bearing carboxylic acid and/or carboxylate groups to obtain water-dispersible dextran-crosslinked nanoparticles;
optionally, Step 9 of adding an iron precursor (e.g., iron chloride) or an aqueous solution of iron oxide nanoparticles to the water-dispersible dextran-crosslinked nanoparticles prepared in the previous step, to obtain (i) dextran-crosslinked nanoparticles having a shell formed of divalent or trivalent iron ions, or (ii) complexes in which the surface of iron oxide nanoparticles is surface-modified with the dextran-crosslinked nanoparticles; and
optionally, Step 10 of purifying or concentrating the nanostructures obtained in the previous step by ultrafiltration.

20. A composition for modifying a substance intended for in vivo administration, comprising:
a polysaccharide-crosslinked colloidal particle formed by intra- and/or inter-molecular crosslinking of 1 to 3 branched polysaccharides or 2 to 30 cyclic polysaccharides at hydroxyl groups of constituent monosaccharide building blocks using a crosslinker,
wherein the polysaccharide-crosslinked colloidal particle is engineered to:
(i) resist enzymatic hydrolysis in vivo;
(ii) be dispersible in vivo without aggregation and to be squeezable, such that it is capable of migrating within in vivo structures;
(iii) exhibit bio-inertness by minimizing interactions with biological systems and suppressing biological responses; and
(iv) be filterable through the kidney;
and wherein the type and number of crosslinker-derived functional groups exposed on the surface of the polysaccharide-crosslinked colloidal particle are controlled such that the substance intended for in vivo administration can be conjugated thereto.

21. The composition for modifying the substance of claim 20,
wherein the polysaccharide-crosslinked colloidal particle functions as one or more of the following:
a colloidal stability imparting modifier for in vivo administration,
a squeezable modifier for tissue penetration,
a hydrodynamic size modifier,
a surface charge modifier,
an anti-opsonization agent,
a blood circulation half-life regulator,
a pharmacokinetic modulator,
an extravasation modulator,
a blood capillary permeability modulator,
a modifier for blood circulation,
a modifier for lymph circulation,
a modulator for avoidance of phagocytosis by macrophages,
a modifier for body location tracking for MRI,
a modifier for intravenous administration,
a modifier for lymphatic administration or drainage,
a modifier for oral administration,
a regulator for distribution and excretion, or
a drug vehicle for drug loading and release.

22. The composition for modifying the substance of claim 20,
wherein the substance is a functional nanoparticle or a drug intended for in vivo administration.

23. The composition for modifying the substance of any one of claims 20 to 22,
wherein the polysaccharide-crosslinked colloidal particle or the functional nanoparticle as the substance is engineered to function as an MRI contrast agent.
